# EUROPEAN PATENT APPLICATION

(11) **EP 4 497 376 A2**
(43) Date of publication of application: **29.01.2025**
(21) Application number: 24202945.2
(22) Date of filing: 08.01.2021
(51) Int. Cl.: A61B 5/0245

(54) **DEVICES AND METHODS FOR TREATING CANCER AND CARDIAC WASTING**

(30) Priority: 08.01.2020 US 202062958621 P
(62) Divisional of application: 21711016.2
(71) Applicant: Myotec Therapeutics Limited, Dublin, Dublin 7, D07 P4AX (IE)
(72) Inventor: COATS, Andrew, Richmond (AU)
(74) Representative: Avidity IP

(57) **Abstract**

Disclosed herein are methods that can comprise detecting a cardiopulmonary measurement in a subject. Disclosed herein are methods that can comprise monitoring a disease in a patient by detecting a cardiopulmonary measurement. Disclosed herein are methods that can comprise treating a patient after detecting a cardiopulmonary measurement in a subject.

## Description

### CROSS-REFERENCE

This application claims the benefit of U.S. Provisional Application No. 62/958,621, filed January 8th, 2020, which application is incorporated herein by reference.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### SUMMARY

Disclosed herein in some embodiments is a method that can comprise detecting a cardiopulmonary measurement in a subject suspected of having cancer or suspected of having a risk of developing a cancer and performing a cancer screening. In some embodiments, a cardiopulmonary measurement can comprise: a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that can comprise less than about 100 msec, a percentage of successive normal sinus R-R intervals >50 msec (pNN50) that can comprise less than 10% and an SDANN during a time period of from about 1 minute to about 60 minutes of at least 100 msec, a left ventricular (LV) mass of from about 1% to about 70% (for example, from about 2% to about 20%) below that of an LV mass of an average human or below a previously determined LV mass of that patient, as determined by a computer tomography (CT) scan, or any combination thereof. In some cases, a cardiopulmonary measurement can comprise a left ventricular (LV) mass of from about 2% to about 20% below that of an LV mass of an average human or below a previously determined LV mass of that patient, as determined by a computer tomography (CT) scan can further comprise diagnosing a subject with cardiac wasting. In some cases, diagnosing can comprise echocardiography, or a magnetic resonance imaging (MRI)assessment, or a PET-CT assessment or a scintigraphy assessment. In some cases, the subject can have a terminal illness and can be considered to be in need of palliative and/or hospice care whether at home or in institutional care structures. In some cases, a subject can have elevated plasma levels of a natriuretic peptide, as compared to a healthy subject. In some cases, a subject can have elevated plasma levels of a troponin, as compared to a healthy subject. In some cases, a troponin can comprise troponin C, troponin I or troponin T. In some cases, a subject can have elevated plasma levels of aldosterone, as compared to a healthy subject.

Also disclosed herein in some embodiments is a method that can comprise contacting a cardioverter defibrillator with a subject in need thereof based on a detection of a cardiopulmonary measurement and actuating a cardioverter defibrillator that is directly or indirectly contacted with a subject. In some embodiments, a cardiopulmonary measurement can comprise: from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that can comprise less than about 100 msec, a percentage of successive normal sinus R-R intervals >50 msec (pNN50) that can comprise less than 10% and an SDANN during a time period of from about 1 minute to about 60 minutes of at least 100 msec, a left ventricular (LV) mass of from about 1% to about 70% below that of an LV mass of an average human or below a previously determined LV mass of that patient, as determined by a computer tomography (CT) scan, or any combination thereof. In some cases, a subject can have or can be suspected of having cardiac wasting. In some cases, actuating a cardioverter defibrillator that is directly or indirectly contacted with a subject can treat cardiac wasting in a subject. In some cases, a cardiopulmonary measurement can comprise a left ventricular (LV) mass of from about 2% to about 20% below that of an LV mass of an average human or below a previously determined LV mass of that patient, as determined by a computer tomography (CT) scan further comprising diagnosing a subject with cardiac wasting. In some cases, diagnosing can comprise echocardiography, or a magnetic resonance imaging (MRI)assessment, or a PET-CT assessment or a scintigraphy assessment. In some cases, a subject can have a terminal illness and can be considered to be in need of palliative and/or hospice care whether at home or in institutional care structures. In some cases, a subject can have elevated plasma levels of a natriuretic peptide, as compared to a healthy subject. In some cases, a subject can have elevated plasma levels of a troponin, as compared to a healthy subject. In some cases, a troponin can comprise troponin I or troponin T. In some cases, a subject can have elevated plasma levels of aldosterone, as compared to a healthy subject.

Also disclosed herein in some embodiments is a method that can comprise preventing or treating cardiac wasting in a subject having cardiac wasting or suspected of being at risk of cardiac wasting. Such a method can comprise detecting a cardiopulmonary measurement and administering a medicament to a subject, thereby treating cardiac wasting in a subject. In some embodiments, a cardiopulmonary measurement can comprise a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that can comprise less than about 100 msec, a percentage of successive normal sinus R-R intervals >50 msec (pNN50) that can comprise less than 10% and an SDANN during a time period of from about 1 minute to about 60 minutes of at least 100 msec, a left ventricular (LV) mass of from about 1% to about 70% below that of an LV mass of an average human or below a previously determined LV mass of that patient, as determined by a computer tomography (CT) scan, or any combination thereof. In some cases, a method can improve an outcome selected from the group consisting of: a quality of life of a subject, a symptom of cardiac wasting, a general wellbeing of a subject, a cardiac health of a subject, a predicted survival time, a duration of hospitalization of a subject, a cost of hospitalization for a subject, or any combination thereof. In some cases, a medicament can comprise: a beta receptor blocker, a beta-3 receptor agonist, a beta-3 receptor antagonist, an ACE inhibitor, a renin inhibitor, a vasopressin antagonist, angiotensin receptor antagonist, a neutral endo-peptidase inhibitor, a dual vasopeptidase inhibitor, an aldosterone antagonist, a mineralocorticoid receptor antagonist, an aldosterone synthesis inhibitor, cardioxyl, omecamtiv mecarbil, relaxin, serelaxin, staroxime, bucindolol, a phosphodiesterase 5 inhibitor, a vasopressin inhibitor, levosimendan, a sodium-glucose cotransporter-2 (SGLT2) inhibitor, an If channel blocker, a metabolism-modifying agent, a soluble guanylate cyclase stimulator, a soluble guanylate cyclase activator, a stereoisomer of any of these, a salt of any of these, or any combination thereof. In some cases, a beta receptor blocker or salt thereof can be administered, where a beta receptor blocker or salt thereof can comprise at least one stereocenter in an *S*-configuration. In some cases, a beta-blocker can be *S*-pindolol, *S*-oxprenolol, *S-*atenolol, acebutolol, atenolol, bisoprolol, metoprolol, nadolol, nebivolol, propranolol, or an isomer or stereoisomer or salt of any of these. In some cases, a phosphodiesterase 5 inhibitor can comprise amrinone, milrinone, avanafil, lodenafil, mirodenafil, sildenafil, tadalafil, vardenafil, udenafil, zaprinast, benzaminenafil, carvedilol, or a salt of any of these. In some cases, a vasopressin inhibitor can comprise tolvaptan, or a salt thereof. In some cases, an SGLT2 inhibitor can comprise dapagliflozin, empagliflozin, canagliflozin, sotagliflozin, ertugliflozin, ipragliflozin, luseogliflozin, remogliflozin etabonate, sergliflozin etabonate, tofogliflozin a stereoisomer of any of these, or a salt of any of these, or any combination thereof. In some cases, an aldosterone antagonist or salt thereof can comprise spironolactone, eplerenone, a stereoisomer of any of these, or a salt of any of these. In some cases, a mineralocorticoid receptor antagonist or salt thereof can comprise finerenone, potassium canrenoate, spironolactone, CaroSpir, Inspra, a stereoisomer of any of these, or a salt of any of these. In some cases, an aldosterone synthesis inhibitor or a salt thereof can comprise fadrozol, FAD 286, LCI699, or any combination thereof. In some cases, an angiotensin receptor antagonist or a salt thereof can comprise a sartan, candesartan, irbesatan, valsartan, telmisartan, eprosartan, Olmesartan, azilsartan, fimasartan, sacubitril/valsartan, losartan, EXP 3174, amlodipine, a stereoisomer of any of these, a salt of any of these, or any combination thereof. In some cases, an I*_{f}* channel blocker or a salt thereof can comprise ivabradine, zatebradine, cilobradine, ZD7288, alinidine, a salt of any of these, or any combination thereof. In some cases, a metabolism-modifying agent or a salt thereof can comprise elamipretide, trimetazidine, perhexiline, etomoxir, dichloroacetate, coenzyme Q10, ubiquinol, nicotinamide riboside, metformin, resveratrol, pterostilbene, rapamycin, ghrelin, a salt of any of these, or any combination thereof.

In some cases, a cardioverter defibrillator can comprise an implantable cardioverter defibrillator. In some cases, a cardioverter defibrillator can comprise a wearable cardioverter defibrillator. In some cases, a wearable cardioverter defibrillator can comprise a device that can be worn on a subject and taken off when needed. In some cases, a wearable cardioverter defibrillator can comprise a battery. In some cases, a batter can be charged. In some cases, a wearable cardioverter defibrillator can be at least partially waterproof. In some cases, a cardioverter defibrillator can comprise a wireless communication device. In some cases, a wireless communication device can transmit a signal to a second device. In some cases, a signal can comprise data. In some cases, data can comprise a cardiopulmonary measurement, an alert, information regarding a defibrillation that has been or will be administered, an emergency signal, or any combination thereof. In some embodiments, an emergency signal can alert a first responder, a healthcare professional, or a combination thereof. In some cases, a wireless communication can comprise a Wi-Fi transmitter, a Bluetooth transmitter, an ANT+ transmitter, a radio transmitter, or any combination thereof. In some cases, a cardioverter defibrillator can be applied or held in proximity to a skin of a subject. In some cases, a cardiopulmonary measurement can comprise from about 10 to about 100 premature ventricular contractions in a time period, wherein a time period can comprise about 24 hours. In some cases, a cardiopulmonary measurement can comprise an average heart rate during a time period of from about 60 to about 100 bpm and an SDANN during a time period that can comprise less than about 100 msec, wherein a time period can comprise about an hour. In some cases, a cardiopulmonary measurement can comprise a pNN50 that can comprise less than 10% and an SDANN during a time period of at least 100 msec, wherein a time period can comprise about an hour.

Also disclosed herein in some embodiments is a method that can comprise performing a cancer screen based on a detection of a cardiopulmonary measurement in a subject, wherein a cardiopulmonary measurement can comprise: from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that can comprise less than about 100 msec, a percentage of successive normal sinus R-R intervals >50 msec (pNN50) that can comprise less than 10% and an SDANN during a time period of from about 1 minute to about 60 minutes of at least 100 msec, a left ventricular (LV) mass of from about 1% to about 70% below that of an LV mass of an average human or below a previously determined LV mass of that patient, as determined by a computer tomography (CT) scan, or any combination thereof.

Also disclosed herein in some embodiments is an interoperable electronic device configured to measure a cardiopulmonary measurement, interpret a risk of a presence of a cancer using an algorithm stored on computer readable memory operatively coupled to a device, and transmit a detection of a risk of a presence of a cancer to a second device.

Also disclosed herein in some embodiments is an interoperable electronic device configured to measure a cardiopulmonary measurement, interpret a risk of a presence of wasting of left ventricular muscle mass using an algorithm stored on computer readable memory operatively coupled to a device, and transmit a detection of a presence of a wasting of left ventricular muscle mass to a second device. In some cases, an interoperable electronic device transmits a detection of a cancer to a second device by a wired or wireless transmission. In some cases, an interoperable electronic device transmits a detection of a wasting of left ventricular muscle mass to a second device by a wired or wireless transmission.

Also disclosed herein in some embodiments is a kit that can comprise an interoperable electronic device as disclosed herein at least partially in a packaging. In some cases, a kit can comprise instructions to assess a risk of cancer by recording a cardiopulmonary measurement using an interoperable electronic device. In some cases, a kit can comprises instructions to assess a risk of wasting of left ventricular muscle mass by recording a cardiopulmonary measurement using an interoperable electronic device. In some cases, a cardiopulmonary measurement can comprise: from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that can comprise less than about 100 msec, a percentage of successive normal sinus R-R intervals >50 msec (pNN50) that can comprise less than 10% and an SDANN during a time period of from about 1 minute to about 60 minutes of at least 100 msec, a left ventricular (LV) mass of from about 1% to about 70% below that of an LV mass of an average human or below a previously determined LV mass of that patient, as determined by a computer tomography (CT) scan, or any combination thereof.

Also disclosed herein in some embodiments is a method that can comprise monitoring a cancer in a subject, comprising: (a) recording a cardiopulmonary measurement in a subject using an interoperable electronic device as disclosed herein, comparing a cardiopulmonary measurement to a reference level, and repeating the previous steps.

Also disclosed herein in some embodiments is a method that can comprise monitoring a wasting of a left ventricular muscle mass in a subject, comprising: recording a cardiopulmonary measurement in a subject using an interoperable electronic device as disclosed herein, comparing a cardiopulmonary measurement to a reference level, and repeating the previous steps.

Also disclosed herein in some embodiments is a method of determining efficacy of an anti-cancer therapeutic in a subject administered an anti-cancer therapeutic, comprising: recording a cardiopulmonary measurement in a subject using an interoperable electronic device as disclosed herein, comparing a cardiopulmonary measurement to a reference level, repeating the previous steps for as long as a cardiopulmonary measurement remains higher than a reference level, and performing one of the following: terminating the administering of an anti-cancer therapeutic; continuing the administering of an anti-cancer therapeutic; or recommending a change in an anti-cancer therapeutic if a cardiopulmonary measurement remains higher than a reference level for a time period of at least about a month after a first cardiopulmonary recording.

Also disclosed herein in some embodiments is a method of determining a cancer prognosis in a subject comprising: recording a number of premature ventricular contractions (PVCs) in a 24 hour time period in a subject using an electronic device configured to monitor PVCs, repeating a recording, and delivering a prognosis to a subject, wherein a subject can have a more favorable prognosis if a subject displays a number of PVCs that can comprise less than 10 or greater than 100, relative to a subject that displays a number of PVCs of from about 10 to about 100. In some cases, determining a cancer prognosis can further comprise conducting a cancer diagnostic test, wherein a cancer diagnostic test determines a level of a biomarker. In some cases, a biomarker can comprise a genetic biomarker. In some cases, a biomarker can comprise an epigenetic genetic biomarker. In some cases, determining can comprise sequencing a sample from a subject. In some cases, sequencing a sample from a subject can comprise next generation sequencing. In some cases, a sample from a subject can comprise a cell free nucleic acid. In some cases, determining can comprise imaging. In some cases, a level of a biomarker can be greater than a reference level. In some cases, a level of a biomarker can be less than a reference level.

Also disclosed herein in some embodiments is a method of determining efficacy of a cardiac-active therapeutic in a subject administered a cardiac-active therapeutic, comprising: recording a cardiopulmonary measurement in a subject using an interoperable electronic device as disclosed herein, comparing a cardiopulmonary measurement to a reference level, repeating these steps for as long as a cardiopulmonary measurement remains higher than a reference level, and performing one of the following: terminating the administering of a cardiac-active therapeutic; continuing the administering of a cardiac-active therapeutic; or recommending a change in a cardiac-active therapeutic if a cardiopulmonary measurement remains higher than a reference level for a time period of at least about a month after a first recording of a cardiopulmonary measurement. In some cases, a cardiac-active therapeutic can comprise one of the group consisting of: a modulator of a renin angiotensin aldosterone system (RAAS); a neutral endopeptidase (NEP) inhibitor, a natriuretic peptide (NP) receptor agonist, a recombinant NP, a synthetic NP, ularitide, a relaxin receptor activator, a serelaxin receptor activator, a beta-adrenergic receptor modulating agent, a dual vasopeptidase inhibitor, cardioxyl, omecamtiv mecarbil, a phosphodiesterase 5 inhibitor, levosimendan, a sodium-glucose cotransporter-2 (SGLT2) inhibitor, an If channel blocker, a metabolism-modifying agent, a soluble guanylate cyclase stimulator, a soluble guanylate cyclase activator, a stereoisomer of any of these, a salt of any of these, or any combination thereof. In some cases, a modulator of RAAS or salt thereof can comprise a renin inhibitor, an angiotensin-converting enzyme (ACE) inhibitor, an angiotensin receptor antagonist, an angiotensin receptor agonist, an aldosterone synthesis inhibitor, or a salt of any of these. In some cases, a beta-adrenergic receptor modulating agent can comprise a competitive inhibitor, a partial agonist, an agonist, an activator, or an inverse agonist.

Also disclosed herein in some embodiments is a method of determining a cardiac wasting prognosis in a subject. Such a method can comprise recording a number of premature ventricular contractions (PVCs) in a 24 hour time period in a subject using an electronic device configured to monitor PVCs, repeating the previous steps, and delivering a prognosis to a subject, wherein a subject can have a more favorable prognosis if a subject displays a number of PVCs that comprises less than 10 or greater than 100, relative to a subject that displays a number of PVCs of from about 10 to about 100. In some cases, a method of determining cardiac wasting can further comprise conducting a cardiac wasting diagnostic test, wherein a cardiac wasting diagnostic test determines a level of a biomarker. In some cases, a biomarker can comprise a genetic biomarker. In some cases, determining can comprise sequencing. In some cases, sequencing can be performed on a cell free nucleic acid from a subject. In some cases, a subject can have a level of a biomarker that can be greater than a reference level. In some cases, a subject can have a level of a biomarker that can be less than a reference level.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

Novel features are set forth with particularity in the appended claims. A better understanding of the features and advantages of the present disclosure will be obtained by reference to the following detailed description that sets forth illustrative embodiments, in which certain principles are utilized, and the accompanying drawings of which:
**FIG. 1** shows factors affecting a heart and potential associated diseases and outcomes
**FIG. 2** shows the relationship between wall stress, pressure, radius, and wall thickness in determining the development of cardiac wasting induced heart failure versus the development of heart failure with cardiac remodeling.
**FIG. 3** shows a proportion of subjects with NSVT (non-sustained ventricular tachycardia) & >1 NSVT *p<0.05 vs. controls in a prospective analysis of ventricular tachycardia, premature ventricular contractions, and mortality in unselected patients with advanced cancer.
**FIG. 4** shows a proportion of subjects with ≥20 PVC (premature ventricular contraction) & ≥50 PVC in a prospective analysis of ventricular tachycardia, premature ventricular contractions, and mortality in unselected patients with advanced cancer.
**FIG. 5** shows a cumulative survival of all cancer patients (n=120) in a prospective analysis of ventricular tachycardia, premature ventricular contractions, and mortality in unselected patients with advanced cancer.
**FIG. 6** shows a cumulative survival of all cancer patients (n=120) in a prospective analysis of ventricular tachycardia, premature ventricular contractions, and mortality in unselected patients with advanced cancer.
**FIG. 7** shows cumulative survival of patients with colorectal and pancreatic cancer (n=87) in a prospective analysis of ventricular tachycardia, premature ventricular contractions, and mortality in unselected patients with advanced cancer.
**FIG. 8** shows indication for 24-hour ECG in cancer patients (n=261) ECG = Electrocardiogram; CV= cardiovascular event; AF = atrial fibrillation.
**FIG. 9** shows a proportion of subjects with NSVT ≥3 beats & ≥4 beats & ≥6 beats.
**FIG. 10** shows a proportion of subjects with ≥20 PVC & ≥50 PVC & ≥3000 PVC / 24h.
**FIG. 11** shows cumulative survival of all cancer patients (n=261); NSVT = non-sustained ventricular tachycardia, PVC = premature ventricular contraction.
**FIG. 12** shows cumulative survival of all cancer patients (n=261); NSVT = non-sustained ventricular tachycardia, PVC = premature ventricular contraction.
**FIG. 13** shows cumulative survival of all cancer patients (n=261); NSVT = non-sustained ventricular tachycardia, PVC = premature ventricular contraction.
**FIG. 14** shows improvements in quality of life (QoL) using the Kansas City Cardiomyopathy Questionnaire (KCCQ). N=4, LVEF > 50%. A 5 unit increase was considered a clinically meaningful change. Follow up scores suggested clinically relevant improvements from baseline to follow up (≥5). The KCCQ score range is 0-100, with higher scores indicating better QoL, and improvements of ≥5 indicated clinically important changes.
**FIG. 15** shows improvements in quality of life (QoL) using the Minnesota Living with Heart Failure Questionnaire (MLHFQ). N=4, LVEF > 50%. Follow up scores suggest improvements from baseline to follow-up, with good QoL at follow-up. MI,HFQ has a total score range of 0-105. A lower score indicated a better QoL, with a score of < 24 indicating good QoL, scores from 24 to 25 indicating moderately impaired QoL, scores > 45 indicating low QoL.

### DETAILED DESCRIPTION

### I. Overview

Disclosed herein are methods that can comprise detecting a cardiopulmonary measurement in a subject. Disclosed herein are methods that can comprise monitoring a disease in a patient by detecting a cardiopulmonary measurement. Disclosed herein are methods that can comprise treating a patient after detecting a cardiopulmonary measurement in a subject.

Disclosed in some embodiments herein is a method comprising detecting a cardiopulmonary measurement in a subject suspected of having cancer or suspected of having a risk of developing a cancer, and performing a cancer screening, wherein a cardiopulmonary measurement comprises: a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that comprises less than about 100 msec, a percentage of successive normal sinus R-R intervals >50 msec (pNN50) that comprises less than 10% and an SDANN during a time period of from about 1 minute to about 60 minutes of at least 100 msec, a left ventricular (LV) mass of from about 1% to about 70% below that of an LV mass of an average human or below a previously determined LV mass of a subject, as determined by a computer tomography (CT) scan, or any combination thereof.

Also disclosed in some embodiments herein is a method comprising actuating a cardioverter defibrillator that is directly or indirectly contacted with a subject in need thereof based on a detection of a cardiopulmonary measurement, wherein a cardiopulmonary measurement comprises: a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that comprises less than about 100 msec, a percentage of successive normal sinus R-R intervals >50 msec (pNN50) that comprises less than 10% and an SDANN during a time period of from about 1 minute to about 60 minutes of at least 100 msec, a left ventricular (LV) mass of from about 1% to about 70% below that of an LV mass of an average human or below a previously determined LV mass of a subject, as determined by a computer tomography (CT) scan, or any combination thereof.

Also disclosed in some embodiments herein is a method of preventing or treating cardiac wasting in a subject having cardiac wasting, or suspected of being at risk of cardiac wasting, comprising detecting a cardiopulmonary measurement, wherein a cardiopulmonary measurement comprises: a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that comprises less than about 100 msec, a percentage of successive normal sinus R-R intervals >50 msec (pNN50) that comprises less than 10% and an SDANN during a time period of from about 1 minute to about 60 minutes of at least 100 msec, a left ventricular (LV) mass of from about 1% to about 70% below that of an LV mass of an average human or below a previously determined LV mass of a subject, as determined by a computer tomography (CT) scan, or any combination of thereof, and administering a medicament to a subject, thereby treating a cardiac wasting in a subject.

Also disclosed in some embodiments herein is a method comprising performing a cancer screen based on a detection of a cardiopulmonary measurement in a subject, wherein a cardiopulmonary measurement comprises: a tachycardia or non-sustained ventricular tachycardia of at least about 100 bpm, from about 10 to about 5,000,000 premature ventricular contractions in a time period of from about 30 seconds to about 3 weeks, an average heart rate during a time period of from about 60 to about 100 bpm and a standard deviation of a 5-minute average NN interval (SDANN) during a time period of from about 1 minute to about 60 minutes that comprises less than about 100 msec, a percentage of successive normal sinus R-R intervals >50 msec (pNN50) that comprises less than 10% and an SDANN during a time period of from about 1 minute to about 60 minutes of at least 100 msec, a left ventricular (LV) mass of from about 1% to about 70% below that of an LV mass of an average human or below a previously determined LV mass of a subject, as determined by a computer tomography (CT) scan, or any combination thereof.

Also disclosed in some embodiments herein is an interoperable electronic device configured to measure a cardiopulmonary measurement, interpret a risk of a presence of a cancer using an algorithm stored on computer readable memory operatively coupled to a device, and transmit a detection of a risk of a presence of a cancer to a second device.

Also disclosed in some embodiments herein is an interoperable electronic device configured to measure a cardiopulmonary measurement, interpret a risk of a presence of wasting of left ventricular muscle mass using an algorithm stored on computer readable memory operatively coupled to a device, and transmit a detection of a presence of a wasting of a left ventricular muscle mass to a second device.

Also disclosed in some embodiments herein is a method of determining a cancer prognosis in a subject comprising (a) recording a number of premature ventricular contractions (PVCs) in a 24 hour time period in a subject using an electronic device configured to monitor PVCs, (b) repeating (a), and (c) delivering a prognosis to a subject, wherein a subject has a more favorable prognosis if a subject displays a number of PVCs that comprises less than 10 or greater than 100, relative to a subject that displays a number of PVCs of from about 10 to about 100.

Also disclosed in some embodiments herein is a method comprising determining a cardiac wasting prognosis in a subject comprising (a) recording a number of premature ventricular contractions (PVCs) in a 24 hour time period in a subject using an electronic device configured to monitor PVCs, (b) repeating (a), and (c) delivering a prognosis to a subject, wherein a subject has a more favorable prognosis if a subject displays a number of PVCs comprising less than about 10 or greater than about 100, relative to a subject that displays a number of PVCs comprising from about 10 to about 100.

### II. Definitions

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" can mean within an acceptable error range for the particular value as determined by one of ordinary skill in the art, which will depend in part on how the value is measured or determined, e.g., the limitations of the measurement system. For example, "about" can mean plus or minus 10%, per the practice in the art. Alternatively, "about" can mean a range of plus or minus 20%, plus or minus 10%, plus or minus 5%, or plus or minus 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value should be assumed. Also, where ranges and/or subranges of values are provided, the ranges and/or subranges can include the endpoints of the ranges and/or subranges.

The term "substantially" as used herein can refer to a value approaching 100% of a given value. For example, a compound that is "substantially localized" in an organ can indicate that about 90% by weight of a compound, salt, or metabolite is present in an organ relative to a total amount of a compound, salt, or metabolite. In some cases, the term can refer to an amount that can be at least about 90%, 91%, 92%, 93%, 94%, 95%, 96%, 97%, 98%, 99%, 99.9%, or 99.99% of a total amount. In some cases, the term can refer to an amount that can be about 100% of a total amount.

The term "subject", "patient" or "individual" as used herein can encompass a mammal and a non-mammal. A mammal can be any member of the Mammalian class, including but not limited to a human, a non-human primates such as a chimpanzee, an ape or other monkey species; a farm animal such as cattle, a horse, a sheep, a goat, a swine; a domestic animal such as a rabbit, a dog (or a canine), and a cat (or a feline); a laboratory animal including a rodent, such as a rat, a mouse and a guinea pig, and the like. A non-mammal can include a bird, a fish and the like. In some embodiments, a subject can be a mammal. In some embodiments, a subject can be a human. In some instances, a human can be an adult. In some instances, a human can be a child. In some instances, a human can be age 0-17 years old. In some instances, a human can be age 18-130 years old. In some instances, a subject can be a male. In some instances, a subject can be a female. In some instances, a subject can be diagnosed with, or can be suspected of having, a condition or disease. In some instances, a disease or condition can be cancer. In some instances, a disease or condition can be cardiac wasting. A subject can be a patient. A subject can be an individual. In some instances, a subject, patient or individual can be used interchangeably.

In some instances, "treat," "treating", "treatment," "ameliorate" or "ameliorating" and other grammatical equivalents can include prophylaxis. "Treat," "treating", "treatment," "ameliorate" or "ameliorating" and other grammatical equivalents can further include achieving a therapeutic benefit and/or a prophylactic benefit. Therapeutic benefit can mean eradication of the underlying disease being treated. Also, a therapeutic benefit can be achieved with the eradication of one or more of the physiological symptoms associated with the underlying disease such that an improvement can be observed in a subject notwithstanding that, in some embodiments, the subject can still be afflicted with the underlying disease.

### III. Subjects

Disclosed herein are methods of diagnosing and treating a subject based on a detection of a cardiopulmonary measurement. In some cases, a subject can be a patient. In some cases, a subject can have or be at risk of having a disease. In some cases, a disease can comprise a cancer. In some cases, a disease can comprise muscle wasting. In some cases, muscle wasting can comprise cardiac wasting. In some cases, cardiac wasting can comprise wasting of ventricular muscle mass. In some cases, wasting of ventricular muscle mass can comprise wasting of left ventricular muscle mass. In some cases, a subject can be suspected of having a cancer or suspected of having a risk of developing a cancer. In some cases, a cancer screening can be performed. In some cases, a subject can have cardiac wasting. In some cases, a subject can be suspected of having cardiac wasting.

In some cases, a cardiopulmonary measurement can be used to inform a diagnosis of cardiac wasting. In some cases, a diagnosing can comprise echocardiography, magnetic resonance imaging (MRI) assessment, PET-CT assessment, scintigraphy assessment, or any combination thereof. In some cases, a subject can have a terminal illness. In some cases, a subject can have elevated plasma levels of a natriuretic peptide, as compared to a healthy subject. In some cases, a subject can have elevated plasma levels of a troponin, as compared to a healthy subject. In some cases, a troponin can comprise troponin I or troponin T. In some cases, a subject can have elevated plasma levels of aldosterone, as compared to a healthy subject. In some cases, a subject can have Chronic Obstructive Pulmonary Disease (COPD). In some cases, a subject can have COPD exacerbations.

In some cases, a subject can be considered to be in need of palliative and/or hospice care. In some cases, palliative care can be at home or in institutional care structures.

### IV. Cardiopulmonary measurements

As described herein, a method of treating a subject can comprise performing a cardiopulmonary measurement. In some cases, a cardiopulmonary measurement can comprise a tachycardia or non-sustained ventricular tachycardia of at least about 100 bpm, about 105 bpm, about 110 bpm, about 115 bpm, about 120 bpm, about 125 bpm, about 130 bpm, about 135 bpm, about 140 bpm, about 145 bpm, about 150 bpm, about 155 bpm, about 160 bpm, about 165 bpm, about 170 bpm, about 180 bpm, about 185 bpm, about 190 bpm, about 195 bpm, or about 200 bpm.

In some cases, a cardiopulmonary measurement can comprise a number of premature ventricular contractions in a time period. In some cases, a number of premature ventricular contractions can comprise from about 10 to about 100, from about 20 to about 100, from about 30 to about 100, from about 40 to about 100, from about 50 to about 100, from about 60 to about 100, from about 70 to about 100, from about 80 to about 100, or from about 90 to about 100 in a time period. In some cases, a time period can comprise from about 30 seconds to about 1 hour, from about 1 minute to about 1 hour, from about 2 minutes to about 1 hour, from about 3 minutes to about 1 hour, from about 4 minutes to about 1 hour, from about 5 minutes to about 1 hour, from about 10 minutes to about 1 hour, from about 15 minutes to about 1 hour, from about 20 minutes to about 1 hour, from about 25 minutes to about 1 hour, from about 30 minutes to about 1 hour, from about 35 minutes to about 1 hour, from about 40 minutes to about 1 hour, from about 45 minutes to about 1 hour, from about 50 minutes to about 1 hour, from about 55 minutes to about 1 hour, from about 1 hour to about 24 hours, about 2 to about 24 hours, about 3 to about 24 hours, about 4 to about 24 hours, about 5 to about 24 hours, about 6 to about 24 hours, about 7 to about 24 hours, about 8 to about 24 hours, about 9 to about 24 hours, about 10 to about 24 hours, about 11 to about 24 hours, about 12 to about 24 hours, about 13 to about 24 hours, about 14 to about 24 hours, about 15 to about 24 hours, about 16 to about 24 hours, about 17 to about 24 hours, about 18 to about 24 hours, about 19 to about 24 hours, about 20 to about 24 hours, about 21 to about 24 hours, about 21 to about 24 hours, about 22 to about 24 hours, about 32 to about 24 hours, about 1 to about 36 hours, or about 1 to about 48 hours.

In some cases, a cardiopulmonary measurement can comprise a number of premature ventricular contractions in a time period. In some cases, a number of premature ventricular contractions can comprise from about 10 to about 200, from about 10 to about 300, from about 10 to about 400, from about 10 to about 500, from about 10 to about 600, from about 10 to about 700, from about 10 to about 800, from about 10 to about 900, from about 10 to about 1000, from about 10 to about 1,500, from about 10 to about 2,000, from about 10 to about 2,500, from about 10 to about 3,000, from about 10 to about 3,500, from about 10 to about 4,000, from about 10 to about 4,500, from about 10 to about 5,000, from about 10 to about 5,500, from about 10 to about 6,000, from about 10 to about 6,500, from about 10 to about 7,000, from about 10 to about 7,500, from about 10 to about 8,000, from about 10 to about 8,500, from about 10 to about 9,000, from about 10 to about 9,500, from about 10 to about 10,000, from about 10 to about 15,000, from about 10 to about 20,000, from about 10 to about 25,000, from about 10 to about 30,000, from about 10 to about 35,000, from about 10 to about 40,000, from about 10 to about 45,000, from about 10 to about 50,000, from about 10 to about 55,000, from about 10 to about 60,000, from about 10 to about 65,000, from about 10 to about 70,000, from about 10 to about 75,000, from about 10 to about 80,000, from about 10 to about 85,000, from about 10 to about 90,000, from about 10 to about 95,000, from about 10 to about 100,000, from about 10 to about 150,000, from about 10 to about 200,000, from about 10 to about 250,000, from about 10 to about 300,000, from about 10 to about 350,000, from about 10 to about 400,000, from about 10 to about 450,000, from about 10 to about 500,000, from about 10 to about 550,000, from about 10 to about 600,000, from about 10 to about 650,000, from about 10 to about 700,000, from about 10 to about 750,000, from about 10 to about 800,000, from about 10 to about 850,000, from about 10 to about 900,000, from about 10 to about 950,000, from about 10 to about 1,000,000, from about 10 to about 1,500,000, from about 10 to about 2,000,000, from about 10 to about 2,500,000, from about 10 to about 3,000,000, from about 10 to about 3,500,000, from about 10 to about 4,000,000, from about 10 to about 4,500,000, from about 10 to about 5,000,000, from about 10 to about 5,500,000, from about 10 to about 6,000,000, from about 10 to about 6,500,000, from about 10 to about 7,000,000, from about 10 to about 7,500,000, from about 10 to about 8,000,000, from about 10 to about 8,500,000, from about 10 to about 9,000,000, from about 10 to about 9,500,000, from about 10 to about 10,000,000, from about 10 to about 1,500,000, from about 10 to about 2,000,000, from about 10 to about 2,500,000, from about 10 to about 3,000,000, from about 10 to about 3,500,000, from about 10 to about 4,000,000, from about 10 to about 4,500,000, from about 10 to about 5,000,000, from about 10 to about 5,500,000, from about 10 to about 6,000,000, from about 10 to about 6,500,000, from about 10 to about 7,000,000, from about 10 to about 7,500,000, from about 10 to about 8,000,000, from about 10 to about 8,500,000, from about 10 to about 9,000,000, from about 10 to about 9,500,000, or from about 10 to about 10,000,000. In some cases, a time period can be from about

In some cases, a cardiopulmonary measurement can comprise an average heart rate during a time period of from about 60 to about 100 bpm, about 61 to about 100 bpm, about 62 to about 100 bpm, about 63 to about 100 bpm, about 64 to about 100 bpm, about 65 to about 100 bpm, about 66 to about 100 bpm, about 67 to about 100 bpm, about 68 to about 100 bpm, about 69 to about 100 bpm, about 70 to about 100 bpm, about 71 to about 100 bpm, about 72 to about 100 bpm, about 73 to about 100 bpm, about 74 to about 100 bpm, about 75 to about 100 bpm, about 76 to about 100 bpm, about 77 to about 100 bpm, about 78 to about 100 bpm, about 79 to about 100 bpm, about 80 to about 100 bpm, about 81 to about 100 bpm, about 82 to about 100 bpm, about 83 to about 100 bpm, about 84 to about 100 bpm, about 85 to about 100 bpm, about 86 to about 100 bpm, about 87 to about 100 bpm, about 88 to about 100 bpm, about 89 to about 100 bpm, about 90 to about 100 bpm, about 91 to about 100 bpm, about 92 to about 100 bpm, about 93 to about 100 bpm, about 94 to about 100 bpm, about 95 to about 100 bpm, about 96 to about 100 bpm, about 97 to about 100 bpm, about 98 to about 100 bpm, about 99 to about 100 bpm.

In some cases, a cardiopulmonary measurement can comprise a standard deviation of a 5-minute average NN interval (SDANN) during a time period. In some cases, a time period can comprise from about 1 minute to about 60 minutes, about 10 minutes and about 60 minutes, about 20 minutes and about 60 minutes, about 30 minutes and about 60 minutes, about 40 minutes and about 60 minutes, or about 50 minute and about 60 minutes.

In some cases, a standard deviation of a 5-minute average NN interval (SDANN) can comprise at least about 10 msec, about 20 msec, about 30 msec, about 40 msec, about 50 msec, about 60 msec, about 70 msec, about 80 msec, about 90 msec, about 100 msec, about 110 msec, about 120 msec, about 130 msec, about 140 msec, about 150 msec, about 160 msec, about 170 msec, about 180 msec, about 190 msec, about 200 msec, about 210 msec, about 220 msec, about 230 msec, about 240 msec, about 250 msec, about 260 msec, about 270 msec, about 280 msec, about 290 msec, or about 300 msec.

In some cases, a cardiopulmonary measurement as disclosed herein can comprise an SDANN during a time period of less than about 10 msec, about 20 msec, about 30 msec, about 40 msec, about 50 msec, about 60 msec, about 70 msec, about 80 msec, about 90 msec, about 100 msec, about 110 msec, about 120 msec, about 130 msec, about 140 msec, about 150 msec, about 160 msec, about 170 msec, about 180 msec, about 190 msec, about 200 msec, about 210 msec, about 220 msec, about 230 msec, about 240 msec, about 250 msec, about 260 msec, about 270 msec, about 280 msec, about 290 msec, or about 300 msec.

In some cases, a cardiopulmonary measurement can comprise a percentage of successive normal sinus R-R intervals >50 msec (pNN50) that can comprise less than about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

In some cases, a cardiopulmonary measurement can comprise a left ventricular (LV) mass of from about 1% to about 20%, about 2% to about 20%, about 3% to about 20%, about 4% to about 20%, about 5% to about 20%, about 6% to about 20%, about 7% to about 20%, about 8% to about 20%, about 9% to about 20%, about 10% to about 20%, about 11% to about 20%, about 12% to about 20%, about 13% to about 20%, about 14% to about 20%, about 15% to about 20%, about 16% to about 20%, about 17% to about 20%, about 18% to about 20%, about 19% to about 20%, about 1% to about 30%, about 1% to about 40%, about 1% to about 50%, about 1% to about 60%, about 1% to about 70%, about 1% to about 80%, about 1% to about 90%, or about 1% to about 100% below that of an LV mass of an average human or below a previously determined LV mass of that patient.

In some cases, a cardiopulmonary measurement of ventricular mass can comprise an echocardiographic assessment. In some cases, a cardiopulmonary measurement of ventricular mass can comprise a computer tomography (CT) scan. In some cases, a cardiopulmonary measurement of ventricular mass can comprise a magnetic resonance imaging (MRI) scan. In some cases, a cardiopulmonary measurement of ventricular mass can comprise X-ray tomography. In some cases, a cardiopulmonary measurement of ventricular mass can comprise a positron emission tomography (PET) scan.

In some cases, a cardiopulmonary measurement can comprise one or more combinations of measurements as described herein.

### V. Association of measurement with disease

As described herein, a measured value of a subject can be associated with a disease state. In some cases, an association between a measured value of a subject and a disease state can be indicative of a disease in a subject. In some cases, a disease can comprise a cancer. In some cases, a disease can comprise a cardiac disease. In some cases, cancer and anti-cancer treatments can cause cardiac atrophy. In some cases, a disease can comprise symptoms. In some cases, symptoms can comprise breathlessness, lethargy, reduced exercise tolerance, over congestive cardiac failure, increased mortality, or any combination thereof. In some cases, a reduction in a size and number of cardiac muscle fibers can be indicative of a presence of a cancer in a subject. In some cases, increased extracellular muscle stroma can be indicative of a presence of a cancer in a subject.

In some cases, loss of cardiomyocyte volume and replacement with fibrotic tissue can be indicative of a presence of a cancer in a subject. In some cases, reduced left ventricular (LV) wall thickness can be indicative of a presence of a cancer in a subject. In some cases, reduced total heart weight can be indicative of a presence of a cancer in a subject.

In some cases, a cancer cachexia may show characteristics of cardiac atrophy. In some cases, cardiac atrophy can comprise decreased heart weight and LV mass as shown by echocardiography. In some cases, cardiac atrophy can comprise the thinning of septal, interventricular and posterior walls and chamber dilation as shown by echocardiography. In some cases, cardiac atrophy in cancer cachexia may be driven more by cellular atrophy than cell death by apoptosis. In some cases, the imbalance between protein degradation and protein synthesis can comprise the major cause of cardiac atrophy in cancer cachexia. In some cases, cardiac atrophy can comprise part of a complex systemic metabolic syndrome caused by a cancer that results in widespread muscle wasting. In some cases, widespread muscle wasting can comprise the myocardium.

In some cases, heart size can decrease when a protein degradation rate exceeds a protein synthesis rate. In some cases, a protein degradation rate exceeding a protein synthesis rate can be caused by decreased protein synthesis, increased autophagy, increased proteolysis via the ubiquitin-proteasome system, or any combination thereof. In some cases, suppression of a mechanistic target of a rapamycin pathway can result in a significant reduction in cardiac myofibrillar protein synthesis rate. In some cases, increased autophagy in cardiac atrophy can be detected by an upregulation of biomarkers beclin-1, p62 and LC3B.

In some cases, autophagic vacuoles can be observed in atrophic hearts using electron microscopy. In some cases, acetate treatment can improve cardiac function by downregulating autophagy, revealing the contribution of autophagy to cardiac atrophy. In some cases, UPS can be upregulated by pro-inflammatory cytokine interleukin-6 via the P44/42 mitogen-activated protein kinase (MAPK) pathway, or high levels of oxidative stress in the heart. In some cases, protein ubiquitination in atrophic hearts can comprise no difference compared with controls.

In some cases, chemotherapeutic agents can be associated with the development of heart failure in cancer survivors. In some cases, doxorubicin can be associated with dose dependent cardiotoxicity, which limits its clinical use and efficacy as a chemotherapy treatment. In some cases, doxorubicin-induced cardiomyopathy can be associated with reduction in heart weight, and the thinning of septal and LV posterior wall. In some cases, doxorubicin-induced cardiomyopathy can involve cardiac atrophy. In some cases, doxorubicin-induced cardiotoxicity can be associated with cardiomyocyte hypertrophy. In some cases, a response to doxorubicin varies depending on conditions and genetic predisposition, rather than representing a single pathological entity.

In some cases, use of a wearable or implantable interoperable electronic device can allow measurement of heart mass in a situation where direct measurement of heart mass in a clinical setting may not be possible. In some cases, use of a wearable or implantable interoperable electronic device can allow non-invasive estimates of LV mass. In some cases, non-invasive estimates of LV mass can be calculated most accurately using cardiac magnetic resonance imaging (cMRI).

In some cases, a subject presenting with anthracycline-associated cardiomyopathy can show a dose-related reduction in LV mass. In some cases, a dose-related reduction in LV mass can be in keeping with atrophic changes in the myocardium.

In some cases, cMRI measures of extracellular volume can be higher in asymptomatic patients three years after treatment with anthracyclines. In some cases, a reduction in cardiomyocyte density and increased fibrotic change can appear before an onset of cardiac dysfunction or heart failure symptoms in cancer patients treated with anthracycline chemotherapy.

In some cases, doxorubicin can induce cell death in cardiomyocytes. In some cases, cell death in cardiomyocytes can contribute to cardiac atrophy as a turnover of adult cardiomyocytes can be too limited to replace cell loss. In some cases, a molecular mechanism of doxorubicin-induced cardiotoxicity may comprise reactive oxygen species (ROS) generation and oxidative stress, topoisomerase-IIb (Top2b) inhibition, mitochondrial damage, or any combination thereof. In some cases, generation of excessive ROS can comprise redox cycling between quinone and semiquinone forms of doxorubicin. In some cases, ROS accumulation can cause deleterious effects. In some cases, deleterious effects can comprise lipid peroxidation, destruction of mitochondrial membranes, and DNA damage. In some cases, suppression of Top2b can protect against doxorubicin-induced cardiotoxicity.

In some cases, damage to mitochondrial structure and function can comprise early cardiotoxic effects of doxorubicin. In some cases, early cardiotoxic effects of doxorubicin have been observed in both animal and cell models. In some cases, mitochondrial dysfunction and damage can contribute to cardiac atrophy as mitochondrial content and function are important mediators of muscle size. In some cases, doxorubicin can bind irreversibly to cardiolipin in the inner mitochondrial membrane of cardiomyocytes, therefore concentrating doxorubicin in the mitochondria.

Mitochondria targeted antioxidants are shown to attenuate cardiotoxic effects of doxorubicin in rats, revealing the importance of mitochondrial ROS in doxorubicin-induced cardiotoxicity. In some cases, doxorubicin can induce cellular atrophy in cardiomyocytes by disrupting the balance of protein synthesis and degradation. In some cases, doxorubicin can induce protein ubiquitination and activates ubiquitin ligase atrogin-1 in cardiomyocytes via the p38-MAPK pathway.

In some cases, doxorubicin can induce autophagy in a heart, which in turn can increase apoptosis and decrease cardiomyocyte size. In some cases, doxorubicin can activate calpain in cardiac muscles. In some cases, calpain can degrade over 100 cellular proteins. In some cases, calpain can degrade key sarcomeric proteins. In some cases, a calpain specific inhibitor can comprise SJA. In some cases, SJA can attenuate doxorubicin induced cardiac wall thinning. In some cases, SJA can reduce caspase-3 activation and apoptosis. In some cases, cardiac dysfunction can be a physiological consequence of molecular changes of cardiac atrophy and fibrosis.

In some cases, a reduction in a quantity and quality of contractile cells can cause deterioration in systolic function. In some cases, cardiomyocytes can be replaced by fibrotic tissue. In some cases, a replacement of cardiomyocytes with fibrotic tissue can cause a deterioration of a diastolic relaxation of a heart. In some cases, identifying atrophic changes in a heart of a subject prior to an onset of cardiac dysfunction can enable earlier treatment. In some cases, identifying atrophic changes in a heart of a subject prior to an onset of cardiac dysfunction can improve outcomes. In some cases, endomyocardial biopsy and histological biopsy scoring can allow direct quantification of a degree of cardiomyocyte damage and cardiac atrophy. In some cases, quantification of a degree of cardiomyocyte damage and cardiac atrophy can correlate with a development of cardiac dysfunction as measured by invasive cardiac hemodynamics and can appear prior to functional deterioration when measured by echocardiogram.

In some cases, this can comprise an invasive test with significant risks and may not be justifiable or practical to perform as a screening test in asymptomatic patients. In some cases, measurement of LV ejection fraction (LVEF) by 2D echocardiography may be used to monitor deterioration in cardiac function following cancer. In some cases, measurement using an interoperable electronic device can comprise a more useful, more reproducible and more accurate measurement than 2D echocardiography.

In some cases, deterioration in ejection fraction can represent a late change in a development of cancer-induced cardiomyopathy. In some cases, deterioration in function may only occur after significant cellular damage and death has already occurred. In some cases, a method to improve sensitivity of echocardiography can comprise an improved echocardiography method. In some cases, an improved echocardiography method can comprise 3D echocardiography, dobutamine stress echo and global longitudinal strain by 2D echocardiography. In some cases, an improved cardiography method can comprise a more sensitive method for detecting deterioration in cardiac function in patients following cancer than 2D echocardiography.

In some cases, detection using an implantable cardioverter defibrillator (ICD), a wearable device, or an implantable loop recorder can comprise an advantage over echocardiography, improved echocardiography, or other techniques, as an ICD or implantable loop recorder can measure an early feature of cardiomyocyte damage and cell death.

In some cases, measuring an early feature of cardiomyocyte damage and cell death can result in an earlier diagnosis, earlier intervention, improved prognosis, improved outcomes, improved quality of life, improved life expectancy, amelioration of symptoms, or any combination thereof. In some cases, cardiac magnetic resonance imaging (cMRI) can have several benefits over echocardiography. In some cases, cMRI can provide an accurate and reproducible measure of LVEF36. In some cases, an accurate and reproducible measure of LVEF36 can allow earlier and more reliable detection of a deterioration in cardiac function.

In some cases, an accurate and reproducible measure of LVEF36 can allow measurement of several structural changes which can precede functional changes. In some cases, a particular measurement of T1-weighted early gadolinium signal enhancement in the myocardium and comparison with skeletal muscle signal intensity 3 days after anthracycline therapy can predict deterioration in LVEF at both 3 and 6 months.

In some cases, a measurement of myocardial edema with T2-weighted imaging can predict subsequent deterioration in LVEF following anthracycline treatment. In some cases, a method can comprise predicting a diagnosis of anthracycline-mediated cardiotoxicity and cause an expedited treatment of affected individuals.

In some cases, a measurement of cardiac biomarkers in cancer patients receiving chemotherapy can predict future reduction in symptoms. In some cases, detection of rises in cardiac troponin during and after anthracycline-containing chemotherapy can predict future reduction in LVEF and clinical cardiac events. In some cases, higher sensitivity troponin assays can detect rises in cardiac troponin in breast cancer patients receiving anthracycline and human epidermal growth factor receptor 2-targeted therapies. In some cases, higher sensitivity troponin assays can be combined with measurement of an inflammatory biomarker. In some cases, an inflammatory biomarker can comprise myeloperoxidase. In some cases, an increase in troponin can predict future decline in left ventricular function. In some cases, troponin rises during cardiotoxic chemotherapy can comprise an indicator of cardiac injury.

In some cases, measurement of cardiac biomarkers can be made of patients at diagnosis of a new malignancy, prior to cancer treatment. In some cases, detection of an elevated troponin or natriuretic peptide level in the blood of treatment-naive cancer patients can predict all-cause mortality. In some cases, cardiac stress may be secondary to an underlying malignancy, with cardiac atrophy a potential contributing factor.

### Role of protein kinases in cancer and cardiac dysfunction

In some cases, a cancer can comprise a mutation to a protein kinase (PKI). In some cases, a protein kinase can comprise a tyrosine kinase or tyrosine kinase receptor. In some cases, a tyrosine kinase can comprise Abl, growth factor receptors, EGFR, (ERBB1), HER2 (ERBB2), c-Kit, VEGFR, PDGFR, PI3K pathway, PI3K(p110α), PI3K(p110γ), Akt, PDK1, Ras/Raf/MEK/ERK pathway, Raf-1/B-Raf, MEK1/2, Cell cycle regulation, CDK, Aurora kinases, PLKs, mTOR, JAK2, or p38. In some cases, a tyrosine kinase receptor can comprise imatinib, imatinib D, imatinib N, bosutinib, gefitinib, lapatinib, XI,647, BIBW-2992, sunitinib, sorafenib pazopanib, vatalanib, vandetanib, cediranib, pazopanib, SF-1126, XL765, SF-1126, XL765, SF-1126, XL765, VQD-002, perifosine, UCN-01, sorafenib RAF-265, PD-0325901, AZD-6244, ARRY-162, alvocidib, BI2526, AZD-1152, BI2526, temsirolimus, everolimus, sirolimus, deforolimus, lestaurtinib, CP690550, TG101348, or GW856553. In some cases, a tyrosine kinase inhibitor (TKI) can cause a cardiotoxic effect in a subject. In some cases, a cardiotoxic effect can comprise congestive heart failure. In some cases, a tyrosine kinase inhibitor can comprise imatinib. In some cases, a cardiotoxic effect can be detected by measurement of LV ejection fractions. In some cases, a cardiotoxic effect can be detected by measurement of a biomarker. In some cases, a biomarker can comprise troponin I. In some cases, a biomarker can comprise troponin C. In some cases, a biomarker can comprise troponin T. In some cases, a cancer can cause an increase in cardiac mass. In some cases, a cardiac mass can comprise ventricular mass. In some cases, ventricular mass can comprise left ventricular mass. In some cases, a cardiotoxic effect can comprise left ventricular dysfunction. In some cases, administration of sunitinib to a subject can cause a cardiotoxic effect. In some cases, administration of imatinib can cause a cardiotoxic effect.

### Hypertrophic growth in cancer

In some cases, cancers can be associated with excess growth signals. In some cases, excess growth signals can be to a heart muscle. In some cases, cancer can at least partially cause a cardiac mass change. In some cases, a cardiac mass change can be an increase. In some cases, a cardiac mass change can be a loss. In some cases, a cardiac mass increase can comprise left ventricular (LV) mass. In some cases, cardiac mass can comprise right ventricular (RV) mass. In some cases, cardiac mass can comprise atrial mass. In some cases, atrial mass can comprise left atrial mass. In some cases, atrial mass can comprise right atrial mass. In some cases, cardiac mass can comprise aorta or vena cava mass. In some cases, cancer can at least partially cause a left ventricular mass loss. In some cases, cancer can at least partially cause a left ventricular mass increase. In some cases, a change in cardiac mass can at least partially be caused by administration of a therapeutic such as described herein. In some cases, a therapeutic can comprise a targeted therapeutic. In some cases, a targeted therapeutic can target a specific protein kinase. In some cases, a specific protein kinase can be dysregulated in cancer. In some cases, a change in cardiac mass can be associated with a cardiopulmonary measurement as described herein. In some cases, a cardiopulmonary measurement can comprise non-sustained ventricular tachycardia (nsVT). In some cases, Chronic Obstructive Pulmonary Disease (COPD) can be associated with COPD exacerbations. In some cases, a patient with COPD exacerbations can have more frequent non-sustained ventricular tachycardia. In some cases, a detection of COPD can comprise a measurement of more frequent non-sustained ventricular tachycardia as described herein. In some cases, COPD can be associated with a left ventricular mass increase.

In some cases, a change in cardiac mass can predict incident cardiovascular events. In some cases, of mild to moderate COPD, hyperinflation can be associated with an increase in LV mass. In some cases, an increase in LV mass can comprise an about 5% increase in LV mass per 1-SD increase in residual volume. In some cases, an increase in LV mass can be associated with an about 6% increase in all-cause mortality. In some cases, an increase in LV mass can be associated with an about 7% increase in risk of cardiovascular death. In some cases, an increase in LV mass can be associated with an about 20% increase in risk of incident heart failure. In some cases, COPD can increase a risk of sudden death. In some cases, COPD can increase a risk of heart failure. In some cases, increased LV mass in COPD can contribute in part to a risk of sudden death or heart failure. In some cases, a measure of hyperinflation can be associated with an LV mass to end-diastolic volume ratio. In some cases, a metric of concentric LV remodeling can predict incident cardiovascular events. In some cases, a metric of concentric LV remodeling can be associated with heart failure with preserved ejection fraction. In some cases, heart failure with preserved ejection fraction can be associated with COPD. In some cases, a COPD induced increase in LV mass can increase a risk of sudden death. In some cases, COPD can be associated with a cardiopulmonary measurement as described herein. In some cases, detection of a cardiopulmonary measurement as described herein can predict a presence of COPD. In some cases, detection of a cardiopulmonary measurement as described herein can predict a risk of developing COPD. In some cases, COPD can be associated with non-sustained ventricular tachycardia.

### Degenerative cardiomyopathy

Disclosed herein are methods of detecting cardiac wasting. In some cases, cardiac wasting can comprise cancer related cardiac wasting. In some cases, a method of detecting cardiac wasting can comprise a non-invasive method. In some cases, a method of detecting cancer related cardiac wasting can comprise an earlier detection compared to other methods. In some cases, other methods can comprise echocardiography. In some cases, other methods can comprise imaging methodologies. In some cases, other methods can comprise biomarker methodologies. In some cases, other methods can comprise enhance echocardiography methods. In some cases, an earlier detection can lead to: an earlier diagnosis, improved diagnosis, a more accurate diagnosis, an earlier initiation of treatment, a better prognosis, better clinical outcomes, improved chance of survival, improved quality of life, or any combination thereof. In some cases, cardiac wasting can be predicted by detecting aberrant patterns of cardiopulmonary signals. In some cases, treating cardiac wasting before an onset of cardiac wasting detectable by echocardiography can be beneficial. In some cases, treating cardiac wasting in an absence of myocardial damage or dysfunctional can be beneficial.

In some cases, a development of global cardiac muscle wasting in advanced cancer can lead to a more advanced type of cardiomyopathy. In some cases, an advanced type of cardiomyopathy can comprise degenerative cardiomyopathy. In some cases, specific cellular wasting processes can affect a state and function of electrical cells and pathways of a heart. In some cases, cellular wasting processes affecting a state and function of electrical cells and pathways of a heart can lead to significant arrhythmias.

In some cases, death due to cardiovascular illness can comprise the 3rd most frequent cause of death after multi-organ failure and sepsis. In some cases, 15-30% of patients with cancer can die due to cardiovascular causes. In some cases, cancer patients can suffer from heart failure like signs and symptoms. In some cases, symptoms can comprise congestion, shortness of breath, greatly reduced functional capacity, or any combination thereof. In some cases, patients can frequently die suddenly.

In some cases, advanced cancer can comprise a cardiovascular disease. In some cases, advance cancer can comprise a development of (i) a clinical heart failure syndrome, and (ii) of significant and clinically relevant arrhythmias in such patients. In some cases, developments can occur independent of known cardiotoxic effects of anti-cancer therapies. In some cases, developments can occur in addition to known cardiotoxic effects of anti-cancer therapies. In some cases, a pathophysiologic link between cancer and heart failure can be two-fold.

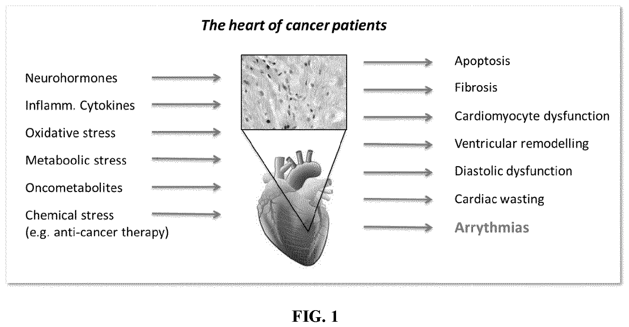

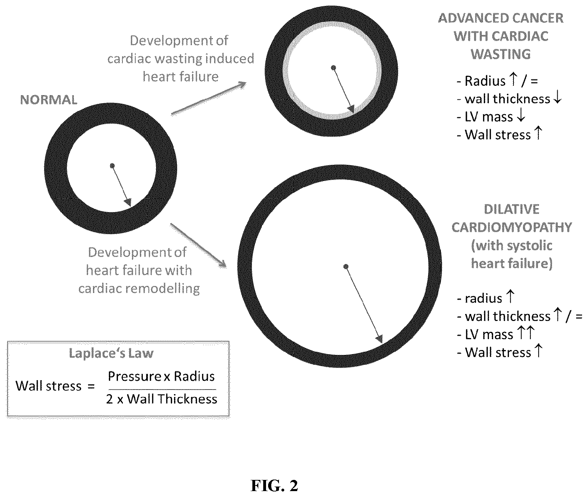

In some cases, cardiac wasting can comprise LV wall mass thinning. In some cases, cardiac wasting can lead to increases in LV wall stress. In some cases, cardiac wasting can lead to increases in LV wall stress, even in an absence of ventricular dilatation. In some cases, the relation between cardiac wasting and LV wall stress can follow Laplace's law. In some cases, disease associations as described herein can be missed, as they can occur in very late-stage cancer. In some cases, in very late-stage cancer a patient can be in palliative or hospice care, and detailed cardiac investigations may never be performed. In some cases, cancer diseases can cause tissue inflammation, oxidative stress, local neurohormonal activation, or any combination thereof. In some cases, local neurohormonal activation can comprise tissue homeostasis changes. In some cases, tissue inflammation, oxidative stress, local neurohormonal activation, or any combination thereof can lead to cardiac wasting. In some cases, cardiac wasting can comprise fibrosis, apoptosis, and a combination thereof. In some cases, alterations described herein can lead to impaired cardiac function. In some cases, alterations described herein can result in significant arrhythmias. In some cases, cardiac wasting can occur in advanced cancer. In some cases, changes in an interstitial cardiac milieu resulting in myocardial cell death may be an important substrate for arrhythmia development in cancer patients. In some cases, onco-metabolites can cause cardiac dysfunction. In some cases, such mechanisms as described herein can trigger cardiac arrhythmias in patients suffering from malignant carcinomas. In some cases, such mechanisms as described herein can influence a global strain independent of chemotherapy as shown in **FIG. 1** and **FIG. 2**. In some cases, therapeutic options for an arrythmia component can require a thoughtful ethical assessment involving patients as early as possible. In some cases, a cardiac wasting-based heart failure aspect can have strong implications for new quality of life - focused therapeutic approaches in end-stage cancer.

### Correlation between cancer cachexia and cardiac wasting

In some cases, cancer cachexia (CC) can be defined based on clinical and/or pathological diagnosis, body mass index (BMI) <20.0 kg/m2, the presence or absence of edema, body weight loss of 5.0% during the previous year or less, or any combination thereof. In some cases, cancer cachexia diagnosis can comprise measurement of BMI, heart weight (HW), left and right ventricular wall thicknesses (LVWT and RVWT, respectively), or any combination thereof. In some cases, a recording of biochemical parameters and medication data can allow detection of cancer cachexia. In some cases, individuals with CC can have a significantly lower HW than non-cachectic subjects. BMI correlated with HW in cases with GI cancer. In some cases, cancer patients can demonstrate slightly lower left ventricular ejection fraction than control subjects. In some cases, a slightly lower left ventricular ejection fraction in cancer patients can comprise an LVEF, 66±5% and a range of 42-78%. In some cases, a left ventricular ejection fraction in control subjects can comprise 69±4%, range 62-79%, p<0.006. In some cases, a lower left ventricular ejection fraction can comprise a lower E/A ratio. In some cases, a lower E/A ratio can comprise (1.1±0.4 vs 1.2±0.3, p=0.036). In some cases, cancer patients can comprise a higher E/e', and higher estimated pulmonary artery systolic pressure (PASP,29±9 vs 25±4mmHg, p=0.027) than controls. In some cases, 96% of cancer patients and 100% of controls can have an LVEF=55%. In some cases, 45% of cancer patients can demonstrate diastolic dysfunction grade 1 (E/A<1.0), 30% grade 2 (E/e'=8 with E/A=1), 1% grade 3 (E/e'=15). In some cases, 24% can have no diastolic dysfunction. In some cases, in controls a distribution can comprise 15, 19, 0, and 66%, respectively. In some cases, an interventricular septal and posterior wall thickness can be significantly elevated in cancer patients vs controls (10.4±1.7 vs 9.6±1.2 mm, p=0.022; 10.2±1.5 vs 9.6±1.1 mm, p=0.038). In some cases, subgroup analyses of treatment naive cancer patients vs healthy controls can show increased E/e' (9±4 vs 7±2; p=0.015), PASP (30±8 vs 25±4 mmHg; p=0.016), interventricular septal thickness (10.8±2.0 vs 9.6±1.2mm; p=0.0073), and posterior wall thickness (10.5±1.7 vs 9.6±1.1mm; p=0.015). In some cases, an LVEF (67±4 vs 69±4%; p=0.12) and E/A ratio (1.2±0.5 vs 1.3±0.3; p=0.50). In some cases, a subgroup analysis of patients with prior chemotherapy known to be cardiotoxic and/or radiotherapy compared to treatment naive cancer patients can show lower LVEF, lower interventricular septal thickness, a tendency to lower posterior wall thickness, or any combination thereof.

### VI. Treating a subject

Also disclosed herein in some embodiments are methods of treating a subject after a cardiopulmonary measurement has been taken as disclosed herein. In some cases, a method can comprise contacting a cardioverter defibrillator with a subject in need thereof. In some cases, a decision to contact a cardioverter defibrillator with a subject in need thereof can be based at least in part on a detection of a cardiopulmonary measurement as described herein. In some cases, a cardioverter defibrillator can be actuated while in contact with a subject in need thereof. In some cases, actuating a cardioverter defibrillator that is directly or indirectly contacted with a subject can prevent or treat cardiac wasting in a subject.

In some cases, a medicament can be administered to a subject. In some cases, a decision to administer a medicament can be based on a detection of a cardiopulmonary measurement as described herein. In some cases, a medicament can comprise a drug, or a salt, polymorph, solvate, isomer, stereoisomer, prodrug, or metabolite thereof. A drug can be licensed or approved by a regulatory agency. In some cases, a medicament may not be licensed or approved by a regulatory agency. In some cases, a subject can suffer from a disease condition as described herein. In some embodiments, a subject can suffer from cancer, be suspected of being at risk of developing cancer, have cancer cachexia, have cancer associated cardiac wasting, be at risk of developing cardiac wasting, or any combination thereof.

In some embodiments, medicament can be administered orally, rectally, or parenterally, in formulations containing conventionally acceptable carriers, adjuvants, and vehicles as desired. The term "parenteral" as used herein can include subcutaneous, intravenous, intramuscular, or intrasternal injection and infusion techniques. Administration can include injection or infusion, including intra-arterial, intracardiac, intracerebroventricular, intradermal, intraduodenal, intramedullary, intramuscular, intraosseous, intraperitoneal, intrathecal, intravascular, intravenous, intravitreal, epidural and subcutaneous), inhalational, transdermal, transmucosal, sublingual, buccal and topical (including epicutaneous, dermal, enema, eye drops, ear drops, intranasal, vaginal) administration. In some exemplary embodiments, a route of administration can be via an injection such as an intramuscular, intravenous, subcutaneous, or intraperitoneal injection.

In some cases, a medicament can be present as a pharmaceutical composition in a form suitable for administration. Solid dosage forms for oral administration can include capsules, tablets, caplets, pills, troches, lozenges, powders, and granules. A capsule can comprise a core material comprising a nutritive protein or composition and a shell wall that encapsulates a core material. In some embodiments a core material can comprise at least one of a solid, a liquid, and an emulsion. In some embodiments a shell wall material can comprise at least one of a soft gelatin, a hard gelatin, and a polymer. Suitable polymers can include but not limited to: cellulosic polymers such as hydroxypropyl cellulose, hydroxyethyl cellulose, hydroxypropyl methyl cellulose (HPMC), methyl cellulose, ethyl cellulose, cellulose acetate, cellulose acetate phthalate, cellulose acetate trimellitate, hydroxypropylmethyl cellulose phthalate, hydroxypropylmethyl cellulose succinate and carboxymethylcellulose sodium; acrylic acid polymers and copolymers, such as those formed from acrylic acid, methacrylic acid, methyl acrylate, ammonio methylacrylate, ethyl acrylate, methyl methacrylate and/or ethyl methacrylate (e.g., those copolymers sold under the trade name "Eudragit"); vinyl polymers and copolymers such as polyvinyl pyrrolidone, polyvinyl acetate, polyvinylacetate phthalate, vinylacetate crotonic acid copolymer, and ethylene-vinyl acetate copolymers; and shellac (purified lac). In some embodiments at least one polymer can function as taste-masking agents.

In some embodiments, tablets, pills, and the like can be compressed, multiply compressed, multiply layered, and/or coated. A coating can be single or multiple. In some embodiments, a coating material can comprise at least one of a saccharide, a polysaccharide, and glycoproteins extracted from at least one of a plant, a fungus, and a microbe. Non-limiting examples can include corn starch, wheat starch, potato starch, tapioca starch, cellulose, hemicellulose, dextrans, maltodextrin, cyclodextrins, inulins, pectin, mannans, gum arabic, locust bean gum, mesquite gum, guar gum, gum karaya, gum ghatti, tragacanth gum, funori, carrageenans, agar, alginates, chitosans, or gellan gum. In some embodiments a coating material can comprise a protein. In some embodiments, a coating material can comprise at least one of a fat and/or an oil. In some embodiments the at least one of a fat and/or an oil can be high temperature melting. In some embodiments the at least one of a fat and/or an oil can be hydrogenated or partially hydrogenated. In some embodiments the at least one of a fat and/or an oil can be derived from a plant. In some embodiments the at least one of a fat and/or an oil can comprise at least one of glycerides, free fatty acids, and fatty acid esters. In some embodiments a coating material can comprise at least one edible wax. An edible wax can be derived from animals, insects, or plants. Non-limiting examples can include beeswax, lanolin, bayberry wax, carnauba wax, and rice bran wax. Tablets and pills can additionally be prepared with enteric coatings.

In some embodiments, liquid formulations can include a syrup (for example, an oral formulation), an intravenous formulation, an intranasal formulation, an ocular formulation (e.g. for treating an eye infection), an otic formulation (e.g. for treating an ear infection), an ointment, a cream, an aerosol, and the like. In some instances, a combination of various formulations can be administered. In some embodiments, a tablet, pill, and the like can be formulated for an extended release profile.

In some instances, a medicament can be administered in a composition for topical administration. For topical administration, an active agent may be formulated as is known in the art for direct application to a target area. Forms chiefly conditioned for topical application can take the form, for example, of creams, milks, gels, powders, dispersion or microemulsions, lotions thickened to a greater or lesser extent, impregnated pads, ointments or sticks, aerosol formulations (e.g. sprays or foams), hydrogel, soaps, detergents, lotions or cakes of soap. Other conventional forms for this purpose include wound dressings, coated bandages or other polymer coverings, ointments, creams, lotions, pastes, jellies, sprays, and aerosols. Thus, a medicament disclosed herein can be delivered via patches or bandages for dermal administration. Alternatively, a medicament can be formulated to be part of an adhesive polymer, such as polyacrylate or acrylate/vinyl acetate copolymer. For long-term applications it might be desirable to use microporous and/or breathable backing laminates, so hydration or maceration of a skin can be minimized. A backing layer can be any appropriate thickness that will provide a desired protective and support functions. A suitable thickness will generally be from about 1 to about 1000 microns. For example, form about 10 to about 300 microns. Topical administration may be in the form of a nail coating or lacquer. For example, a medicament can be formulated in a solution for topical administration that contains ethyl acetate (NF), isopropyl alcohol (USP), and butyl monoester of poly[methylvinyl ether/maleic acid] in isopropyl alcohol.

In some embodiments, drops, such as eye drops or nose drops, may be formulated with a medicament in an aqueous or non-aqueous base also comprising one or more dispersing agents, solubilizing agents or suspending agents. Liquid sprays can be pumped or are conveniently delivered from pressurized packs. Drops can be delivered via a simple eye dropper-capped bottle, via a plastic bottle adapted to deliver liquid contents drop-wise, or via a specially shaped closure.

In some embodiments, ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

In some embodiments, an aerosol can be employed to administer a medicament to a respiratory tract. For administration by inhalation or insufflation, a composition may take the form of a dry powder, for example, a powder mix of a therapeutic agent and a suitable powder base such as lactose or starch. A medicament can also be administered in an aqueous solution when administered in an aerosol or inhaled form. An inhalable formulation can be an inhalable respiratory formulation. Thus, other aerosol pharmaceutical formulations may comprise, for example, a physiologically acceptable buffered saline solution containing from about 0.001 mg/ml to about 100 mg/ml for example from about 0.1 to about 100 mg/ml, such as 0.5-50 mg/ml, 0.5-20 mg/ml, 0.5-10 mg/ml, 0.5-5 mg/ml or 1-5 mg/ml of a medicament specific for an indication or disease to be treated.

In some embodiments, administration of a medicament to a subject can be used to at least partially ameliorate a condition as described herein. Administration of a medicament can be performed for a treatment duration of at least about at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 days consecutive or nonconsecutive days. In some cases, a treatment duration can be from about 1 to about 30 days, from about 2 to about 30 days, from about 3 to about 30 days, from about 4 to about 30 days, from about 5 to about 30 days, from about 6 to about 30 days, from about 7 to about 30 days, from about 8 to about 30 days, from about 9 to about 30 days, from about 10 to about 30 days, from about 11 to about 30 days, from about 12 to about 30 days, from about 13 to about 30 days, from about 14 to about 30 days, from about 15 to about 30 days, from about 16 to about 30 days, from about 17 to about 30 days, from about 18 to about 30 days, from about 19 to about 30 days, from about 20 to about 30 days, from about 21 to about 30 days, from about 22 to about 30 days, from about 23 to about 30 days, from about 24 to about 30 days, from about 25 to about 30 days, from about 26 to about 30 days, from about 27 to about 30 days, from about 28 to about 30 days, or from about 29 to about 30 days.

In some embodiments, administration of a medicament can be performed at least 1, 2, 3, 4, 5, 6, 7, 8, 9, 10, 11, 12, 13, 14, 15, 16, 17, 18, 19, 20, 21, 22, 23, or 24 times a day. In some cases, administration of a medicament can be performed at least 1, 2, 3, 4, 5, 6, 7, 8, 9, 10, 11, 12, 13, 14, 15, 16, 17, 18, 19, 20, or 21 times a week. In some cases, administration of a medicament can be performed at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 times a month.

In some cases, a medicament can comprise an anticoagulant, an antiplatelet agent, an angiotensin-converting enzyme (ACE) inhibitor, an angiotensin II receptor blocker or inhibitor, an angiotensin receptor-neprilysin inhibitor (ARNI), an I*f* channel blocker, a Hydralazine and isosorbide dinitrate, a beta receptor blocker, an alpha receptor blocker, a calcium channel blocker, an aldosterone antagonist, a calcium channel blocker, a cholesterol-lowering medication, a digitalis preparation, a diuretic, a vasodilator, a mineralocorticoid receptor antagonist, an aldosterone synthesis inhibitor, cardioxyl, omecamtiv mecarbil, relaxin, serelaxin, staroxime, bucindolol, a phosphodiesterase 5 inhibitor, a vasopressin inhibitor, levosimendan, a sodium-glucose cotransporter-2 (SGLT2) inhibitor, an If channel blocker, a beta-3 receptor agonist, a beta-3 receptor antagonist, a vasopressin antagonist, a neutral endo-peptidase inhibitor, a dual vasopeptidase inhibitor, a metabolism-modifying agent, a soluble guanylate cyclase stimulator, a soluble guanylate cyclase activator, an iron therapy medicament, a soluble guanylate cyclase stimulator, an aldosterone receptor antagonist, a stereoisomer of any of these, a salt of any of these, or any combination thereof.

In some cases, an anticoagulant can comprise an apixaban, a dabigatran, an edoxaban, a heparin, a rivaroxaban, a warfarin, a stereoisomer of any of these, a salt of any of these, or any combination thereof.

In some cases, an antiplatelet agent can comprise an aspirin, a clopidogrel, a dipyridamole, a prasugrel, a ticagrelor, a stereoisomer of any of these, a salt of any of these, or any combination thereof.

In some cases, an angiotensin-converting enzyme (ACE) inhibitor can comprise a benazepril, a captopril, an enalapril, a fosinopril, a lisinopril, a moexipril, a perindopril, a quinapril, a ramipril, a trandolapril, a stereoisomer of any of these, a salt of any of these, or any combination thereof.

In some cases, an angiotensin II receptor blocker or inhibitor can comprise an azilsartan, a candesartan, an eprosartan, an irbesartan, a losartan, an olmesartan, a telmisartan, a valsartan, a stereoisomer of any of these, a salt of any of these, or any combination thereof.

In some cases, an angiotensin receptor-neprilysin inhibitor (ARNI) can comprise a sacubitril, a valsartan, a stereoisomer of any of these, a salt of any of these, or any combination thereof.

In some cases, an I*f* channel blocker, an ivabradine a stereoisomer thereof, a salt of any of these, or any combination thereof.

In some cases, a Hydralazine and isosorbide dinitrate, a stereoisomer of any of these, a salt of any of these, or any combination thereof.

In some cases, a beta-blocker can comprise S-pindolol, S-oxprenolol, S-atenolol, acebutolol, atenolol, betaxolol bisoprolol, metoprolol, nadolol, nebivolol, propranolol, sotalolol, a stereoisomer of any of these, a salt of any of these, or any combination thereof.

In some cases, a calcium channel blocker can comprise an amlodipine, a diltiazem, felodipine, nifedipine, a nimodipine, a nisoldipine, a verapamil, a stereoisomer of any of these, a salt of any of these, or any combination thereof.

In some cases, a phosphodiesterase 5 inhibitor can comprise amrinone, milrinone, avanafil, lodenafil, mirodenafil, sildenafil, tadalafil, vardenafil, udenafil, zaprinast, benzaminenafil, carvedilol, a stereoisomer of any of these, or a salt of any of these, or any combination thereof. In some cases, a vasopressin inhibitor can comprise tolvaptan, a stereoisomer of any of these, a salt of any of these, or any combination thereof.

In some cases, a SGLT2 inhibitor can comprise dapagliflozin, empagliflozin, canagliflozin, sotagliflozin, ertugliflozin, ipragliflozin, luseogliflozin, remogliflozin etabonate, sergliflozin etabonate, tofogliflozin a stereoisomer of any of these, a salt of any of these, or any combination thereof.

In some cases, an aldosterone antagonist or salt thereof can comprise a spironolactone, an eplerenone, a finerenone, an esaxerenone, an apararenone, a stereoisomer of any of these, a salt of any of these, or any combination thereof.

In some cases, a cholesterol-lowering medication, a statin, a nicotinic acid, a cholesterol absorption inhibitor, a stereoisomer of any of these, a salt of any of these, or any combination thereof. In some embodiments, a statin can comprise an atorvastatin, a fluvastatin, a lovastatin, a pitavastatin, a pravastatin, a rosuvastatin, a simvastatin, a stereoisomer of any of these, a salt of any of these, or any combination thereof. In some embodiments, a nicotinic acid can comprise niacin, a stereoisomer thereof, a salt of either of these, or any combination thereof. In some embodiments, a cholesterol absorption inhibitor can comprise ezetimibe, a stereoisomer thereof, a salt of either of these, or any combination thereof. In some embodiments, a combination statin and cholesterol absorption inhibitor can comprise ezetimibe and simvastatin, a stereoisomer of either of these, a salt of either of these, or any combination thereof.

In some cases, a digitalis preparation, can comprise digoxin, a stereoisomer thereof, a salt of either of these, or any combination thereof.

In some cases, a diuretic, can comprise an acetazolamide, an amiloride, a bumetanide, a chlorothiazide, a chlorthalidone, a furosemide, a hydro-chlorothiazide, an indapamide, a metalozone, a spironolactone, a torsemide, a stereoisomer of any of these, a salt of any of these, or any combination thereof.

In some cases, a vasodilator can comprise an isosorbide dinitrate, an isosorbide mononitrate, a hydralazine, a nitroglycerin, a minoxidil, a stereoisomer of any of these, a salt of any of these, or any combination thereof.

In some cases, a mineralocorticoid receptor antagonist can comprise eplerenone, spironolactone, finerenone, potassium canrenoate, spironolactone, a stereoisomer of any of these, a salt of any of these, or any combination thereof.

In some cases, an aldosterone synthesis inhibitor can comprise fadrozol, FAD 286, LCI699, a stereoisomer of any of these, a salt of any of these, or any combination thereof.

In some cases, an angiotensin receptor antagonist or a salt thereof can comprise a sartan, candesartan, irbesatan, valsartan, telmisartan, eprosartan, olmesartan, azilsartan, fimasartan, sacubitril/valsartan, losartan, EXP 3174, amlodipine, a stereoisomer of any of these, a salt of any of these, or any combination thereof.

In some cases, an If channel blocker or a salt thereof can comprise ivabradine, zatebradine, cilobradine, ZD7288, alinidine, a salt of any of these, or any combination thereof.

In some cases, a soluble guanylate cyclase stimulator or a salt thereof can comprise BAY 41-8543, BAY 41-2272, Riociguat, vericiguat, or any combination thereof.

In some cases, an iron therapy medicament can comprise an oral iron, an injectable iron, an intravenous preparation, an IV ferric carboxymaltose, an iron sucrose, an iron isomaltose, an iron, a stereoisomer of any of these, a salt of any of these, or any combination thereof.

In some cases, a soluble guanylate cyclase stimulator can comprise a BAY 41-8543, a BAY 41-2272, a riociguat, a vericiguat, or any combination thereof.

In some cases, an aldosterone receptor antagonist can comprise spironolactone, eplerenone, finerenone, esaxerenone, apararenone, a stereoisomer of any of these, a salt of any of these, or any combination thereof.

In some cases, a cardioverter defibrillator can comprise an implantable cardioverter defibrillator. In some cases, a cardioverter defibrillator can comprise a wearable cardioverter defibrillator. In some cases, a cardioverter defibrillator can be applied or held in proximity to a subject's skin.

In some cases, a medicament can be a metabolism-modifying agent. In some cases, a metabolism-modifying agent can include elamipretide, trimetazidine, perhexiline, etomoxir, dichloroacetate, coenzyme Q10, ubiquinol, nicotinamide riboside, metformin, resveratrol, pterostilbene, rapamycin, a salt of any of these, or any combination thereof.

### VII. Device

Also disclosed herein in some embodiments is an interoperable electronic device that can be configured to measure a cardiopulmonary measurement. In some cases, an interoperable electronic device can comprise a cardiac resynchronization device (CRT). In some cases, an interoperable electronic device can comprise an automated cardiac defibrillator (ICD). In some cases, an interoperable electronic device can comprise a cardiac pacemaker. In some cases, an interoperable electronic device can be connected to a heart. In some cases, an interoperable electronic device can regulate the beating of a heart to which it is connected. In some cases an interoperable electronic device can comprise a wearable device.

In some cases, an interoperable electronic device can interpret a risk of a presence of a disease. In some cases, an interoperable electronic device can comprise an algorithm stored on computer readable memory operatively coupled to a device.

In some cases, an interoperable electronic device can transmit a detection of a risk of disease to a second device. In some cases, an interoperable electronic device can transmit information about detection of a disease to a second device by a wired or wireless transmission.

Also disclosed herein in some embodiments is a kit that can comprise an interoperable electronic device as disclosed herein at least partially in a packaging. In some cases, a kit can further comprise instructions to assess a risk of a disease by recording a cardiopulmonary measurement using an interoperable electronic device.

Disclosed herein in some embodiments, are methods of treating a patient with a second device. In some embodiments, a second device can be a device configured to alter an autonomic balance, a device configured to alter an abnormal intracardiac pressure, or a combination thereof. Disclosed herein in some embodiments, are a device configured to alter an autonomic balance, a device configured to alter an abnormal intracardiac pressure, or a combination thereof. In some embodiments, a subject can be treated using a device configured to alter an autonomic balance, a device configured to alter an abnormal intracardiac pressure, or a combination thereof, as disclosed herein. In some embodiments, an autonomic balance can comprise a baroflex stimulator, a chemoflex inhibition, a sympathetic nerve ablation, a renal artery nerve ablation, phrenic nerve stimulation, a vagal nerve stimulation, arterial wall electrical stimulation, a cardiac plexus nerve stimulator, or any combination thereof. In some embodiments, a device configured to alter an abnormal intracardiac pressure can comprise an inter-atrial decompression device, an intracardiac stent, an intra-cardiac conduit, a tissue ablation device, or any combination thereof.

### VIII. Disease monitoring

Also disclosed herein in some embodiments is a method of monitoring a disease in a subject. In some cases, monitoring a disease can comprise recording a cardiopulmonary measurement in a subject. In some cases, a cardiopulmonary measurement can comprise using an interoperable electronic device as disclosed herein. In some cases, a cardiopulmonary measurement can be compared to a reference level. In some cases, measurements and comparisons can be performed repeatedly. In some cases, monitoring can be used to determine a prognosis for a patient.

In some cases, monitoring can be used to determine efficacy of a treatment. In some cases, determining an efficacy of a treatment can comprise making one or more measurements over a time period. In some cases, one or more measurements can be compared to previously recorded measurements, a reference value, or a combination thereof. In some cases, a value of a recorded measurement can decrease over time. In some cases, a value of a recorded measurement can increase over time. In some cases, a measurement value not decreasing over a time period can be used to determine at least in part if a treatment is effective. In some cases, a measurement value not increasing over a time period can be used to determine at least in part if a treatment is effective. In some cases, a measurement value not staying constant over a time period can be used to determine at least in part if a treatment is effective. In some cases, a decrease or increase in a measurement value over a time period can be used to determine whether to continue, modify, change, or cease treatment.

In some cases, a time period can comprise about 1 day, about 2 days, about 3 days, about 4 days, about 5 days, about 6 days, about 7 days, about two weeks, about three weeks, about one month, about two months, about three months, about four months, about five months, about six months, about seven months, about eight months, about nine months, about ten months, about eleven months, about one year, about two years, or more than about two years after a first cardiopulmonary treatment.

In some cases, a detection of a cardiopulmonary measurement can be used to determine a prognosis for a disease. In some cases, detection of a cardiopulmonary measurement can be comprised with a further test. In some cases, a further test can comprise a cardiac test. In some cases, a further test can comprise measurement of a biomarker. In some cases, a biomarker can comprise low density lipoprotein (LDL) cholesterol. In some cases, a biomarker can comprise an inflammation biomarker, an oxidative stress biomarker, a metabolic biomarker or any combination thereof. In some cases, an inflammation biomarker can comprise C-reactive protein, interleukin 6, Fibrinogen, monocyte chemotactic protein-1, tumor necrosis factor alpha, or any combination thereof. In some cases, an oxidative stress biomarker can comprise an isoprostane. In some cases, a metabolic biomarker can comprise lipoprotein (a), low-density lipoproteins, high density lipoprotein, ApoB 100, Lipoprotein-associated phospholipase A2, homocysteine, vitamin D, fibroblast growth factor 23, adiponectin, glycated hemoglobin, haptoglobin, or any combination thereof. In some cases, a biomarker can comprise a natriuretic peptide. In some cases, a natriuretic peptide can comprise a circulating natriuretic peptide. In some cases, a natriuretic peptide can comprise atrial natriuretic peptide (ANP), brain-type natriuretic peptide (BNP), neural endopeptidase (neprilysin), or any combination thereof. In some cases, a biomarker can comprise a genetic biomarker. In some cases, a biomarker can comprise an epigenetic biomarker.

In some cases, a method of monitoring can comprise monitoring a wasting of muscle mass in a subject. In some cases, a muscle mass can comprise cardiac muscle mass. In some cases, cardiac muscle mass can comprise ventricular muscle mass. In some cases, ventricular muscle mass can comprise left ventricular muscle mass. In some cases, monitoring wasting of muscle mass can comprise recording a cardiopulmonary measurement in a subject using an interoperable electronic device as disclosed herein, comparing a cardiopulmonary measurement to a reference level, and repeating the previous steps.

In some cases, monitoring can be used to determine efficacy of an anti-cancer therapeutic in a subject administered an anti-cancer therapeutic. In some cases, monitoring can comprise recording a cardiopulmonary measurement in a subject using an interoperable electronic device as disclosed herein, comparing a cardiopulmonary measurement to a reference level, and repeating the previous steps for as long as a cardiopulmonary measurement remains higher than a reference level. In some cases, information from monitoring can be used to determine whether to terminate administering of an anti-cancer therapeutic, continue administering of an anti-cancer therapeutic, or recommending a change in an anti-cancer therapeutic if a cardiopulmonary measurement remains higher than a reference level for a time period of at least about a month after a first cardiopulmonary recording.

Also disclosed herein in some embodiments is a method of determining a cancer prognosis in a subject that can comprise: recording a number of premature ventricular contractions (PVCs) in a 24 hour time period in a subject using an electronic device configured to monitor PVCs, repeating a recording, and delivering a prognosis to a subject, wherein a subject has a more favorable prognosis if a subject displays a number of PVCs that can comprise less than 10 or greater than 100, relative to a subject that displays a number of PVCs of from about 10 to about 100.

In some cases, determining a cancer prognosis can further comprise conducting a cancer diagnostic test, wherein a cancer diagnostic test determines a level of a biomarker. In some cases, a biomarker can comprise a genetic biomarker. In some cases, a biomarker can comprise an epigenetic or genetic biomarker. In some cases, determining can comprise sequencing a sample from a subject. In some cases, sequencing can comprise next generation sequencing. In some cases, next generation sequencing can comprise sequencing by synthesis, sequencing by ligation, sequencing by amplification, or any combination thereof. In some cases, sequencing can comprise generating a DNA or RNA library from nucleic acids. In some cases, generating a DNA or RNA library can comprise fragmenting nucleic acids and adding adaptors. In some cases, sequencing reads can be aligned to a reference genome to create a contig. In some cases, variants can be called by comparing differences in a contig sequence from a reference sequence. In some cases, variants can be determined to be pathogenic or disease associated. In some cases, a detection of a pathogenic or disease associated variant can be used to inform a clinical diagnosis, prognosis, or choice of treatment. In some cases, sequencing can be performed on a cell free nucleic acid sample from a subject. In some cases, a determining can comprise imaging. In some cases, a level of a biomarker can be greater than a reference level. In some cases, a level of a biomarker can be less than a reference level.

Also disclosed herein in some embodiments is a method of determining efficacy of a cardiac-active therapeutic in a subject administered a cardiac-active therapeutic that can comprise: recording a cardiopulmonary measurement in a subject using an interoperable electronic device as disclosed herein, comparing a cardiopulmonary measurement to a reference level, repeating these steps for as long as a cardiopulmonary measurement remains higher than a reference level, and performing one of the following: termination of administering a cardiac-active therapeutic; continuing administering a cardiac-active therapeutic; or recommending a change in a cardiac-active therapeutic if a cardiopulmonary measurement remains higher than a reference level for a time period of at least about a month after a first recording of a cardiopulmonary measurement.

In some cases, a cardiac-active therapeutic can comprise a member selected from a group consisting of: a modulator of a renin angiotensin aldosterone system (RAAS); a neutral endopeptidase (NEP) inhibitor, a natriuretic peptide (NP) receptor agonist, a recombinant NP, a synthetic NP, ularitide, a relaxin receptor activator, a serelaxin receptor activator, a beta-adrenergic receptor modulating agent, a salt of any of these, or any combination thereof.

In some cases, a modulator of RAAS or salt thereof can comprise a renin inhibitor, an angiotensin-converting enzyme (ACE) inhibitor, an angiotensin receptor antagonist, an angiotensin receptor agonist, an aldosterone synthesis inhibitor, or a salt of any of these.

In some cases, a beta-adrenergic receptor modulating agent can comprise a competitive inhibitor, a partial agonist, an agonist, an activator, or an inverse agonist.

Also disclosed herein in some embodiments is a method of determining a cardiac wasting prognosis in a subject that can comprise recording a number of premature ventricular contractions (PVCs) in a 24 hour time period in a subject using an electronic device configured to monitor PVCs, repeating the previous steps, and delivering a prognosis to a subject, wherein a subject has a more favorable prognosis if a subject displays a number of PVCs that can comprise less than 10 or greater than 100, relative to a subject that displays a number of PVCs of from about 10 to about 100.

In some cases, a method of determining cardiac wasting can further comprise conducting a cardiac wasting diagnostic test, wherein a cardiac wasting diagnostic test determines a level of a biomarker. In some cases, a biomarker can comprise a genetic biomarker. In some cases, determining can comprise sequencing a sample from a subject. In some cases, sequencing can be performed on a cell free nucleic acid from a subject. In some cases, a subject can have a level of a biomarker that can be greater than a reference level. In some cases, a subject can have a level of a biomarker that can be less than a reference level.

In some cases, cardiotoxicity can be monitored by an interoperable electronic device.

### IX. Disease treatment

Also disclosed herein in some embodiments are methods that can comprise treating a disease in a subject as disclosed herein. In some cases, treating a disease can comprise regulating the beating of a heart of a subject. In some cases, treating a disease can comprise administering a medicament to a subject as described herein. In some cases, treating a disease can result in a reduction of plasma levels of natriuretic peptides. In some cases, natriuretic peptides can comprise ANP and/or BNP.

In some cases, treating a disease can comprise an anti-cachexic effect. In some cases, an anti-cachexic effect can comprise a clinical benefit. In some cases, treating a disease can result in a reduction in a risk of sudden cardiac death. In some cases, treating a disease can result in a reduction of weight loss.

In some cases, treating a disease can result in an improvement of symptom status. In some cases, a symptom can comprise shortness of breath, fatigue status, muscle strength, exercise capacity, or any combination thereof. In some cases, a treatment can aim to improve patients' quality of life, symptoms, general wellbeing, cardiac health, survival, duration of hospitalization, cost of hospitalization, or any combination thereof.

### EXAMPLES

While preferred embodiments have been shown and described herein, it will be obvious to those skilled in the art that such embodiments are provided by way of example only. Numerous variations, changes, and substitutions will now occur to those skilled in the art without departing from the disclosure provided herein. It should be understood that various alternatives to the exemplary embodiments described herein may be employed.

### Example 1: A prospective analysis of ventricular tachycardia, premature ventricular contractions, and mortality in unselected patients with advanced cancer

In this study the presence and predictive impact of non-sustained ventricular tachycardia (NSVT) and premature ventricular contractions (PVC) in patients with advanced cancer was investigated.

### 1. Methods

### 1.1 Patient recruitment

In this study 169 cancer patients with histologically confirmed diagnoses of pancreatic (PC, n=91), colorectal (CRC, n=37), or non-small cell lung cancer (NSCLC, n=41) were recruited at a study center. Of these, a total of 120 patients and 43 healthy controls agreed to undergo 24h ECG and were included in this analysis.

Exclusion criteria included: (1) age <18 years, (2) ongoing infection, (3) inflammatory disease, (4) another cancer diagnosis within 5 years, (5) predicted life expectancy <2 months, (6) chronic obstructive pulmonary disease with FEV1 <50%, (7) clinical signs or symptoms of chronic heart failure, (8) prior myocardial infarction, (9) prior treatment related cardiotoxicity, (10) evidence of ischemic heart disease, (11) other known significant cardiovascular diseases, except uncomplicated hypertension or type 2 diabetes mellitus, (12) chemo-, immuno-, radio-, targeted therapy within 21 days before inclusion, (13) major surgery within the last 3 months, and (14) an Eastern Cooperative Oncology Group (ECOG) performance status of more than 2. All cancer patients had been on stable medical regimens for at least four weeks prior to enrolment. All chemotherapy regimens were unchanged and administered as planned.

Age-similar controls were included with a ratio of patients to controls of three to one. All were generally healthy and free of known significant cardiovascular diseases, except uncomplicated hypertension or type 2 diabetes mellitus. All cancer patients and control subjects underwent a 24h ECG and physical examination. Follow-up of the patients was conducted by monitoring the electronic database of the study center and telephone contact with patients, relatives and home physicians. If the patients died in the study center and an autopsy was conducted, the cause of death was recorded from the autopsy protocol. If the patients died in the study center, but no autopsy was conducted, two independent reviewers not involved in the clinical care of the patient assessed the specific cause of death for each patient by reviewing the medical records. If they disagreed, a third reviewer made the final decision. Controls were allowed to receive antihypertensive and antidiabetic medication as clinically indicated. The study was approved by the study center ethics committee. Written informed consent was obtained from all subjects.

### 1.2 24-hour electrocardiograms

All patients had a standard 24h ECG. They were analyzed with the help of MTM CardioScan software (MTM MultiTechMed GmbH, Hünfelden-Dauborn, Germany) and verified by two independent medical reviewers. The following recordings were made; average 24-hour heart rate in beats per minute (bpm), total number of premature ventricular contractions (PVCs) and premature atrial contractions, presence of non-sustained ventricular tachycardia (NSVT), standard deviation of all normal sinus RR intervals over 24 hours (SDNN 24h) in milliseconds (ms), and heart rhythm. Non-sustained ventricular tachycardia was defined according to the WHO and EHRA/HRS/APHRS, as ≥3 consecutive heart beats originating from the ventricular chamber exceeding a heart rate of 100 beats per minute.

### 1.3 Blood Collection

In the morning, after 10 minutes of supine rest, venous blood was collected from an antecubital vein. A small breakfast was allowed. A complete blood count and routine clinical biochemistry was performed, according to standard laboratory operating procedures.

### 1.4 Statistical analyses

Results are presented as mean ± standard deviation (SD) or median with interquartile range. To assess normal distribution Kolmogorov-Smirnov test was used. Unpaired Student's t-test, Mann-Whitney U test, Analysis of variance, and Fisher's post Hoc Test were used as appropriate. For the analysis of the contingency tables, Chi-squared tests were preferably used. If the contingency tables contained more than two columns or more than two rows and at least one cell assignment was smaller than five, the Fisher's Exact test was chosen. The Barnard's test was used for 2 x 2 tables with a cell assignment smaller than five. With regards to Cox-proportional hazard survival analyses (log-rank test), hazard ratios (HRs) are given with 95% confidence intervals (95% CI) for risk factors. For illustrative purposes, Kaplan-Meier survival curves were constructed. Since this study was not designed as a confirmatory study, but to identify patterns, it was not necessary to make an adjustment for tests for multiplicity. The significance tests used therefore have a descriptive character. Statistical analyses were conducted using the "Stat View 5.0 software" (SAS Institute Inc., Cary, NC, USA), "SAS 9.4" and "R 3.5.1". In all analyses a p-value <0.05 was considered statistically significant.

### 2. Results

### 2.1 Study population

Cancer patients and controls (all Caucasians) had similar age and sex (60±10 years versus 61±11 years; p=0.67; men: 65 [54%] versus 24 [56%]; p=0.85). Baseline characteristics are shown in Table 1 and baseline medication in Table 3. In 106 cancer patients, left ventricular ejection fraction (LVEF) was assessed: mean 60±8%, with no one with an LVEF <35%. Patients were followed until August 2018 for a median of 21 months (max. 154 months). 94 patients (78%) had advanced cancer stage (III/IV). A total of 96 patients (80%) died during follow-up: 1-year mortality 31% [95%CI 23-39%], 3-year mortality 68% [60-76%], and 5-year mortality 73% [65-81%]. 33 patients (34%) died in the study center. Of these, 5 patients had an autopsy in which the exact cause of death was determined by a pathologist. In the other 28 patients a cause of death could be adjudicated. The cause of death of these 33 patients were: 15 (45%) cancer progression, 11 (33%) sepsis/pneumonia/infection, 4 (12%) gastrointestinal bleeding, 3 (9%) cardiovascular. At time of death, 24 patients (73%) had diagnosed cachexia. With regards to all 96 deceased patients, 63 (66%) died at home or in a hospice and therefore adjudicating a cause of death was not possible. Information about all-cause mortality was available for all patients and hence used for further analyses.

79 patients (66%) had an operation due to the underlying cancer disease >3 months prior to enrolment, 89 patients (74%) had received chemotherapy prior to enrolment, and 38 patients (32%) had previously received potentially cardiotoxic chemotherapies. No significant age and sex distribution differences were noted between groups (Table 4). Body mass index, hemoglobin and blood pressure were lower in cancer patients. There was no difference in the frequency of use of antidiabetic medication, beta-blockers, or angiotensin-converting-enzyme inhibitor/ angiotensin II receptor antagonist (all p>0.1) between controls and the overall cancer cohort. Within the cancer cohort, subgroup analysis showed that NSCLC patients were more often treated with beta blockers (39% vs. 9% in colorectal cancer (p=0.0043) and vs. 11% in pancreatic cancer (p=0.0068), Table 5).

### 2.2 24-hour electrocardiograms

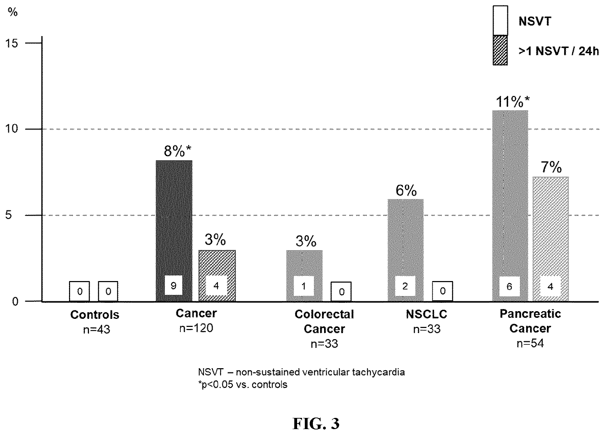

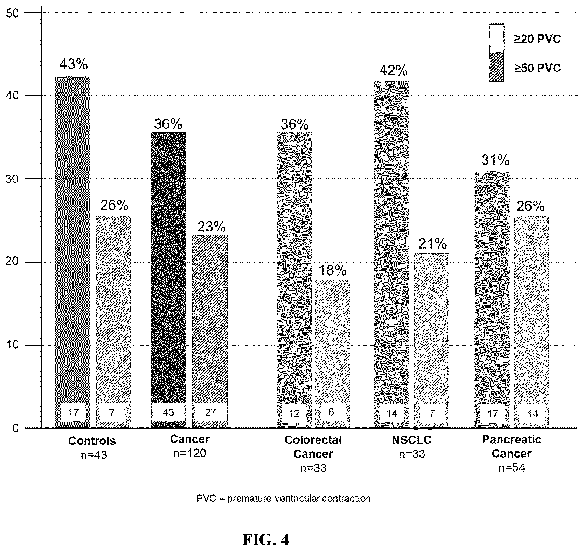

In 24h ECG analyses, all but one cancer patient was found to be in sinus rhythm. One NSCLC patient had normo-frequent atrial fibrillation. Cancer patients more frequently showed non-sustained ventricular tachycardia than controls (NSVT, 9 [8%] vs. 0, p=0.021, **FIG. 3****),** and had a higher mean 24h heart rate and reduced heart rate variability (Table 1), whereas controls showed more premature atrial contractions. In 8 of the 9 cancer patients with detection of NSVTs an echocardiogram was performed (all LVEF>50%). Some patients had more than one episode of NSVT. The total number of NSVT episodes in these patients were the following: pancreatic cancer: 43, 35, 7, 6, 1, and 1; non-small cell lung cancer: 1 and 1; colorectal cancer: 1. The number of premature ventricular contractions (PVCs) was not significantly different between cancer patients and controls **(****FIG. 4****).**

### 2.3 Survival analyses

With respect to baseline characteristics, patients with a fatal event during the first 5 years were compared with cancer patient survivors (Table 1). Patients with fatal events had lower baseline BMI, hemoglobin, and leucocytes, and higher gamma-glutamyl transferase. Patients with fatal events demonstrated higher average 24-hour heart rates and more frequently had NSVTs (9 [10%] vs. 0, p=0.019).

Subgroup analyses showed that patients with NSVTs in their 24h ECG were older, had higher creatinine, more atrial premature beats, and PVCs (Table 6).

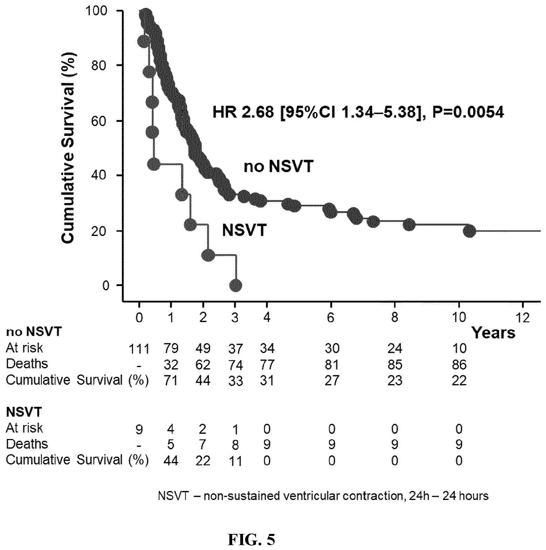

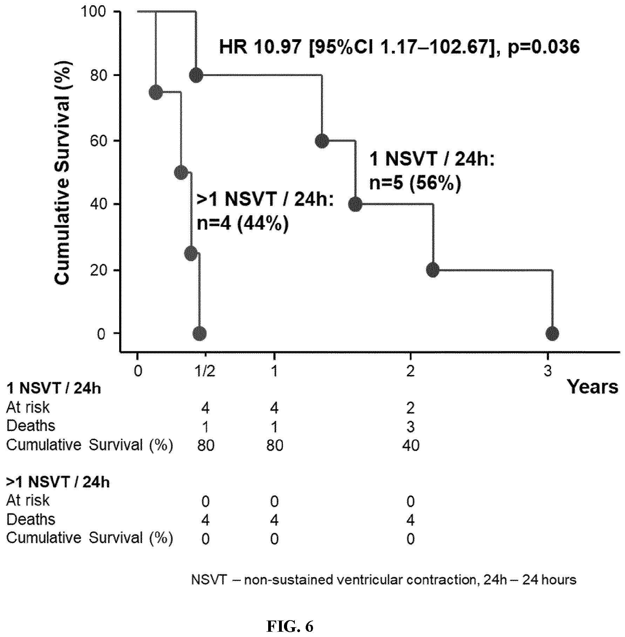

All patients with NSVTs in 24h ECG had died within 37 months. In univariable and multivariable Cox-proportional hazard analyses, including all univariable predictors of mortality as well as age and sex, the presence of NSVT and the number of PVCs/24h were associated with higher mortality (Table 2). When LVEF was included in this model (n=106), NSVT and PVCs/24h remained significant predictors of mortality. The number of premature atrial contractions/24h in cancer patients was not a significant predictor of mortality (p=0.8). The Kaplan-Maier curve emphasizes the underlying survival advantage of patients without presence of NSVT **(****FIG. 5****).** The subgroup of cancer patients with >1 NSVT in 24 hours had worse prognosis then patients with only 1 NSVT episode (HR 10.97 [1.17-102.67], p=0.036, **FIG. 6****).**

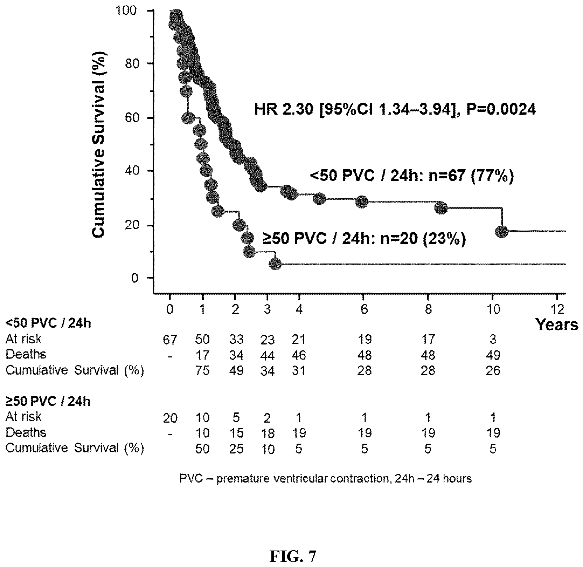

Subgroup analyses revealed that PVCs/24h were a significant predictor of mortality in patients with colorectal cancer (HR per 100 PVCs: 1.13 [1.02-1.25], p=0.024), and pancreatic cancer (HR per 100 PVCs: 1.03 [1.01-1.05], p=0.0036), but not in patients with NSCLC (p>0.1). Therefore, the best predictive cut-off value for PVCs to predict survival only in patients with pancreatic and colorectal cancer was analyzed and found to be at ≥50 PVCs/24h (HR 2.30 [1.34-3.94], p=0.0024, **FIG. 7** )- 26% in pancreatic and 18% in colorectal cancer had ≥50 PVCs/24h.

### 3. Discussion

In this study the frequency of ventricular arrhythmias was assessed during 24h ECGs in cancer patients and their long-term prognostic value was examined. In unselected cancer patients with advanced cancer but without significant cardiovascular disease, the frequency of non-sustained ventricular tachycardia (NSVT) on 24-hour ECG recordings compared to healthy controls was increased, while premature ventricular contractions (PVCs) were not more frequent. However, both the number of NSVT and PVCs carried significant prognostic value in these cancer patients, independent of other univariate predictors of mortality such as tumor stage, diagnosis, potassium, prior surgery, and hemoglobin. In 106 cancer patients where LVEF assessment was available, the prognostic value of NSVT and PVCs was also independent of cardiac function of patients.

Investigations were focused on unselected patients with advanced malignant cancer due to one of three histologically confirmed diagnoses, i.e. colorectal and pancreatic cancer as well as NSCLC. All these cancers are associated with very high mortality burden. The frequency of NSVT in other cancers in general may differ, and it may differ in patients exposed to significant cardiotoxic chemo- and radiation therapy. The detection of only one case of atrial fibrillation in this study may demonstrate the unselected nature of this cancer population. In contrast, in studies focusing on cancer patients with risk factors of atrial fibrillation including use of cardiotoxic anti-cancer therapy, frequencies of atrial fibrillation prevalence of 4-5% are reported. For this reason, the research into the general burden of arrhythmia in cancer may require a prospective and unbiased approach regarding patient selection for inclusion. Pre-selection of patients with higher cardiovascular risk for such analyses - as would be the case for instance in a retrospective analysis of 24h-ECGs that were performed in cancer patients for clinical reasons - may not yield reliable prevalence information for the true general burden of arrhythmia in cancer patients.

The frequency of observed NSVT in 24h-ECG of 8% can be considered as surprisingly high. Longer assessments of ECG (over 1-2 weeks or longer) may be required to show whether the frequency of NSVT in cancer patients is even higher than that.

The mortality burden represented by ventricular arrhythmias in these cancer patients may also be considered as unexpectedly high. The results were independent of administered cardiotoxic chemotherapy. In subgroup analysis, PVCs were found to be predictive of survival only in CRC and PC and not in NSCLC, but it was observed that NSCLC patients were more frequently treated with beta-blockers. This treatment is well known to inhibit PVCs and therefore could have reduced the number of PVCs in NSCLC patients.

Previous studies have shown that even chemotherapy-naive cancer patients with advanced disease demonstrate mildly but significant reduced LVEF and reduced heart rate variability compared to healthy controls. This may represent a specific cancer-associated cardiomyopathy that may predispose both to the Holter abnormalities described and their ability to predict subsequent mortality. Many different factors can act as cardiac stressors. For example, during cardiac contraction in cancer patients a higher maximal rate of pressure rise and cardiac output has been described, possibly adding additional stress to the myocardium. Other cardiac stressors include chemo-, radiation-, immuno-, and targeted therapies, as well as cardiac cachexia, and metabolic abnormalities such as hyperkalemia. It is possible that these factors could contribute to the burden of ventricular arrhythmias in cancer patients, which can be associated with excess mortality. This raises the possibility of cardiac-oriented therapies being effective in reducing the risk of CV mortality in selected cancer patients.

### Example 2: Echocardiographic assessment in cancer patients

### 1. Methods

100 unselected cancer patients without significant cardiovascular (CV) disease (age 62±15yrs, 55% female, body mass index (BMI) 25±5kg/m2) and 26 controls (age 56±9yrs, 61% female, BMI 25±3kg/m2) were studied. The cancer group had the following histologically proven malignancies: 41 solid cancers (20 breast, 8 colorectal, 6 non-small cell lung, 7 others) and 59 lymphoma. Cancer stage was I/II/III/IV in 8, 16, 23, and 53%, respectively. 37% of cancer patients were treatment naive. Transthoracic echocardiography was performed with VividE9.

### 2. Results

Cancer patients demonstrated slightly lower left ventricular ejection fraction (LVEF, 66±5%, range 42-78%) than control subjects (69±4%, range 62-79%, p<0.006), lower E/A ratio (1.1±0.4 vs 1.2±0.3, p=0.036), higher E/e' (9±3 vs 7±2, p=0.015), and higher estimated pulmonary artery systolic pressure (PASP, 29±9 vs 25±4mmHg, p=0.027). 96% of cancer patients and 100% of controls had a LVEF=55%. 45% demonstrated diastolic dysfunction grade 1 (E/A<1.0), 30% grade 2 (E/e'=8 with E/A=1), 1% grade 3 (E/e'=15), 24% had no diastolic dysfunction - in controls the distribution was 15, 19, 0, and 66%, respectively. Interventricular septal and posterior wall thickness were significantly elevated in cancer patients vs controls (10.4±1.7 vs 9.6±1.2 mm, p=0.022; 10.2±1.5 vs 9.6±1.1 mm, p=0.038). Subgroup analyses of treatment naive cancer patients vs healthy controls showed increased E/e' (9±4 vs 7±2; p=0.015), PASP (30±8 vs 25±4mmHg; p=0.016), interventricular septal thickness (10.8±2.0 vs 9.6±1.2mm; p=0.0073), and posterior wall thickness (10.5±1.7 vs 9.6±1.1mm; p=0.015). Not significantly different were LVEF (67±4 vs 69±4%; p=0.12) and E/A ratio (1.2±0.5 vs 1.3±0.3; p=0.50). Subgroup analysis of patients with prior chemotherapy known to be cardiotoxic and/or radiotherapy (n=16) vs treatment naive cancer patients (n=37) showed lower LVEF (62±9 vs 67±4%; p=0.0046), lower interventricular septal thickness (9.8±1.0 vs 10.8±2.0mm; p=0.0498), and a tendency to lower posterior wall thickness (9.6±1.0 vs 10.5±1.7mm; p=0.057).

### 3. Conclusion

Cancer patients showed normal but slightly reduced left ventricular ejection fraction. 76% of cancer patients (all without relevant CV disease) demonstrated abnormal diastolic function.

### Example 3: Ventricular arrhythmias and survival in cancer

Do routine 24-hour electrocardiogram recordings of patients with malignant cancer show ventricular arrhythmias and does this carry prognostic information?

### 1. Methods

A retrospective, observational cohort study was performed of patients with active malignant cancer who underwent 24h ECG recording (all left ventricular ejection fraction ≥45%) between February 1, 2012, and May 31, 2018 and were followed for survival until July 3, 2019.

Of 261 consecutive cancer patients in routine care referred for a 24-hour electrocardiogram, 44 (17%) had non-sustained ventricular tachycardia (NSVT) with ≥3 beats, 25 (10%) had NSVT with ≥4 beats, 12 (5%) had NSVT with ≥6 beats, 139 (53%) had ≥20 premature ventricular contractions (PVCs) /24h, and 24 (9%) had ≥3000 PVCs/24h. NSVT with ≥4 and ≥6 beats (HR 1.81 and HR 2.27, respectively, both p<0.02), and ≥20 PVCs/24h (HR 1.60, p<0.01) independently predicted mortality in multivariable survival analyses.

Between February 1, 2012, and May 31, 2018 the total number of cancer patients cared for on the oncology wards at the study center was 39,699. Of these patients the medical records were reviewed for all adult (age ≥18 years) cancer patients with histologically confirmed malignant cancer who had undergone 24h ECG recording (n=277). Patients with left ventricular ejection fraction (LVEF) <45% were excluded (n=16) - leaving 261 patients with malignant cancer for the analyses. This represents a subgroup of 0.7% of cancer patients cared for in that time period in the study center (total number 39,699). In a subgroup of patients of interest, the medical records were reviewed for cancer stage and entity, clinical and laboratory baseline characteristics, echocardiograms, and secondary diagnoses. All cancer patients were followed for all-cause mortality through July 3, 2019. Additionally, a cohort of 35 generally heathy controls were included, of similar age and sex, who were all free of cancer and significant cardiovascular disease, other than controlled arterial hypertension.

All cancer patients were followed-up by monitoring of the electronic database of the study center. The cause of death was recorded, if an autopsy was conducted at the study center after death. If no autopsy was conducted after death at the study center, two independent reviewers that were not involved in the treatment of the patients reviewed the medical records and assessed the cause of death. If they disagreed, a third reviewer decided on the cause of death. The study plan was approved by the study center ethics committee and complies with the Declaration of Helsinki.

### 1.1 24-hour electrocardiograms

A standard 24h ECG was performed in all patients. CardioDay Version 2.4.5 was used for analysis of 24h ECGs and results were verified. total number of premature atrial contractions were recorded. Also recorded were premature ventricular contractions (PVCs), presence and length of non-sustained ventricular tachycardia (NSVT), and average 24-hour heart rate (in beats per minute, bpm). Following the recommendations of the WHO and EHRA/HRS/APHRS, NSVT was defined as ≥3 consecutive heart beats exceeding a heart rate of 100 beats per minute and originating from the ventricular heart chamber.

### 1.2 Statistical analyses

Data are presented as either mean ± standard deviation (SD) or median with interquartile range (IQR). Kolmogorov-Smirnov test was used to assess normal distribution. Analysis of Variance, unpaired Student's t-test and Mann-Whitney U test were used as appropriate. The contingency tables were preferably analyzed with Chi-squared test. For 2 x 2 tables with at least one cell assignment smaller than five, we used the Barnard's test. For survival analyses the Cox-proportional hazard survival analyses (log-rank test) was used. Results are given as hazard ratios (HRs) with 95% confidence intervals (95% CI) for risk factors. Kaplan Meier curves were generated for illustration purposes. Since this study was designed to identify patterns, it was unnecessary to test for multiplicity and the significance tests are descriptive. For Statistical analyses "R 1.1.463", "Stat View 5.0 software" (SAS Institute Inc., Cary, NC, USA), and "IBM SPSS Version 25.0" (IBM, Armonk, New York, USA) were used. A p-value <0.05 was considered as statistically significant in all analysis.

### 2. Results

Presence of NSVT and PVCs in routine care of cancer patients predict survival. Prospective studies are needed to confirm these results. We included 261 consecutive cancer patients and 35 healthy controls of similar age and sex. Non-sustained ventricular tachycardia (NSVT) with ≥3 and ≥4 beats were more frequent in cancer patients than controls (17% vs 0%, p=0.0008; 10% vs 0%, p=0.016) - that of NSVT ≥6 beats did not reach statistical significance (5% vs 0%, p=0.11). Premature ventricular contractions (PVCs) /24 hours were not more frequent in cancer patients compared to controls (median (IQR), 26 (2-360) vs 9 (1-43), p=0.06; ≥20PVC 53% vs 37%, p=0.07; ≥3000PVCs 9% vs 3%, p=0.38). During follow-up, (up to 7.2 years, median 15 month) of the cancer patients, 158 (61%) died (1-/3-/5-year mortality rates: 45% [95%CI 39-51%], 66% [95%CI 59-73%], 73% [95%CI 64-82%]). Non-sustained ventricular tachycardia with ≥4 and ≥6 beats (HR 1.81 and HR 2.27, respectively, both p<0.02), and ≥20 PVCs/24h (HR 1.60, p<0.01) independently predicted mortality in univariable and multivariable survival analyses, adjusted for all other univariate predictors of mortality as well as clinical relevant factors, including cancer stage and type, prior potentially cardiotoxic anti-cancer drug therapy, coronary artery disease, potassium concentration, and hemoglobin. Non-sustained ventricular tachycardia and premature ventricular contractions seen in routine 24h ECGs of patients with malignant cancer carry prognostic relevance.

### 2.1 Study population

261 cancer patients (129 with solid cancers and 132 patients with hematological malignancies, **Table 3**) and 35 healthy controls of similar age and sex (all Caucasians). Baseline characteristics are shown in Table 1 and baseline medication in **Table 4**. In 181 cancer patients and all 35 controls LVEF was assessed. LVEF was similar between cancer patients and controls (65 ± 7 vs 64 ± 7, p=0.81). Follow-up of patients ended on July 3, 2019 after a median of 15 months (maximum 7.2 years). 188 patients (72%) had an advanced cancer stage (≥III). 158 cancer patients (61%) died during follow-up (1-/3-/5-year mortality: 45% [95%CI 39-51%], 66% [95%CI 59-73%], 73% [95%CI 64-82%]). Of those, 37 (23%) cancer patients died within the study center. The cause of death was determined by an autopsy in 3 patients. In the other 34 it was possible to adjudicate the cause of death. In those 37 patients, the cause of death was: 17 (46%) cancer progression, 16 (43%) infection/pneumonia/sepsis, 2 (5%) cardiovascular, 1 (3%) gastrointestinal bleeding, 1 (3%) intracerebral bleeding. Of the patients that died in the study center, at time of death, 23 patients (62%) had been diagnosed with cachexia. The remaining 121 cancer patients (77% of all deaths) died at home or in a hospice, where adjudicating the cause of death was not possible. Therefore, all-cause mortality was used for all further survival analysis.

199 patients (76%) had previously received any kind of anti-cancer drug therapy before and 162 patients (62%) any kind of potentially cardiotoxic chemotherapy **(Table 5).** Hemoglobin, sodium, and potassium concertation were lower in cancer patients compared to controls. Since only generally healthy controls were included, secondary diagnoses and different drug treatments were more frequently observed in cancer patients than controls.

### 2.2 24-hour electrocardiograms

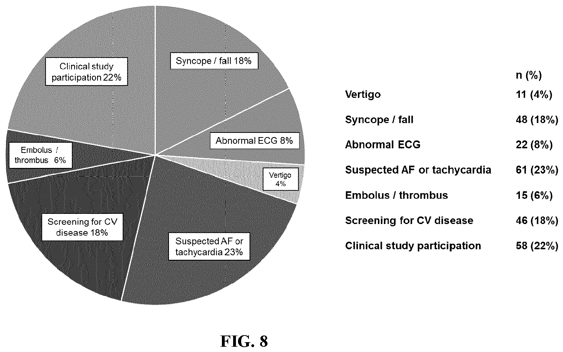

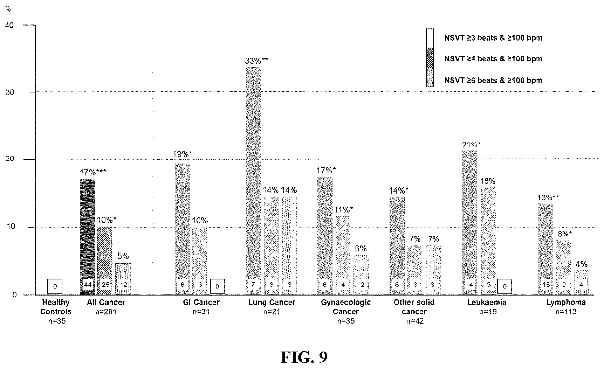

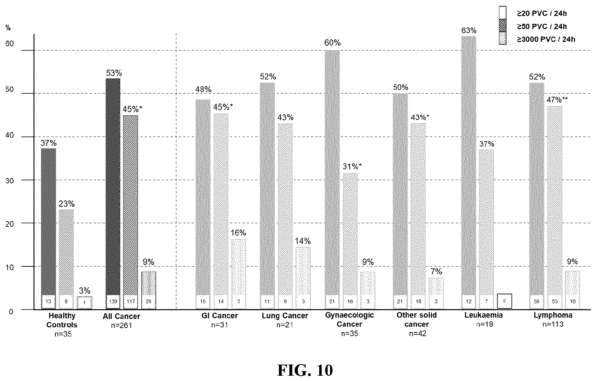

The most frequent reasons for conducting a 24h ECG were syncope/fall, suspected atrial fibrillation or tachycardia, screening for CV disease, or clinical study participation (each ~20%, **FIG. 8**). Analysis of all 24h ECGs showed that non-sustained ventricular tachycardia (NSVT) with ≥3 and ≥4 beats were more frequent in cancer patients than controls (17% vs 0%, p=0.0008; 10% vs 0%, p=0.016) - NSVT ≥6 beats was not (5% vs 0%, p=0.11, Table 1, **FIG. 9**). Premature atrial contractions /24h was not significantly increased in cancer patients compared to controls. Premature ventricular contractions (PVCs) /24hours showed a tendency towards higher frequency in cancer patients (median (IQR), 26 (2-360) vs 9 (1-43), p=0.06; ≥20PVC 53% vs 37%, p=0.07; ≥50PVC 23% vs 45%, p=0.013 **(****FIG. 10****).** Average 24h heart rate was elevated in cancer patients. In cancer patients with NSVT ≥4, ≥6 beats, and ≥20PVC/24h, LVEF was not significantly decreased **(Tables 6-8).**

### 2.3 Comparing patients with and without ventricular arrhythmias

Subgroup analyses showed that patients with NSVT ≥4 beats more frequently had coronary artery disease, more often used angiotensin-converting enzyme inhibitors, had higher average 24h heart rate, and more PVCs/24h **(Table 6).** Patients with NSVT ≥6 beats had higher BMI, and more premature atrial and ventricular contractions /24h **(Table 7).** Patients with ≥20PVCs were older, had higher average 24h heart rate, had more premature atrial contractions/24h, NSVT with ≥4 and ≥6 beats, were more often female, had more frequently arterial hypertension, coronary artery disease, and previous myocardial infarction, more often used angiotensin-converting enzyme inhibitor and had more frequently received alkylating chemotherapy. **(Table 8).**

### 2.4 Comparing patients with and without death during follow-up

A comparison was made of patients with and without a fatal event during follow-up **(Table 1).** Patients with a fatal event had higher age, more frequently advanced cancer stage, solid cancers, and use of antibiotics, and lower body mass index (BMI), hemoglobin, and sodium levels. NSVT ≥4 beats and ≥6 beats were more frequent in patients with fatal event (13% vs 4%, p=0.0066; 7% vs 1%, p=0.011). Patients with fatal events had a higher number of PVCs/24h (median (IQR), 54 (4 - 467) vs 12 (1 - 280), p=0.013) and more frequently ≥20 PVCs/24h (60% vs 43%, p=0.0059).

### 2.5 Survival analyses

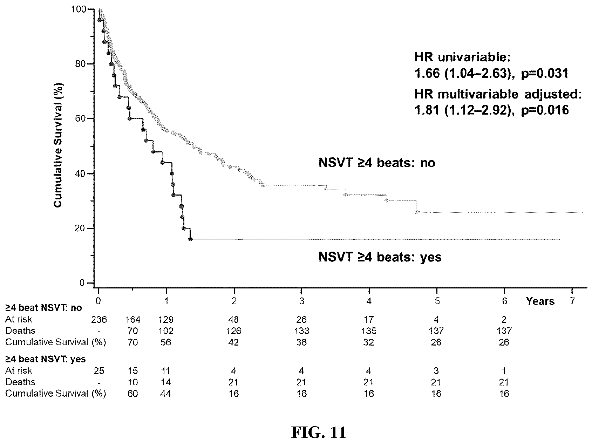

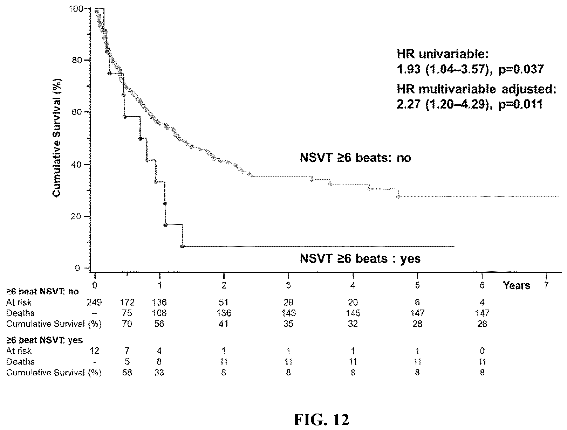

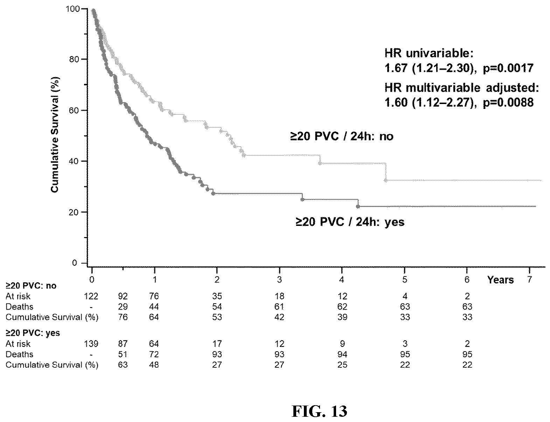

Univariable and multivariable Cox-proportional hazard analyses showed that NSVT ≥4 beats, ≥6 beats, and ≥20 PVCs/24h were associated with higher mortality **(Table 2**). Additional non-significant univariable variables in Cox-proportional hazard analyses are shown in **Table 9**. For each of the three risk factors, two multivariable models were conducted. In the first all univariable predictors of death were included and in the second all other clinically relevant variables were added. Univariable Cox-proportional hazard analyses showed that PVC/24h in continuous analyses were no significant predictor of mortality. ROC analyses identified ≥20 PVCs as a nominal predictor of mortality (area under the curve 59.1%, 95%CI 52.0%-66.3%), which was therefore used for the multivariable survival analysis described above. For illustrative purposes Kaplan-Maier curves were constructed showing the survival benefit of patients without NSVT with ≥4 beats, ≥6 beats, or ≥20 PVCs **(****FIG. 11-13**).

### 3.0 Discussion

This study documents that non-sustained ventricular tachycardia (NSVT) is seen more frequently in patients with malignant cancer compared to healthy control subjects of similar age and sex. The presence of NSVT independently carries relevant prognostic information. Premature ventricular contractions (PVCs) are not seen (on average) more often than is normal in 24h ECGs, but nevertheless, at these relatively low levels higher numbers of PVCs also carry relevant prognostic information. These results present important therapeutic consequences to tackle ventricular arrhythmias in cancer patients.

A strength of the study is that 35 different cancer entities were included (129 patients with solid cancers and 132 with hematological malignancies). Most of the patients had an advanced disease (72%) with a high mortality burden (median survival 15 month).

It has been previously shown that chemotherapy naive cancer patients demonstrate mildly reduced LVEF. We have also found that advanced cancer patients, irrespective of their prior treatment and other concomitant factors have an elevated resting heart. Higher maximal pressure rise during cardiac contraction has also been observed in cancer patients. Additionally, many other cardiac stressors are known that influence the heart such as chemo-, immuno-, radiotherapy and targeted therapy3, metabolic alterations such as hyperkaliemia, and cardiac cachexia. We therefore think, that cancer patients develop a vulnerability of the heart leading to ventricular arrhythmias, which are associated with increased mortality. Hence, new anti-arrhythmic therapies for cancer patients seem possible, that could reduce cardiovascular mortality in these patients.

**Table 1 - Baseline characteristics**

| **Variable** | **Healthy controls (*n* = 35)** | **Cancer patients (*n* = 261)** | ***P*-value** | **Cancer deaths (*n* = 158)** | **Cancer survivors (*n* = 103)** | ***P*-value** |
|---|---|---|---|---|---|---|
| Clinical characteristics | | | | | | |
| Age (years) | 68 ± 6 (IQR 62 - 73) | 68 ± 12 (IQR 61 - 75) | 0.86 | 69 ± 10 (IQR 62 - 75) | 65 ± 17 (IQR 58 - 76) | **0.016** |
| Female sex, *n* (%) | 19 (54) | 132 (51) | 0.68 | 76 (48) | 56 (54) | 0.32 |
| BMI (kg/m²) | 26 ± 4 | 24 ± 5 | 0.28 | 25 ± 5 | 26 ± 5 | **0.049** |
| Cancer stage ≥III, *n* (%) | - | 188(72) | - | 123 (78) | 65 (62) | **0.0095** |
| Cancer type: solid | - | 129 (49) | - | 93 (59) | 36 (35) | **0.0002** |
| Prior potentially cardiotoxic anti-cancer drugs, *n* (%) | - | 162 (62) | - | 104 (66) | 58 (56) | 0.12 |
| Left ventricular ejection fraction (%) | 64 ± 7 | 65 ± 7 (*n*=181) | 0.81 | 64 ± 8 (*n*=100) | 65 ± 7 (*n*=81) | 0.27 |
| Laboratory parameters | | | | | | |
| Haemoglobin (g/dL) | 14.2 ± 1.3 | 10.8 ± 2.0 | **<0.0001** | 10.6 ± 1.9 | 11.1 ± 2.1 | **0.040** |
| Leucocytes (/nL) | 6.6 (5.5 - 7.6) | 6.5 (4.5 - 93) | 0.99 | 6.8 (4.4 - 9.9) | 6.1 (4.3 - 9.2) | 0.40 |
| Platelets (/nL) | 227 ± 46 | 225 ± 166 | 0.97 | 231 ± 195 | 217 ± 108 | 0.48 |
| Sodium (mmol/L) | 141 ± 2 | 139 ± 4 | **0.0002** | 138 ± 4 | 140 ± 3 | **0.0027** |
| Potassium (mmol/L) | 4.2 ± 0.4 | 3.9 ± 0.5 | **0.0004** | 3.9 ± 0.5 | 3.9 ± 0.5 | 0.91 |
| Creatinine (mg/dL) | 0.87 ± 0.19 | 1.00 ± 0.59 | 0.22 | 1.03 ± 0.68 | 0.94 ± 0.40 | 0.23 |
| GOT (U/L) ^{c} | 26 (22 - 28) | 26 (20 - 35) (*n*=171) | 0.75 | 27 (19 - 39) (*n*=101) | 25 (19 - 34) (*n*=70) | 0.34 |
| Secondary diagnoses | | | | | | |
| Arterial hypertension, *n* (%) | 7 (20) | 132 (51) | **0.0007** | 79 (50) | 53 (51) | 0.82 |
| Coronary artery disease, *n* (%) | 0 | 34 (13) | **0.0040** | 22 (14) | 12(12) | 0.59 |
| Atrial fibrillation, *n* (%) | 0 | 14 (5) | 0.08 | 8 (5) | 6 (6) | 0.79 |
| Previous myocardial infarction, *n* (%) | 0 | 20 (8) | **0.033** | 13 (8) | 7 (7) | 0.67 |
| Diabetes mellitus type 2, *n* (%) | 0 | 50 (19) | **0.0003** | 31 (20) | 19 (18) | 0.81 |
| Chronic kidney disease, *n* (%) | 0 | 41(16) | **0.0014** | 28 (18) | 13 (13) | 0.27 |
| Previous stroke, *n* (%) | 0 | 27(10) | **0.011** | 17(11) | 10(10) | 0.79 |
| Current use of antibiotics, *n* (%) | 0 | 45 (17) | **0.0007** | 33 (21) | 12(12) | **0.0054** |
| 24h-ECG | | | | | | |
| Average 24h heart rate (bpm) | 70 ± 9 | 78 ± 14 | **0.0007** | 79 ± 16 | 77 ± 11 | 0.41 |
| No. of premature atrial contraction / 24h | 82 (27 - 302) | 195 (27 - 1167) | 0.22 | 227 (32 - 1226) | 146 (24 - 761) | 0.20 |
| No. of premature ventricular contraction / 24h | 9 (1 -43) | 26 (2 - 360) | 0.06 | 54 (4 - 467) | 12 (1 - 280) | **0.013** |
| ≥1 Premature ventricular contractions / 24h, *n* (%) | 29 (83) | 223 (85) | 0.69 | 140 (89) | 83 (81) | 0.07 |
| ≥20 Premature ventricular contractions / 24h, *n* (%) | 13 (37) | 139 (53) | 0.07 | 95 (60) | 44 (43) | **0.0059** |
| ≥50 Premature ventricular contractions / 24h, *n* (%) | 8 (23) | 117 (45) | **0.013** | 80 (51) | 37 (36) | **0.020** |
| ≥100 Premature ventricular contractions / 24h, *n* (%) | 6 (17) | 101 (39) | **0.013** | 71 (45) | 30 (29) | **0.010** |
| ≥500 Premature ventricular contractions / 24h, *n* (%) | 5 (14) | 56 (21) | 0.32 | 36 (23) | 20 (19) | 0.52 |
| ≥1000 Premature ventricular contractions / 24h, *n* (%) | 3(9) | 38 (15) | 0.54 | 24 (15) | 14 (14) | 0.72 |
| ≥3000 Premature ventricular contractions / 24h, *n* (%) | 1(3) | 24 (9) | 0.38 | 15(9) | 9 (9) | 0.84 |
| NSVT with ≥3 beats & ≥100 bpm, *n* (%) | 0 | 44 (17) | **0.0008** | 31 (20) | 13 (13) | 0.14 |
| NSVT with ≥4 beats & ≥100 bpm, *n* (%) | 0 | 25 (10) | **0.016** | 21 (13) | 4 (4) | **0.0066** |
| NSVT with ≥5 beats & ≥100 bpm, *n* (%) | 0 | 14 (5) | 0.08 | 12 (8) | 2 (2) | **0.035** |
| NSVT with ≥6 beats & ≥100 bpm, *n* (%) | 0 | 12 (5) | 0.11 | 11 (7) | 1 (1) | **0.011** |
| NSVT with ≥7 beats & ≥100 bpm, *n* (%) | 0 | 12 (5) | 0.11 | 11 (7) | 1 (1) | **0.011** |
| NSVT with ≥8 beats & ≥100 bpm, *n* (%) | 0 | 9 (3) | 0.16 | 8 (5) | 1(1) | 0.051 |

Values are means ± SD, or n (%), or for abnormal distributed data median and Interquartile Range (IQR). 24h, 24 hours; bpm, beats per minute; BMI, body mass index; GOT, glutamic oxaloacetic transaminase; no., number; NSVT, non-sustained ventricular tachycardia; mmol/L, millimoles per liter; g/dL, grams per deciliter; kg/m2, kilogram per square meter; U/L, Units per liter; /nL, per nanoliter; ms, milliseconds.

**Table 2 - Univariable and Multivariable survival analyses in cancer patients (n=261)**

| **Variable** | **Univariable model** | | | | | | | |
|---|---|---|---|---|---|---|---|---|
| | | **HR** | **95% CI** | | 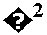 | | **P- value** | |
| **24h ECG** | | | | | | | | |
| Premature ventricular contractions (per 1000 / 24h) | | 1.02 | 0.993 - 1.05 | | 2.1 | | 0.15 | |
| ≥1 Premature ventricular contractions / 24h (yes *vs* no) | | 1.71 | 1.04 - 2.79 | | 4.5 | | **0.033** | |
| ≥20 Premature ventricular contractions / 24h (yes *vs* no) | | 1.67 | 1.21 - 2.30 | | 9.9 | | **0.0017** | |
| ≥50 Premature ventricular contractions / 24h (yes *vs* no) | | 1.57 | 1.15 - 2.16 | | 8.1 | | **0.0044** | |
| ≥100 Premature ventricular contractions / 24h (yes *vs* no) | | 1.60 | 1.17 - 2.19 | | 8.6 | | **0.0033** | |
| ≥500 Premature ventricular contractions / 24h (yes *vs* no) | | 1.26 | 0.87 - 1.82 | | 1.5 | | 0.23 | |
| ≥1000 Premature ventricular contractions / 24h (yes *vs* no) | | 1.19 | 0.77 - 1.84 | | 0.6 | | 0.44 | |
| ≥3000 Premature ventricular contractions / 24h (yes *vs* no) | | 1.16 | 0.68 - 1.98 | | 0.3 | | 0.58 | |
| NSVT ≥3 beats & ≥100bpm (yes *vs* no) | 1.22 | 0.82 - 1.81 | 1.0 | 0.32 | | | | |
| NSVT ≥4 beats & ≥100 bpm (yes *vs* no) | 1.66 | 1.04 - 2.63 | 4.6 | **0.033** | | | | |
| NSVT ≥5 beats & ≥100bpm (yes *vs* no) | 1.66 | 0.92 - 3.00 | 2.8 | 0.09 | | | | |
| NSVT ≥6 beats & ≥100bpm (yes *vs* no) | 1.93 | 1.04 - 3.57 | 4.4 | **0.037** | | | | |
| NSVT ≥7 beats & ≥100bpm (yes *vs* no) | 1.93 | 1.04 - 3.57 | 4.4 | **0.037** | | | | |
| NSVT ≥8 beats & ≥100bpm (yes *vs* no) | 1.99 | 0.98 - 4.07 | 3.6 | 0.06 | | | | |
| **Other significant and clinically relevant variables** | | | | | | | | |
| Age (per lyear) | 1.01 | 1.0007 - 1.03 | 4.3 | **0.039** | | | | |
| BMI (per 1kg/m²) | 0.95 | 0.91 - 0.98 | 8.1 | **0.0043** | | | | |
| Cancer stage (≥III *vs* I/II) | 1.66 | 1.14 - 2.42 | 7.0 | **0.0082** | | | | |
| Cancer type (solid *vs* hematologic) | 2.09 | 1.52 - 2.87 | 20.3 | **<0.0001** | | | | |
| Haemoglobin (per 1g/dL) | 0.91 | 0.84 - 0.986 | 5.3 | **0.021** | | | | |
| Sodium (per 1mmol/L) | 0.94 | 0.90 - 0.98 | 10.4 | **0.0013** | | | | |
| Opioids (yes *vs* no) | 1.67 | 1.13 - 2.47 | 6.7 | **0.0097** | | | | |
| Antidepressants (yes *vs* no) | 1.85 | 1.14 - 2.99 | 6.2 | **0.013** | | | | |
| Prior potentially cardiotoxic anti-cancer drugs (yes *vs* no) | 1.41 | 1.02 - 1.96 | 4.2 | **0.039** | | | | |
| Sex (female *vs* male) | 0.80 | 0.59 - 1.10 | 1.9 | 0.17 | | | | |
| Potassium (per 1mmol/L) | 1.07 | 0.78 - 1.48 | 0.2 | 0.66 | | | | |
| Current use of antibiotics (yes *vs* no) | 1.29 | 0.88 - 1.90 | 1.7 | 0.19 | | | | |
| Coronary artery disease (yes *vs* no) | 0.99 | 0.63 - 1.56 | 0.001 | 0.98 | | | | |

| **Variable** | **Multivariable model 1** | | | | **Multivariable model 2** | | | |
|---|---|---|---|---|---|---|---|---|
| | **HR** | **95% CI** 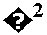 | | **P-value** | **HR** | **95% CI** 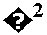 | | **P-value** |
| Multivariable survival analyses in cancer patients with NSVT ≥4 beats & ≥100bpm | | | | | | | | |
| NSVT ≥4 beats & ≥100bpm (yes *vs* no) | 1.86 | 1.15 - 2.99 | 6.4 | **0.011** | 1.81 | 1.12 - 2.92 | 5.8 | **0.016** |
| Age (per lyear) | 1.02 | 1.01 - 1.04 | 9.1 | **0.0025** | 1.02 | 1.01 - 1.04 | 8.9 | **0.0028** |
| Sodium (per 1mmol/L) | 0.96 | 0.92 - 0.998 | 4.2 | **0.041** | 0.96 | 0.92 - 0.998 | 4.2 | **0.040** |
| Haemoglobin (per 1g/dL) | 0.91 | 0.84 - 0.998 | 4.0 | **0.044** | 0.92 | 0.84 - 0.999 | 3.9 | **0.049** |
| BMI (per 1kg/m²) | 0.96 | 0.92 - 0.992 | 5.7 | **0.017** | 0.95 | 0.92 - 0.991 | 6.0 | **0.015** |
| Opioids (yes *vs* no) | 1.42 | 0.95 - 2.13 | 2.8 | 0.09 | 1.47 | 0.97 - 2.22 | 3.3 | 0.07 |
| Antidepressants (yes *vs* no) | 1.56 | 0.95 - 2.56 | 3.1 | 0.08 | 1.66 | 1.01 - 2.73 | 4.0 | **0.046** |
| Cancer stage (≥III *vs* I/II) | 1.52 | 1.03 - 2.24 | 4.4 | **0.036** | 1.55 | 1.05 - 2.29 | 4.9 | **0.027** |
| Cancer type (solid *vs* hematologic) | 2.46 | 1.75 - 3.45 | 27.0 | **<0.0001** | 2.52 | 1.79 - 3.54 | 27.8 | **<0.0001** |
| Prior potentially cardiotoxic anti-cancer drugs (yes *vs* no) | 1.66 | 1.17 - 2.37 | 8.0 | **0.0047** | 1.67 | 0.17 -2.39 | 8.0 | **0.0046** |
| Sex (female *vs* male) | | | | | 0.73 | 0.52 - 1.01 | 3.5 | 0.06 |
| Potassium (per 1mmol/L) | | | | | 1.12 | 0.81 - 1.54 | 0.4 | 0.51 |
| Current use of antibiotics (yes *vs* no) | | | | | 1.28 | 0.85 - 1.92 | 1.4 | 0.23 |
| Coronary artery disease (yes *vs* no) | | | | | 1.08 | 0.66 - 1.77 | 0.1 | 0.75 |
| Multivariable survival analyses in cancer patients with NSVT ≥6 beats & ≥100bpm | | | | | | | | |
| NSVT ≥6 beats & ≥100bpm (yes *vs* no) | 2.25 | 1.19 - 4.24 | 6.3 | **0.012** | 2.27 | 1.20 - 4.29 | 6.4 | **0.011** |
| Age (per lyear) | 1.02 | 1.01 - 1.04 | 8.6 | **0.0034** | 1.02 | 1.01 - 1.04 | 8.5 | **0.0035** |
| Sodium (per 1mmol/L) | 0.95 | 0.92 - 0.995 | 4.8 | **0.029** | 0.95 | 0.91 - 0.99 | 5.0 | **0.026** |
| Haemoglobin (per 1g/dL) | 0.91 | 0.83 - 0.99 | 4.5 | **0.034** | 0.91 | 0.84 - 0.994 | 4.4 | **0.036** |
| BMI (per 1kg/m²) | 0.95 | 0.92 - 0.989 | 6.5 | **0.011** | 0.95 | 0.91 - 0.987 | 6.9 | **0.0086** |
| Opioids (yes *vs* no) | 1.41 | 0.94 - 2.11 | 2.8 | 0.10 | 1.47 | 0.97 - 2.21 | 3.3 | 0.07 |
| Antidepressants (yes *vs* no) | 1.56 | 0.95 - 2.54 | 3.1 | 0.08 | 1.66 | 1.01 - 2.72 | 4.0 | **0.046** |
| Cancer stage (≥III vs I/II) | 1.56 | 1.06 - 2.30 | 5.0 | **0.025** | 1.60 | 1.08 - 2.36 | 5.6 | **0.018** |
| Cancer type (solid *vs* hematologic) | 2.37 | 1.69 - 3.33 | 25.1 | **<0.0001** | 2.42 | 1.72 - 3.40 | 25.7 | **<0.0001** |
| Prior potentially cardiotoxic anti-cancer drugs (yes *vs* no) | 1.64 | 1.15 - 2.33 | 7.5 | **0.0062** | 1.64 | 1.15 - 2.35 | 7.6 | **0.0059** |
| Sex (female *vs* male) | | | | | 0.72 | 0.52 - 1.001 | 3.8 | 0.051 |
| Potassium (per 1mmol/L) | | | | | 1.15 | 0.84 - 1.59 | 0.7 | 0.39 |
| Current use of antibiotics (yes *vs* no) | | | | | 1.26 | 0.84 - 1.89 | 1.3 | 0.26 |
| Coronary artery disease (yes *vs* no) | | | | | 1.09 | 0.67 - 1.77 | 0.11 | 0.74 |
| Multivariable survival analyses in cancer patients with≥20 Premature ventricular contractions | | | | | | | | |
| ≥20 Premature ventricular contractions / 24h (yes *vs* no) | 1.69 | 1.20- 2.36 | 9.1 | **0.0025** | 1.60 | 1.12 - 2.27 | 6.9 | **0.0088** |
| Age (per lyear) | 1.02 | 0.999986 - 1.03 | 3.8 | 0.050 | 1.02 | 1.001 - 1.03 | 4.2 | **0.041** |
| Sodium (per 1mmol/L) | 0.96 | 0.92 - 1.003 | 3.3 | 0.07 | 0.960 | 0.92 - 1.002 | 3.5 | 0.06 |
| Haemoglobin (per 1g/dL) | 0.90 | 0.83 - 0.988 | 5.3 | **0.022** | 0.91 | 0.83 - 0.990 | 5.1 | **0.024** |
| BMI (per 1kg/m²) | 0.95 | 0.92 - 0.990 | 6.6 | **0.0098** | 0.95 | 0.913 - 0.988 | 6.6 | **0.010** |
| Opioids (yes *vs* no) | 1.32 | 0.89 - 1.98 | 1.9 | 0.17 | 1.37 | 0.91 - 2.06 | 2.3 | 0.13 |
| Antidepressants (yes *vs* no) | 1.67 | 1.02 - 2.74 | 4.1 | **0.043** | 1.72 | 1.047 - 2.84 | 4.6 | **0.032** |
| Cancer stage (≥III *vs* I /II) | 1.67 | 1.13 - 2.47 | 6.7 | **0.010** | 1.68 | 1.14 - 2.49 | 6.8 | **0.0090** |
| Cancer type (solid *vs* hematologic) | 2.41 | 1.72 - 3.37 | 26.2 | **<0.0001** | 2.44 | 1.74 - 3.43 | 26.6 | **<0.0001** |
| Prior potentially cardiotoxic anti-cancer drugs (yes *vs* no) | 1.51 | 1.06 - 2.15 | 5.2 | **0.022** | 1.52 | 1.06 - 2.17 | 5.3 | **0.021** |
| Sex (female *vs* male) | | | | | 0.75 | 0.54 - 1.04 | 3.0 | 0.09 |
| Potassium (per 1mmol/L) | | | | | 1.10 | 0.80 - 1.51 | 0.3 | 0.57 |
| Current use of antibiotics (yes *vs* no) | | | | | 1.18 | 1.79 - 0.78 | 0.67 | 0.41 |
| Coronary artery disease (yes *vs* no) | | | | | 0.992 | 0.61 - 1.62 | 0.001 | 0.98 |

Hazard ratios are presented for continuously or binomially distributed variables. Model 1 includes all significant univariable predictors of mortality. Model 2 includes all significant univariable predictors of mortality and other clinically important variables. 24h, 24 hours; BMI, body mass index; bpm, beats per minute; CI, Confidence interval. HR, hazard ratio; NSVT, non-sustained ventricular tachycardia; mmol/L, millimoles per liter; g/dL, grams per deciliter; kg/m2, kilogram per square meter; U/L, Units per liter; /nL, per nanoliter; ms, milliseconds.

**Table 3 - Kind of cancer patients (n=261)**

| **Group** | **Type of Cancer** | **No. of patients (*n*=261) *n*, (%)** | **Solid cancer or Hematological malignancy** |
|---|---|---|---|
| **Group 1- Gastrointestinal cancer (*n*=31)** | Rectal cancer | 4 (2) | Solid cancer |
| | Colon cancer | 12 (5) | Solid cancer |
| | Duodenal cancer | 1 (<1) | Solid cancer |
| | Pancreatic cancer | 2 (1) | Solid cancer |
| | Gastric cancer | 4 (2) | Solid cancer |
| | Esophageal cancer | 6 (2) | Solid cancer |
| | Cholangiocellular carcinoma | 12 (5) | Solid cancer |
| **Group 2 - Lung cancer (*n*=21)** | Non-small-cell-lung-cancer | 15 (6) | Solid cancer |
| | Small-cell-lung-cancer | 6 (2) | Solid cancer |
| **Group 3 - Gynaecologic Cancer (*n*=35)** | Cervical- / Ovarian- / Uterine cancer | 12 (5) | Solid cancer |
| | Breast Cancer | 23 (9) | Solid cancer |
| **Group 4 - Other solid cancers (*n*= 42)** | Tonsillar / Laryngeal / Oro- / Epi- / Hypopharyngeal carcinoma | 15 (6) | Solid cancer |
| | Urothelial carcinoma | 6 (2) | Solid cancer |
| | Kidney cell carcinoma | 2 (1) | Solid cancer |
| | Prostate carcinoma | 7 (3) | Solid cancer |
| | Sarcoma | 4 (1) | Solid cancer |
| | Choroid melanoma | 5 (2) | Solid cancer |
| | Thyroid cancer | 1 (<1) | Solid cancer |
| | Thymus carcinoma | 1 (<1) | Solid cancer |
| | Peritoneal Mesothelioma | 1 (<1) | Solid cancer |
| **Group 5 - Leukaemia (*n*=19)** | Chronic myeloid leukemia | 1 (<1) | Hematological malignancy |
| | Acute myeloid leukemia | 11(4) | Hematological malignancy |
| | Myeloproliferative neoplasia | 3 (1) | Hematological malignancy |
| | Myelodysplastic syndromes | 3 (1) | Hematological malignancy |
| | Acute lymphocytic leukemia | 1 (<1) | Hematological malignancy |
| **Group 6 - Lymphoma (*n*=113)** | B-cell-Non-Hodgkin-lymphoma | 82 (31) | Hematological malignancy |
| | T-cell-Non-Hodgkin-lymphoma | 5 (2) | Hematological malignancy |
| | Hodgkin-lymphoma | 10 (4) | Hematological malignancy |
| | Multiple myeloma | 16(6) | Hematological malignancy |

**Table 4 - Prior chemotherapy, immunotherapy and targeted therapy**

| **Variable** | **Healthy Controls (*n* = 35)** | **Cancer patients (*n* = 261)** | ***P*-value** | **Cancer deaths (*n* = 158)** | **Cancer survivors (*n* = 103)** | ***P*-value** |
|---|---|---|---|---|---|---|
| Aspirin, *n* (%) | 2 (6) | 63 (24) | 0.07 | 39 (25) | 24 (23) | 0.80 |
| Angiotensin-converting enzyme inhibitor, *n* (%) | 2 (6) | 57 (22) | 0.11 | 40 (25) | 17 (17) | 0.09 |
| Calcium channel blocker, *n* (%) | 0 | 37 (14) | **0.0025** | 20 (13) | 17 (17) | 0.38 |
| Angiotensin II receptor blocker, *n* (%) | 0 | 36 (14) | **0.0028** | 16 (10) | 20 (19) | **0.033** |
| Beta-blocker, *n* (%) | 5 (14) | 117 (45) | **0.0001** | 74 (47) | 43 (42) | 0.42 |
| Spironolactone, *n* (%) | 0 | 7 (3) | 0.23 | 5 (3) | 2 (2) | 0.61 |
| Diuretics, *n* (%) | 0 | 71 (27) | **<0.0001** | 45 (28) | 26 (25) | 0.57 |
| Anticoagulants, *n* (%) | 1 (3) | 93 (36) | **0.0002** | 61 (39) | 32 (31) | 0.21 |
| Antidiabetics or Insulin, *n* (%) | 0 | 28 (11) | **0.0099** | 17 (11) | 11 (11) | 0.98 |
| Proton pump inhibitors, *n* (%) | 1 (3) | 167 (64) | **<0.0001** | 98 (62) | 69 (67) | 0.41 |
| Opioids, *n* (%) | 0 | 46 (18) | **0.0006** | 32 (20) | 14 (14) | 0.17 |
| Antidepressants, *n* (%) | 0 | 23 (9) | **0.021** | 19 (12) | 4 (4) | **0.013** |
| Corticosteroids, *n* (%) | 0 | 73 (28) | **<0.0001** | 51 (32) | 22 (21) | 0.055 |
| Prior chemotherapy, *n* (%) | - | 199 (76) | - | 127 (80) | 72 (70) | 0.052 |
| Prior immunotherapy, *n* (%) | - | 125 (48) | - | 75 (47) | 50 49) | 0.87 |
| Alkylating agents, *n* (%) | - | 113 (43) | - | 64 (41) | 49 (48) | 0.26 |
| Platins, *n* (%) | - | 74 (28) | - | 56 (35) | 18 (17) | **0.0016** |
| Anti-metabolites, *n* (%) | - | 98 (38) | - | 64 (39) | 34 (33) | 0.22 |
| Topoisomerase inhibitors, *n* (%) | - | 47 (18) | - | 28 (18) | 19 (18) | 0.88 |
| Antracvclines, *n* (%) | - | 79 (30) | - | 45 (28) | 34 (33) | 0.44 |
| Antimitotics, *n* (%) | - | 60 /23) | - | 30 (19) | 30 (29) | 0.06 |
| Taxanes, *n* (%) | - | 30 (11) | - | 24 (15) | 6 (6) | **0.020** |
| Histone deacetylase inhibitors, *n* (%) | - | 1 (<1) | - | 0 | 1 (1) | 0.34 |
| Antibodies/ Immunotherapy, *n* (%) | - | 105 (40) | - | 63 (40) | 42 (41) | 0.88 |
| Thyrosin kinase inhibitors, *n* (%) | - | 13 (5) | - | 11 (7) | 2 (2) | **0.043** |
| Other kinase inhibitors, *n* (%) | - | 3 (1) | - | 3 (2) | 0 | 0.77 |
| Proteasom inhibitors, *n* (%) | - | 17 (7) | - | 9 (6) | 8 (8) | 0.51 |
| Other chemotherapeutic agents, *n* (%) | - | 16 (6) | - | 11 (7) | 5 (5) | 0.51 |

GI, gastrointestinal. * anti-cancer drugs that may cause left ventricular dysfunction (according to the "2016 ESC Position Paper on cancer treatments and cardiovascular toxicity" - Zamorano et al. EHJ. 2016).

**Table 5 - Medication at study entry**

| **Variables** | **All cancer patients *n* = 261** | **GI Cancer *n* = 31** | **Lung Cancer *n =* 21** | **Gynaecologic Cancer *n* = 35** | **Other solid cancers *n* = 42** | **Leukaemia *n* = 19** | **Lymphoma *n* = 113** |
|---|---|---|---|---|---|---|---|
| Alkylating agents, n | 113 | 0 | 1 | 13 | 3 | 4 | 93 |
| Cyclophosphamide, n* | 77 | 0 | 1 | 10 | 0 | 2 | 64 |
| Ifosfamide, n* | 15 | 0 | 0 | 0 | 1 | 0 | 14 |
| Melphalan, n | 14 | 0 | 1 | 0 | 0 | 0 | 13 |
| Temozolomide, n | 1 | 0 | 0 | 0 | 1 | 0 | 0 |
| Dacarbazine, n | 6 | 0 | 0 | 0 | 0 | 0 | 6 |
| Procarbazine, n | 3 | 0 | 0 | 0 | 0 | 0 | 3 |
| Carmustine, n | 7 | 0 | 0 | 0 | 0 | 0 | 7 |
| Chlorambucil, n | 3 | 0 | 0 | 0 | 0 | 0 | 3 |
| Thiotepa, n | 3 | 0 | 0 | 0 | 0 | 0 | 3 |
| Treosulfan, n | 3 | 0 | 0 | 3 | 0 | 0 | 0 |
| Busulfan, n | 2 | 0 | 0 | 0 | 0 | 2 | 0 |
| Bendamustine,n | 24 | 0 | 0 | 0 | 0 | 0 | 24 |
| Platins, n | 74 | 18 | 16 | 13 | 15 | 1 | 11 |
| Carboplatin, n | 26 | 1 | 8 | 12 | 2 | 0 | 2 |
| Cisplatin, n* | 40 | 4 | 7 | 4 | 13 | 1 | 11 |
| Oxaliplatin, n | 13 | 12 | 0 | 0 | 0 | 0 | 1 |
| Anti-metabolites, n | 98 | 22 | 8 | 8 | 9 | 10 | 41 |
| High-dose Methotrexate, n | 24 | 0 | 0 | 0 | 1 | 1 | 22 |
| Fluorouracil, n | 27 | 18 | 0 | 2 | 6 | 0 | 1 |
| Pemetrexed, n | 1 | 0 | 0 | 0 | 1 | 0 | 0 |
| Tioguanine, n | 1 | 0 | 1 | 0 | 0 | 0 | 0 |
| Capecitabine, n | 13 | 7 | 1 | 5 | 0 | 0 | 0 |
| Gemcitabine, n | 18 | 1 | 7 | 4 | 1 | 1 | 4 |
| Fludarabine, n | 7 | 0 | 0 | 0 | 0 | 4 | 3 |
| Trifluridine + Tipiracil, n | 1 | 1 | 0 | 0 | 0 | 0 | 0 |
| Clofarabine, n* | 0 | 0 | 0 | 0 | 0 | 0 | 0 |
| Cytarabine, n | 32 | 0 | 1 | 0 | 0 | 9 | 22 |
| Topoisomerase inhibitors, n | 47 | 8 | 8 | 1 | 2 | 3 | 25 |
| Irinotecan, n | 8 | 8 | 0 | 0 | 0 | 0 | 0 |
| Topotecan, n | 3 | 0 | 1 | 1 | 0 | 0 | 1 |
| Mitoxantrone, n | 5 | 0 | 0 | 0 | 0 | 3 | 2 |
| Pixantrone, n | 6 | 0 | 0 | 0 | 0 | 0 | 6 |
| Etoposide, n | 29 | 1 | 8 | 0 | 2 | 0 | 18 |
| Antracyclines, n | 79 | 0 | 1 | 13 | 3 | 7 | 55 |
| Doxorubicin / Adriamycin, n* | 70 | 0 | 1 | 9 | 3 | 3 | 54 |
| Pegylated liposomal Doxorubicin, n* | 3 | 0 | 0 | 3 | 0 | 0 | 0 |
| Daunorubicin, n* | 5 | 0 | 1 | 0 | 0 | 3 | 1 |
| Epirubicin, n* | 5 | 0 | 0 | 4 | 1 | 0 | 0 |
| Idarubicin, n* | 3 | 0 | 0 | 0 | 0 | 2 | 1 |
| Antimitotics, n | 60 | 0 | 1 | 2 | 1 | 2 | 54 |
| Vincristine, n | 54 | 0 | 0 | 0 | 1 | 2 | 51 |
| Vinorelbine, n | 3 | 0 | 1 | 1 | 0 | 0 | 1 |
| Vinblastine, n | 6 | 0 | 0 | 0 | 0 | 0 | 6 |
| Taxanes, n | 30 | 5 | 2 | 18 | 5 | 0 | 0 |
| Docetaxel, n* | 11 | 4 | 0 | 3 | 4 | 0 | 0 |
| Eribulin, n | 3 | 0 | 0 | 3 | 0 | 0 | 0 |
| Paclitaxel, n* | 19 | 1 | 2 | 15 | 1 | 0 | 0 |
| Protein-bound paclitaxel, n* | 1 | 0 | 0 | 1 | 0 | 0 | 0 |
| Histone deacetylase inhibitor, n | 1 | 0 | 0 | 0 | 0 | 0 | 1 |
| Belinostat, n | 1 | 0 | 0 | 0 | 0 | 0 | 1 |
| Antibodies/ Immunotherapy, n | 105 | 5 | 6 | 9 | 10 | 1 | 74 |
| Alemtuzumab, n | 1 | 0 | 0 | 0 | 0 | 0 | 1 |
| Bevacizumab, n* | 10 | 3 | 0 | 6 | 0 | 0 | 1 |
| Brentuximab, n | 7 | 0 | 0 | 0 | 0 | 0 | 7 |
| Cetuximab, n | 13 | 4 | 0 | 0 | 9 | 0 | 0 |
| Daratumumab, n | 1 | 0 | 0 | 0 | 0 | 0 | 1 |
| Isatuximab, n | 1 | 0 | 0 | 0 | 0 | 0 | 1 |
| Nivolumab, n | 6 | 0 | 4 | 0 | 1 | 0 | 1 |
| Olaratumab, n | 1 | 0 | 0 | 0 | 1 | 0 | 0 |
| Panitumumab, n | 1 | 1 | 0 | 0 | 0 | 0 | 0 |
| Pertuzumab, n* | 1 | 0 | 0 | 1 | 0 | 0 | 0 |
| Pembrolizumab, n | 2 | 0 | 1 | 1 | 0 | 0 | 0 |
| Rituximab, n | 71 | 0 | 0 | 0 | 0 | 1 | 70 |
| Tomuzotuximab, n | 1 | 0 | 0 | 0 | 1 | 0 | 0 |
| Ofatumumab, n | 1 | 0 | 0 | 0 | 0 | 0 | 1 |
| Trastuzumab, n* | 2 | 0 | 0 | 2 | 0 | 0 | 0 |
| Programmed death-ligand 1-Antibody, n | 1 | 0 | 1 | 0 | 0 | 0 | 0 |
| Thyrosin kinase inhibitors, n | 13 | 1 | 2 | 1 | 3 | 1 | 5 |
| Afatinib, n* | 2 | 0 | 2 | 0 | 0 | 0 | 0 |
| Axitinib, n* | 0 | 0 | 0 | 0 | 0 | 0 | 0 |
| Ibrutinib, n* | 5 | 0 | 0 | 0 | 0 | 0 | 5 |
| Imatinib, n* | 1 | 0 | 0 | 0 | 0 | 1 | 0 |
| Osimertinib, n* | 2 | 0 | 2 | 0 | 0 | 0 | 0 |
| Pazopanib, n* | 1 | 0 | 0 | 0 | 1 | 0 | 0 |
| Sunitinib, n* | 1 | 0 | 0 | 0 | 1 | 0 | 0 |
| Sorafenib, n* | 2 | 0 | 0 | 0 | 2 | 0 | 0 |
| Nilotinib, n* | 1 | 0 | 0 | 0 | 0 | 1 | 0 |
| Lapatinib, n* | 1 | 0 | 0 | 1 | 0 | 0 | 0 |
| Regorafenib, n* | 1 | 1 | 0 | 0 | 0 | 0 | 0 |
| Other Kinase inhibitors, n | 3 | 0 | 0 | 1 | 0 | 0 | 2 |
| Idelalisib, n | 2 | 0 | 0 | 0 | 0 | 0 | 2 |
| Palbociclib, n | 0 | 0 | 0 | 0 | 0 | 0 | 0 |
| Ribociclib, n | 1 | 0 | 0 | 1 | 0 | 0 | 0 |
| Proteasom inhibitors, n | 17 | 0 | 1 | 0 | 0 | 0 | 16 |
| Bortezomib, n* | 17 | 0 | 1 | 0 | 0 | 0 | 16 |
| Ixazomib, n | 1 | 0 | 0 | 0 | 0 | 0 | 1 |
| Other agents, n | 16 | 0 | 0 | 0 | 4 | 1 | 11 |
| Tretinoin (ATRA), n | 1 | 0 | 0 | 0 | 0 | 1 | 0 |
| Everolimus, n* | 1 | 0 | 0 | 0 | 1 | 0 | 0 |
| Temsirolimus, n* | 3 | 0 | 0 | 0 | 0 | 0 | 3 |
| Mitomycin, n | 4 | 0 | 0 | 0 | 3 | 0 | 1 |
| Bleomycin, n | 4 | 0 | 0 | 0 | 0 | 0 | 4 |
| Venetoclax, n | 1 | 0 | 0 | 0 | 0 | 0 | 1 |
| Lenalidomide, n | 2 | 0 | 0 | 0 | 0 | 0 | 2 |
| Pomalidomide, n | 1 | 0 | 0 | 0 | 0 | 0 | 1 |
| Romidepsin, n | 1 | 0 | 0 | 0 | 0 | 0 | 1 |
| Azacitidine, n | 1 | 0 | 0 | 0 | 0 | 1 | 0 |

| | | | | | | | |
|---|---|---|---|---|---|---|---|
| [0202] Values are n (%). | | | | | | | |

**Table 6 - Association of <20 PVC & ≥20 PVC with relevant variables in all cancer patients (n=261)**

| Variable | **No NSVT ≥4 beats & ≥100 bpm (*n* = 236)** | **NSVT ≥4 beats & ≥100 bpm *(n* = 25)** | **P-value** |
|---|---|---|---|
| Clinical characteristics | | | |
| Age (year) | 67 ± 12 | 65 ± 13 | 0.30 |
| Female sex | 123 (52) | 9 (36) | 0.13 |
| BMI (kg/m²) | 25 ± 5 | 26 ± 7 | 0.26 |
| Cancer stage ≥III, *n* (%) | 166 (70) | 22 (88) | 0.33 |
| Cancer type: solid | 116 (49) | 13 (52) | 0.74 |
| Prior potentially cardiotoxic anti-cancer drugs, *n* (%) | 147 (62) | 15 (60) | 0.82 |
| Left ventricular ejection fraction (%) | 65 ± 7 (*n*=163) | 63 ± 10 (*n*=18) | 0.41 |

| Laboratory parameters | | | |
|---|---|---|---|
| Haemoglobin (g/dL) | 10.9 ± 2.0 | 10.4 ± 1.9 | 0.33 |
| Leucocytes (/nL) | 6.3 (4.4 - 9.7) | 6.9 (3.9 - 9.5) | 0.97 |
| Platelets (/nL) | 228 ± 170 | 204 ± 117 | 0.50 |
| Sodium (mmol/L) | 139 ± 4 | 138 ± 3 | 0.64 |
| Potassium (mmol/L) | 3.9 ± 0.5 | 3.9 ± 0.5 | 0.99 |
| Creatinine (mg/dL) | 0.98 ± 0.53 | 1.16 ± 1.01 | 0.15 |
| GOT (U/L) | 26 (19 - 36) (*n*=161) | 33 (23 - 66) (*n*=10) | 0.27 |

| Secondary diagnoses | | | |
|---|---|---|---|
| Arterial hypertension, *n* (%) | 119 (50) | 13 (52) | 0.88 |
| Coronary artery disease, *n* (%) | 27 (11) | 7 (28) | **0.019** |
| Atrial fibrillation, *n* (%) | 12 (5) | 2 (8) | 0.91 |
| Previous myocardial infarction, *n* (%) | 16 (7) | 4 (16) | 0.61 |
| Diabetes mellitus type 2, *n* (%) | 46 (19) | 4 (16) | 0.92 |
| Chronic kidney disease, *n* (%) | 38 (16) | 3 (12) | 0.89 |
| Previous stroke, *n* (%) | 26 (11) | 1 (4) | 0.52 |
| Current use of antibiotics, *n* (%) | 42 (18) | 3 (12) | 0.79 |

| Medication at study entry | | | |
|---|---|---|---|
| Aspirin, *n* (%) | 56 (24) | 7 (28) | 0.64 |
| Angiotensin-converting enzyme inhibitor, *n* (%) | 46 (19) | 11 (44) | **0.0048** |
| Calcium channel blocker, *n* (%) | 33 (14) | 4 (16) | 0.93 |
| Angiotensin II receptor blocker, *n* (%) | 33 (14) | 3 (12) | 0.93 |
| Beta-blocker, *n* (%) | 107 (45) | 10 (40) | 0.61 |
| Spironolactone, *n* (%) | 5 (2) | 2 (8) | 0.64 |
| Diuretics, *n* (%) | 66 (28) | 5 (20) | 0.39 |
| Anticoagulants, *n* (%) | 82 (23) | 11 (44) | 0.36 |
| Antidiabetics or Insulin, *n* (%) | 27 (11) | 1 (4) | 0.52 |
| Proton pump inhibitors, *n* (%) | 152 (64) | 15 (60) | 0.66 |
| Opioids, *n* (%) | 44 (19) | 2 (8) | 0.44 |
| Antidepressants, *n* (%) | 21 (9) | 2 (8) | 0.97 |
| Corticosteroids, *n* (%) | 69 (29) | 4 (16) | 0.52 |

| Prior chemotherapy, immunotherapy and target therapy | | | |
|---|---|---|---|
| Prior chemotherapy, *n* (%) | 178 (75) | 21 (84) | 0.62 |
| Prior immunotherapy, *n* (%) | 116 (49) | 9 (36) | 0.21 |
| Alkylating agents, *n* (%) | 103 (44) | 10 (40) | 0.73 |
| Platins, *n* (%) | 67 (28) | 7 (28) | 0.97 |
| Anti-metabolites, *n* (%) | 90 (38) | 8 (32) | 0.55 |
| Topoisomerase inhibitors, *n* (%) | 42 (18) | 5 (20) | 0.79 |
| Antracyclines, *n* (%) | 72 (31) | 7 (28) | 0.80 |
| Antimitotics, *n* (%) | 55 (23) | 5 (20) | 0.71 |
| Taxanes, *n* (%) | 28 (12) | 2 (8) | 0.89 |
| Histone deacetylase inhibitor, *n* (%) | 1 (<1) | 0 | 0.76 |
| Antibodies/ Immunotherapy, *n* (%) | 96 (41) | 9 (36) | 0.65 |
| Thvrosin kinase inhibitors, *n* (%) | 13 (6) | 0 | 0.14 |
| Other kinase inhibitors, *n* (%) | 3 (1) | 0 | 0.52 |
| Proteasom inhibitor, *n* (%) | 16 (7) | 1 (24) | 0.89 |
| Other chemotherapeutic agents, *n* (%) | 13 (6) | 3 (22) | 0.75 |

| 24h-ECG | | | |
|---|---|---|---|
| Average 24h heart rate (bpm) | 78 ± 13 | 85 ± 20 | **0.016** |
| No. of premature atrial contraction / 24h | 185 (27 - 1028) | 690 (51 -2073) | 0.09 |
| No. of premature ventricular contraction / 24h | 20 (2 - 279) | 439 (114-3263) | **0.0001** |
| ≥20 Premature ventricular contractions / 24h, *n* (%) | 118 (50) | 21 (84) | 0.09 |
| ≥50 Premature ventricular contractions / 24h, *n* (%) | 97 (41) | 20 (80) | **0.0002** |
| ≥3000 Premature ventricular contractions / 24h, *n* (%) | 17 (7) | 7 (28) | **<0.001** |

Values are means ± SD or n (%). 24h, 24 hours; BMI, body mass index; bpm, beats per minute; GOT, glutamic oxaloacetic transaminase; no., number; NSVT, non-sustained ventricular tachycardia.

**Table 7 - Association of NSVT ≥4 beats & ≥100 bpm with relevant variables in all cancer patients (n=261)**

| Variable | **No NSVT ≥6 beats & ≥100 bpm *(n =*** 249) | **NSVT ≥6 beats & ≥100 bpm** *(n* = 12) | **P-value** |
|---|---|---|---|
| Clinical characteristics | | | |
| Age (year) | 68 ± 12 | 69 ± 14 | 0.66 |
| Female sex | 128 (51) | 4 (33) | 0.77 |
| BMI (kg/m²) | 25 ± 5 | 28 ± 7 | **0.036** |
| Cancer stage ≥III, *n* (%) | 178 (71) | 10 (83) | 0.78 |
| Cancer type: solid | 121 (49) | 8 (67) | 0.77 |
| Prior potentially cardiotoxic anti-cancer drugs, n (%) | 155 (62) | 7 (58) | 0.78 |
| Left ventricular ejection fraction (%) | 65 ± 7 (*n*=171) | 64 ± 11 (*n*=10) | 0.81 |
| Laboratory parameters | | | |
| Haemoglobin (g/dL) | 10.8 ± 2.0 | 11.0 ± 1.8 | 0.69 |
| Leucocytes (/nL) | 6.5 (4.3 - 9.6) | 6.6 (4.4 - 9.1) | 0.91 |
| Platelets (/nL) | 225 ± 168 | 230 ± 105 | 0.91 |
| Sodium (mmol/L) | 139 ± 4 | 140 ± 3 | 0.51 |
| Potassium (mmol/L) | 3.9 ± 0.5 | 4.0 ± 0.5 | 0.70 |
| Creatinine (mg/dL) | 0.99 ± 0.53 | 1.22 ± 1.20 | 0.14 |
| GOT (U/L) ^{c} | 26 (19 - 37) (*n*=166) | 15 (22-43) (*n*=5) | 0.84 |
| Secondary diagnoses | | | |
| Arterial hypertension, *n* (%) | 126 (51) | 6 (50) | 0.97 |
| Coronary artery disease, *n* (%) | 30 (12) | 4 (33) | 0.69 |
| Atrial fibrillation, *n* (%) | 13 (5) | 1 (8) | 0.99 |
| Previous myocardial infarction, *n* (%) | 17 (7) | 3 (25) | 0.69 |
| Diabetes mellitus type 2, *n* (%) | 47 (19) | 3 (25) | 0.99 |
| Chronic kidney disease, *n* (%) | 39 (16) | 2 (17) | 1.00 |
| Previous stroke, *n* (%) | 26 (10) | 1 (8) | 1.00 |
| Current use of antibiotics, n (%) | 43 (17) | 2 (17) | 1.00 |
| Medication at study entry | | | |
| Aspirin, *n* (%) | 60 (24) | 3 (25) | 1.00 |
| Angiotensin-converting enzyme inhibitor, *n* (%) | 53 (21) | 4 (33) | 0.90 |
| Calcium channel blocker, *n* (%) | 35 (14) | 2 (17) | 1.00 |
| Angiotensin II receptor blocker, *n* (%) | 34 (14) | 2 (17) | 1.00 |
| Beta-blocker, *n* (%) | 111 (45) | 6 (50) | 0.71 |
| Spironolactone, *n* (%) | 6 (2) | 1 (8) | 0.95 |
| Diuretics, *n* (%) | 69 (28) | 2 (17) | 0.85 |
| Anticoagulants, *n* (%) | 88 (35) | 5 (42) | 0.65 |
| Antidiabetics or Insulin, *n* (%) | 27 (11) | 1 (8) | 1.00 |
| Proton pump inhibitors, *n* (%) ^{b} | 162 (65) | 5 (42) | 0.10 |
| Opioids, *n* (%) | 45 (18) | 1 (8) | 0.78 |
| Antidepressants, *n* (%) | 22 (9) | 1 (8) | 1.00 |
| Corticosteroids, *n* (%) | 71 (29) | 2 (17) | 0.78 |
| Prior chemotherapy, immunotherapy and target therapy | | | |
| Prior chemotherapy, *n* (%) | 188 (76) | 11 (92) | 0.65 |
| Prior immunotherapy, *n* (%) | 121 (49) | 4 (33) | 0.78 |
| Alkylating agents, *n* (%) | 108 (43) | 5 (42) | 0.91 |
| Platins, *n* (%) | 68 (27) | 6 (50) | 0.09 |
| Anti-metabolites, *n* (%) | 95 (38) | 3 (25) | 0.78 |
| Topoisomerase inhibitors, *n* (%) | 43 (17) | 4 (33) | 0.78 |
| Antracyclines, n (%) | 76 (31) | 3 (25) | 0.99 |
| Antimitotics, *n* (%) | 57 (23) | 3 (25) | 1.00 |
| Taxanes, *n* (%) | 29 (12) | 1 (8) | 0.99 |
| Histone deacetylase inhibitor, *n* (%) | 1 (<1) | 0 | 0.83 |
| Antibodies/ Immunotherapy, *n* (%) | 102 (41) | 3 (25) | 0.78 |
| Thyrosin kinase inhibitors, *n* (%) | 13 (5) | 0 | 0.37 |
| Other kinase inhibitors, *n* (%) | 3 (1) | 0 | 0.69 |
| Proteasom inhibitors, *n* (%) | 17(7) | 0 | 0.29 |
| Other chemotherapeutic agents, *n* (%) | 15(6) | 1(8) | 1.00 |
| 24h-ECG | | | |
| Average 24h heart rate (bpm) | 78 ± 14 | 81 ± 16 | 0.49 |
| No. of premature atrial contraction / 24h | 178 (27 - 1088) | 1204 (214 -2680) | **0.043** |
| No. of premature ventricular contraction / 24h | 23 (2 - 336) | 354 (153 - 797) | **0.023** |
| ≥20 Premature ventricular contractions / 24h, *n* (%) | 129 (52) | 10 (83) | 0.50 |
| ≥50 Premature ventricular contractions / 24h, *n* (%) | 107 (43) | 10 (83) | 0.37 |
| ≥3000 Premature ventricular contractions / 24h, *n* (%) | 23 (9) | 1 (8) | 1.00 |

Values are means ± SD or n (%). 24h, 24 hours; BMI, body mass index; bpm, beats per minute; GOT, glutamic oxaloacetic transaminase; no., number; NSVT, non-sustained ventricular tachycardia. P-values are determined using the unpaired t-test.

**Table 8 - Association of NSVT ≥6 beats & ≥100 bpm with relevant variables in all cancer patients (n=261)**

| **Variable** | **<20 PVC *(n* = 122)** | **≥20 PVC *(n* = 139)** | **P-value** |
|---|---|---|---|
| Clinical characteristics | | | |
| Age (year) | 65 ± 13 | 70 ± 11 | **0.0018** |
| Male sex | 73 (60) | 59 (42) | **0.0051** |
| BMI (kg/m²) | 21 ± 5 | 25 ± 5 | 0.94 |
| Cancer stage ≥III, *n* (%) | 93 (76) | 95 (68) | 0.16 |
| Cancer type: solid | 61 (50) | 68 (49) | 0.86 |
| Prior potentially cardiotoxic anti-cancer drugs, *n* (%) | 71 (58) | 91 (65) | 0.23 |
| Left ventricular ejection fraction (%) | 65 ± 7 (w=89) | 64 ± 8 (*n*=92) | 0.38 |
| Laboratory parameters | | | |
| Haemoglobin (g/dL) | 10.9 ± 2.1 | 10.7 ± 2.0 | 0.52 |
| Leucocytes (/nL) | 6.1 (4.3 - 9.1) | 6.8 (4.4 - 9.6) | 0.36 |
| Platelets (/nL) | 218 ± 121 | 232 ± 197 | 0.52 |
| Sodium (mmol/L) | 139 ± 4 | 139 ± 4 | 0.86 |
| Potassium (mmol/L) | 3.9 ± 0.5 | 4.0 ± 0.6 | 0.12 |
| Creatinine (mg/dL) | 1.01 ± 0.66 | 0.99 ± 0.52 | 0.83 |
| GOT (U/L) | 24 (18 - 36) (n=83) | 28 (20 - 37) (n=88) | 0.12 |
| Secondary diagnoses | | | |
| Arterial hypertension, *n* (%) | 55 (45) | 87 (63) | **0.0046** |
| Coronary artery disease, n (%) | 8 (7) | 26 (19) | **0.0036** |
| Atrial fibrillation, *n* (%) | 4 (3) | 10 (7) | 0.17 |
| Previous myocardial infarction, *n* (%) | 4 (3) | 16 (12) | **0.010** |
| Diabetes mellitus type 2, *n* (%) | 22 (18) | 28 (20) | 0.67 |
| Chronic kidney disease, *n* (%) | 15 (12) | 26 (19) | 0.16 |
| Previous stroke, *n* (%) | 13 (21) | 14 (10) | 0.88 |
| Current use of antibiotics, *n* (%) | 19 (16) | 26 (19) | 0.50 |
| Medication at study entry | | | |
| Aspirin, *n* (%) | 27 (22) | 36 (26) | 0.48 |
| Angiotensin-converting enzyme inhibitor, *n* (%) | 20 (16) | 37 (27) | **0.046** |
| Calcium channel blocker, *n* (%) | 18 (15) | 19 (14) | 0.80 |
| Angiotensin II receptor blocker, *n* (%) | 12 (10) | 24 (17) | 0.08 |
| Beta-blocker, *n* (%) | 53 (43) | 64 (46) | 0.67 |
| Spironolactone, *n* (%) | 3 (2) | 4 (3) | 0.85 |
| Diuretics, *n* (%) | 32 (26) | 39 (28) | 0.74 |
| Anticoagulants, *n* (%) | 39 (32) | 54 (39) | 0.25 |
| Antidiabetics or Insulin, *n* (%) | 17 (14) | 11 (8) | 0.12 |
| Proton pump inhibitors, *n* (%) | 80 (66) | 87 (63) | 0.62 |
| Opioids, *n* (%) | 19 (16) | 27 (19) | 0.42 |
| Antidepressants, *n* (%) | 13 (11) | 10 (7) | 0.32 |
| Corticosteroids, *n* (%) | 34 (28) | 39 (28) | 0.97 |
| Prior chemotherapy, immunotherapy and target therapy | | | |
| Prior chemotherapy, n (%) | 87 (71) | 112 (81) | 0.08 |
| Prior immunotherapy, *n* (%) | 59 (48) | 66 (47) | 0.89 |
| Alkylating agents, *n* (%) | 48 (39) | 65 (47) | **0.021** |
| Platins, *n* (%) | 37 (30) | 37 (27) | 0.56 |
| Anti-metabolites, *n* (%) | 47 (39) | 51 (37) | 0.56 |
| Topoisomerase inhibitors, n (%) | 26 (21) | 21 (15) | 0.38 |
| Antracvclines, *n* (%) | 35 (29) | 44 (32) | 0.60 |
| Antimitotics, n (%) | 28 (23) | 32 (23) | 0.31 |
| Taxanes, n (%) | 12 (10) | 18 (13) | 0.39 |
| Histone deacetylase inhibitor, *n* (%) | 0 | 1 (1) | 0.46 |
| Antibodies/ Immunotherapy, *n* (%) | 53 (43) | 52 (37) | 0.27 |
| Thyrosin kinase inhibitors, *n* (%) | 9 (7) | 4 (3) | 0.12 |
| Other kinase inhibitors, *n* (%) | 3 (2) | 0 | 0.09 |
| Proteasom inhobitors, n (%) | 6 (5) | 11 (8) | 0.71 |
| Other chemotherapeutic agents, *n* (%) | 6 (5) | 10 (7) | 0.95 |
| 24h-ECG | | | |
| Average 24h heart rate (bpm) | 77 ± 15 | 80 ± 13 | **0.042** |
| No. of premature atrial contraction / 24h | 53 (20 - 353) | 543 (100 - 2287) | **<0.0001** |
| No. of premature ventricular contraction / 24h | 2 (0 - 7) | 297 (87 - 1312) | **<0.0001** |
| NSVT ≥4 beats & ≥100 bpm, *n* (%) | 4 (3) | 21 (15) | **<0.0001** |
| NSVT ≥6 beats & ≥100 bpm, *n* (%) | 2 (2) | 10 (7) | **0.028** |

Values are means ± SD or n (%). 24h, 24 hours; BMI, body mass index; bpm, beats per minute; GOT, glutamic oxaloacetic transaminase; no., number; NSVT, non-sustained ventricular tachycardia.

**Table 9 - Additional non-significant univariable survival analyses in cancer patients (n=261)**

| **Variable** | **Univariable model** | | | |
|---|---|---|---|---|
| | **HR** | **95% CI** | 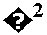 | **P- value** |
| Premature atrial contractions (per 1000 / 24h) | 1.00 | 0.96 - 1.04 | <0.01 | 1.00 |
| Left ventricular ejection fraction (%) | 0.97 | 0.95 - 1.004 | 2.7 | 0.10 |
| Leucocytes (per 1/nL) | 1.01 | 0.992 - 1.02 | 0.7 | 0.39 |
| Platelets (per 1000/nL) | 1.13 | 0.40 - 1.87 | 0.1 | 0.73 |
| Creatinine (per 1mg/dL) | 1.16 | 0.9 - 1.49 | 1.3 | 0.26 |
| GOT (per 1U/L) (*n* = 171) | 1.003 | 0.999- 1.01 | 1.9 | 0.17 |
| Arterial hypertension (yes vs no) | 0.94 | 0.69 - 1.29 | 0.1 | 0.71 |
| Atrial fibrillation (yes vs no) | 1.22 | 0.60 - 2.50 | 0.3 | 0.57 |
| Previous myocardial infarction (yes vs no) | 0.95 | 0.54 - 1.68 | 0.03 | 0.86 |
| Diabetes mellitus type 2 (yes vs no) | 0.95 | 0.64 - 1.40 | 0.08 | 0.78 |
| Chronic kidney disease (yes vs no) | 1.26 | 0.83 - 1.89 | 1.2 | 0.28 |
| Previous stroke (yes vs no) | 0.86 | 0.517 -1.42 | 0.4 | 0.55 |
| Diuretics (yes vs no) | 1.16 | 0.82 - 1.64 | 0.7 | 0.40 |
| Anticoagulation (yes vs no) | 1.31 | 0.95 - 1.81 | 2.7 | 0.10 |

Hazard ratios are presented for continuously or binomially distributed variables. 24h, 24 hours; bpm, beats per minute; CI, Confidence interval. GOT, glutamic oxaloacetic transaminase. HR, hazard ratio; g/dL, grams per deciliter; Units per liter; /nL, per nanoliter; ms, milliseconds.

### Example 4: Use of a loop recorder to determine a risk of a cancer

A healthcare practitioner determines a patient to have cancer or to be at risk of developing a cancer. The healthcare practitioner surgically inserts a loop recorder into the patient. The loop recorder monitors various cardiopulmonary measurements. The loop recorder is used to detect a tachycardia or non-sustained ventricular tachycardia of at least about 100 bpm.

Upon detection of the non-sustained ventricular tachycardia of at least about 100 bpm, the loop recorder transmits the data to a second device by wireless transmission. The data is analyzed using an algorithm encoded on the computer readable memory of the second device which compares the measurements to a reference level. The second device alerts a healthcare practitioner that the patient has an increased risk of developing cancer.

The practitioner conducts a genetic sequencing test of a panel of cancer related genes in the patients' genomic DNA to determine whether the patient has a mutation pre-disposing them to an increased risk of a certain type of cancer. The test shows the patient to have a mutation pre-disposing them to cancer. The healthcare practitioner conducts further diagnostic tests to determine the presence of cancer in the individual including screening for circulating tumor DNA in the blood of the patient. The test shows a positive result for circulating tumor DNA.

Accordingly, the detection of the non-sustained ventricular tachycardia of at least about 100 bpm has significant predictive value in predicting the presence of the cancer. The healthcare practitioner then administers an anti-neoplastic therapeutic to the patient based on the positive detection of the circulating tumor DNA.

### Example 5: Use of an implantable cardioverter defibrillator (ICD) device to determine a risk of cancer

A healthcare practitioner determines a patient to have cancer or to be at risk of developing a cancer. The healthcare practitioner surgically inserts an implantable cardioverter defibrillator (ICD) device into the patient. The ICD device monitors various cardiopulmonary measurements. The ICD device is used to detect about 10 to about 50 premature ventricular contractions per day.

Upon detection of about 10 to about 50 premature ventricular contractions per day, the ICD device transmits the data to a second device by wired transmission. The data is analyzed using an algorithm encoded on the computer readable memory of the second device which compares the measurements to a reference level. The second device alerts a healthcare practitioner that the patient has an increased risk of developing cancer.

The practitioner conducts a genetic sequencing test of a panel of cancer related genes in the patients' genomic DNA to determine whether the patient has a mutation pre-disposing them to an increased risk of a certain type of cancer. The test shows the patient to have a mutation pre-disposing them to cancer. The healthcare practitioner conducts further diagnostic tests to determine the presence of cancer in the individual including screening for circulating tumor DNA in the blood of the patient. The test shows a positive result for circulating tumor DNA.

Accordingly, the detection of about 10 to about 50 premature ventricular contractions per day has significant predictive value in predicting the presence of the cancer. The healthcare practitioner then administers an anti-neoplastic therapeutic to the patient based on the positive detection of the circulating tumor DNA.

### Example 6: Use of a wearable device to determine a risk of cancer

A healthcare practitioner determines a patient to have cancer or to be at risk of developing a cancer. The patient is prescribed the use of a wearable device that can perform cardiopulmonary measurements. The patient continuously wears the wearable device which monitors various cardiopulmonary measurements over time. The wearable device is used to detect an average heart rate during a one-hour period of from about 60 to about 80 bpm combined with about 5-minute epochs of a standard deviation of the 5-minute average NN interval (SDANN) during the hour that vary from less than about 150 msec to less than about 100 msec.

Upon detection of the average heart rate during a one-hour period of from about 60 to about 80 bpm combined with about 5 minute epochs of a standard deviation of the 5-minute average NN interval (SDANN) during the hour that vary from less than about 150 msec to less than about 100 msec, the wearable device transmits the data to a second device by wired transmission. The data is analyzed using an algorithm encoded on the computer readable memory of the second device which compares the measurements to a reference level. The second device alerts a healthcare practitioner that the patient has an increased risk of developing cancer.

The practitioner conducts a genetic sequencing test of a panel of cancer related genes in the patients' genomic DNA to determine whether the patient has a mutation pre-disposing them to an increased risk of a certain type of cancer. The test shows the patient to have a mutation pre-disposing them to cancer. The healthcare practitioner conducts further diagnostic tests to determine the presence of cancer in the individual including screening for circulating tumor DNA in the blood of the patient. The test shows a positive result for circulating tumor DNA.

Accordingly, the detection of an average heart rate during a one-hour period of from about 60 to about 80 bpm combined with about 5 minute epochs of a standard deviation of the 5-minute average NN interval (SDANN) during the hour that vary from less than about 150 msec to less than about 100 msec has significant predictive value in predicting the presence of the cancer. The healthcare practitioner then administers an anti-neoplastic therapeutic to the patient based on the positive detection of the circulating tumor DNA.

### Example 7: Use of a loop recorder to determine a risk of cancer

A healthcare practitioner determines a patient to have cancer or to be at risk of developing a cancer. The healthcare practitioner surgically inserts a loop recorder into the patient. The loop recorder monitors various cardiopulmonary measurements. The loop recorder is used to detect a standard deviation of the 5-minute average NN interval (SDANN) of less than about 100 msec combined with an HR of less than about 80 bpm, a pNN50 less than 10% simultaneous with a standard deviation of the 5-minute average NN interval (SDANN) of more than about 100 msec.

Upon detection of a standard deviation of the 5-minute average NN interval (SDANN) of less than about 100 msec combined with an HR of less than about 80 bpm, a pNN50 less than 10% simultaneous with a standard deviation of the 5-minute average NN interval (SDANN) of more than about 100 msec, the loop recorder transmits the data to a second device by wireless transmission. The data is analyzed using an algorithm encoded on the computer readable memory of the second device which compares the measurements to a reference level. The second device alerts a healthcare practitioner that the patient has an increased risk of developing cancer.

The practitioner conducts a genetic sequencing test of a panel of cancer related genes in the patients' genomic DNA to determine whether the patient has a mutation pre-disposing them to an increased risk of a certain type of cancer. The test shows the patient to have a mutation pre-disposing them to cancer. The healthcare practitioner conducts further diagnostic tests to determine the presence of cancer in the individual including screening for circulating tumor DNA in the blood of the patient. The test shows a positive result for circulating tumor DNA.

Accordingly, the detection of a standard deviation of the 5-minute average NN interval (SDANN) of less than about 100 msec combined with an HR of less than about 80 bpm, a pNN50 less than 10% simultaneous with a standard deviation of the 5-minute average NN interval (SDANN) of more than about 100 msec has significant predictive value in predicting the presence of the cancer. The healthcare practitioner then administers an anti-neoplastic therapeutic to the patient based on the positive detection of the circulating tumor DNA.

### Example 8: Use of an implantable cardioverter defibrillator device (ICD) to determine a risk of and treat sudden cardiac death in a patient with cancer

A healthcare practitioner determines a patient to have cancer or to be at risk of developing a cancer. The healthcare practitioner surgically inserts an implantable cardioverter defibrillator (ICD) device into the patient. The implantable cardioverter defibrillator (ICD) device monitors various cardiopulmonary measurements. The implantable cardioverter defibrillator (ICD) device is used to detect a tachycardia or non-sustained ventricular tachycardia of at least about 100 bpm, or about 10 to about 50 premature ventricular contractions per day, or an average heart rate during a one-hour period of from about 60 to about 80 bpm combined with about 5 minute epochs of a standard deviation of the 5-minute average NN interval (SDANN) during the hour that vary from less than about 150 msec to less than about 100 msec, or a standard deviation of the 5-minute average NN interval (SDANN) of less than about 100 msec combined with an HR of less than about 80 bpm, a pNN50 less than 10% simultaneous with a standard deviation of the 5-minute average NN interval (SDANN) of more than about 100 msec.

Upon detection of one of these criteria the implantable cardioverter defibrillator transmits the data to a second device by wireless transmission. The data is analyzed using an algorithm encoded on the computer readable memory of the second device which compares the measurements to a reference level. The second device alerts a healthcare practitioner that the patient has an increased risk of sudden cardiac death or another adverse cardiac event.

The ICD is used to prevent cardiac arrest or an irregular heart beat by performing antitachycardia or acting as a pacemaker. When an irregular heart rate is detected the ICD delivers low-energy pacing for mild disruptions to cardiac function, cardioversion therapy for more serious heart rhythm problems, and defibrillation therapy to restore normal heartbeat during the most serious disruptions to cardiac function.

### Example 9: Use of an implantable cardioverter defibrillator device (IDC) to monitor cancer in a patient and determine cancer treatment efficacy

A healthcare practitioner determines a patient to have cancer or to be at risk of developing a cancer. The healthcare practitioner surgically inserts an implantable cardioverter defibrillator (ICD) device into the patient. The implantable cardioverter defibrillator (ICD) device monitors various cardiopulmonary measurements. The implantable cardioverter defibrillator (ICD) device is used to detect a tachycardia or non-sustained ventricular tachycardia of at least about 100 bpm, or about 10 to about 50 premature ventricular contractions per day, or an average heart rate during a one-hour period of from about 60 to about 80 bpm combined with about 5 minute epochs of a standard deviation of the 5-minute average NN interval (SDANN) during the hour that vary from less than about 150 msec to less than about 100 msec, or a standard deviation of the 5-minute average NN interval (SDANN) of less than about 100 msec combined with an HR of less than about 80 bpm, a pNN50 less than 10% simultaneous with a standard deviation of the 5-minute average NN interval (SDANN) of more than about 100 msec.

Upon detection of one of these criteria the implantable cardioverter defibrillator transmits the data to a second device by wireless transmission. The data is analyzed using an algorithm encoded on the computer readable memory of the second device which compares the measurements to a reference level, and to previous levels of the patient's cardiac function recorded by the device. The algorithm correlates the cardiopulmonary measurements with the progression of the cancer, generating a report for a healthcare practitioner regarding the progression of the cancer.

The healthcare practitioner correlates the data generated from the device regarding the progression of the cancer with the current course of anti-cancer treatment to determine the effectiveness of the anti-cancer treatment. Based on the effectiveness of this treatment the healthcare practitioner makes a decision to increase the dosage or frequency of the treatment, decrease the dosage or frequency of the treatment, change the course of treatment to another drug or therapy, or cease the treatment if the cancer is in remission.

### Example 10: A prospective study of the effect of the heart drug, ivabradine, on patients with advanced cancer

In this study an effect of a use of a heart drug in advanced cancer was studied. In some cases, a heart drug can comprise ivabradine. In some cases, ivabradine can be a selective antagonist of myocardial I*f* (funny) channels. In some cases, ivabradine can possess anti-anginal and anti-ischemic properties. In some cases, ivabradine can provide pure heart rate reduction, reducing a diastolic depolarization slope, without altering other cardiac and hemodynamic parameters. In some cases, ivabradine can be effective for a treatment of an ischemic heart disease. In some cases, ivabradine can reduce episodes of both symptomatic angina pectoris and myocardial ischemia, and in heart failure where it can reduce mortality and morbidity. In some cases, it may only be approved or recommended for treatment of these two conditions. In some cases, ivabradine may have never been recommended for a treatment of a cancer patient. Disclosed herein in some embodiments, are methods in which ivabradine can be administered for a treatment of a cancer patient.

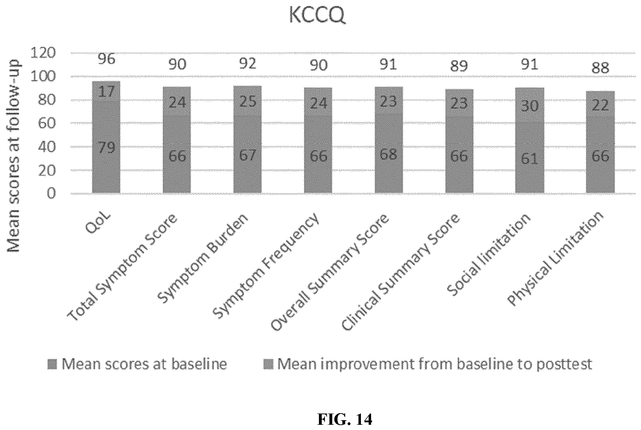

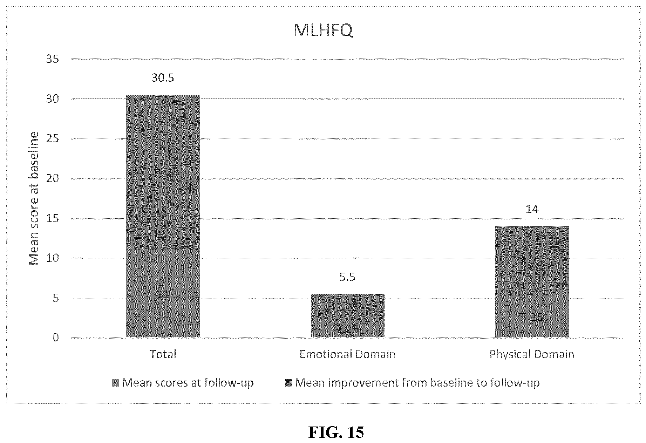

In this study 4 cancer patients with no known heart failure and with normal left ventricular function (LEF>50%) were studied. They all had histologically confirmed diagnoses of cancer (see table 1), and all suffered poor quality of life, with three showing a Karnofksy performance score of 70-80% (indicating an inability to carry on normal activity or to do active work) and one showing a Karnofksy performance score of 50-60% (indicating a need for considerable assistance and frequent medical care). The present disclosure demonstrates the unexpected finding that the elevated heart rate, (>70 beats per minute) seen in all 4 patients may indicate an unexpected wasting of heart muscle and that ivabradine may therefore be useful, in a way that is analogous to how it is useful in the treatment of heart failure. All were given Ivabradine at a starting dose of 5 mg twice a day. Heart rate was reduced in all patients from 104.5 ± 10.4 bpm to 77.0 ± 10.9 bpm. In these 4 patients the addition of ivabradine importantly improved symptoms as measured by two well validated symptom scores based on questionnaires that concentrate on breathlessness and fatigue, the main symptoms of heart failure, and the heart muscle wasting associated with cancer that we have described in this patient. The questionnaires are the Minnesota Living with Heart Failure Questionnaire (MLHFQ) (see figure 15) and the Kansas City Cardiomyopathy Questionnaire (KCCQ) (see figure 14). In the MI,HFQ higher scores indicate a better quality of life and in the MLWHFQ lower scores indicate a better quality of life. The two figures show the clinically important improvement in symptoms and quality of life achieved in these patient by the use of the heart failure drug Ivabradine. This showed that detecting an abnormal cardiopulmonary signal (in this case an elevated heart rate) can identify a group of cancer patients who will benefit from the use of heart failure drugs that would not otherwise be used (see **Table 10).**

**Table 10**

| **Age (years)** | **Gender** | **chemotherapy** | **Cancer type** | **Karnovsky score** |
|---|---|---|---|---|
| 75 | male | MATRIX, then PRIMAIN | High-grade B-cell lymphoma | 70-80% |
| 67 | male | Docetaxel, Carbitaxel | Hodgkin lymphoma AND metastatic prostate cancer | 50-60% |
| 65 | male | Alemtuzumab, Fludarabin, allogene Stx | T-cell prolymphocytic leukemia | 70-80% |
| 71 | male | Cisplatin/Etoposid, Sunitinib | Metastatic urothelial carcinoma | 70-80% |

### Example 11: Use of a heart failure treatment, Ghrelin, to improve physical activity without improving skeletal muscle in advanced cancer.

In some cases, administration of Ghrelin, a gut-derived 28-amino acid peptide hormone, can show beneficial effects in subjects with heart failure. In some cases, administration of Ghrelin can increase a size of a cardiomyocyte, prolong a cardiomyocyte survival, protect a cardiomyocyte against apoptosis, or any combination thereof. In some cases, use of a method disclosed herein can comprise using Ghrelin as a heart-directed treatment to treat advanced cancer where there is heart muscle (and hence cardiomyocyte) wasting due to apoptosis and a reduction in a size of a cardiomyocyte. In some cases, a heart treatment can benefit patients with advanced cancer using methods disclosed herein.

In some cases, a patient with advanced cancer, but no known heart failure, can achieve symptomatic and physical activity benefit from an administration of Ghrelin, despite no improvement in skeletal muscle mass or strength. In some cases, administration of Ghrelin can comprise a heart treatment that would benefit an advanced cancer patient by preventing loss of heart muscle mass.

Blum et al. Journal of Cachexia, Sarcopenia and Muscle (2021 early access) described several patients (Case A, B, C, and D) with advanced cancer receiving Ghrelin thus:

### Case A:

"A 61-year-old male with pancreatic cancer, which had metastasized to the liver, presented with a weight of 75 kg and Body Mass Index (BMI) of 25.4. In the previous six months, he had lost 17 kg, 15 kg of which had been lost in the previous two months. He received Gemcitabine. He had a Karnofsky score of 80 and an Eastern Cooperative Oncology Group (ECOG) score of 1."

"Comparing pre-study measurements to post-study measurements, he exhibited no change in muscle mass, a 2% increase in muscle strength, a 40% increase in steps/day."

The dramatic increase in physical activity (40% increase in steps/day) seen in this case, with no change in peripheral muscle mass and a negligible increase in peripheral muscle strength combined with the inventive step disclosed herein, can be seen as evidence that the Ghrelin had increased the patient's heart muscle mass and thereby improved his level of physical activity, because there was no improvement in peripheral muscle, an effect more commonly associated with Ghrelin.

### Case B:

"A 76-year-old male with gastrointestinal cancer, which had metastasized to the liver, presented with a weight of 48 kg and a BMI of 16.0. In the previous six months, he had lost 14 kg, 13 of which had been lost in the previous two months. He received Capecitabine. He had a Karnofsky score of 60 and an ECOG score of 2. He made the following comment about using ghrelin: "Feel more active."

The subject "was hospitalized for atrial fibrillation, which was an AE deemed unrelated to ghrelin."

The new onset of atrial fibrillation combined with the inventive step disclosed herein could allow a conclusion that the patient developed heart muscle wasting and that "feeling more active" after Ghrelin treatment shows the benefit of using Ghrelin as a result of the inventive disclosure of the importance of detecting and treating heart muscle wasting in advanced cancer.

### Case C:

"A 63-year-old male with advanced mesothelioma presented with a weight of 70 kg and a BMI of 22.6. Data regarding weight loss in the previous six months was not available. He received FOLFIRI. He had a Karnofsky score of 60 and an ECOG score of 1."

His "opinion about using ghrelin was positive, saying, "This is the first therapy which improves my well-being." (He) experienced an increase in tumor pain, an AE deemed unrelated to ghrelin. Comparing pre-study measurements to post-study measurement, he exhibited a 3% decrease in muscle mass. Post-study measurements for muscle strength, steps/day could not be obtained. This patient survived 62 days."

The dramatic report of improved well-being seen in this case, despite a decrease in peripheral muscle mass combined with the inventive step disclosed herein, can be seen as evidence that the Ghrelin had increased the patient's heart muscle mass and thereby improved his feeling of well-being.

### Case D:

"A 57-year-old male with head & neck cancer, which had metastasized to the lungs, presented with a weight of 59 kg and a BMI of 18.6. He had lost 19 kg in the previous six months, including three kg of weight gain in the previous two months. He received Cetuximab. He had a Karnofsky score of 90 and an ECOG score of 1."

"Comparing pre-study measurements to post-study measurements, he exhibited no change in muscle mass, a 6% increase in muscle strength, a 63% increase in steps/day."

The dramatic increase in physical activity (a 63% increase in steps/day) seen in this case, with no change in peripheral muscle mass and a small increase in peripheral muscle strength combined with the inventive step disclosed herein, can be seen as evidence that the Ghrelin had increased the patient's heart muscle mass and thereby improved his level of physical activity.

### Example 12: Evidence that cancer patients who are not taking drugs which are beneficial for heart failure are not protected against heart muscle wasting, poor exercise performance, ventricular tachycardia and early death

Consistent with the findings in example 1, in which the presence and predictive impact of non-sustained ventricular tachycardia (NSVT) and premature ventricular contractions (PVC) was shown to be associated with advanced cancer, it was found that cancer patients who are not taking drugs which are beneficial for heart failure are not protected against heart muscle wasting, poor exercise performance, ventricular tachycardia and early death.

### CASE 1:

A subject with Colorectal Cancer, UICC stage 4, received the following medication: L-Thyroxine 0.1 mg (for hypothyroidism), Amitryptiline 10 mg (for depression) Pantoprazole 40 mg (for acid reflux), Allopurinol 300 mg (for Gout), Emend 125 mg (for chemotherapy-associated nausea), Ondasentron 16 mg (for cancer therapy-associated nausea), Furosemide I.V. 60 mg (for fluid retention) , and Dexamethasone 6 mg (as a cancer adjuvant therapy). An echocardiogram showed a normal LVEF 50%, but reduced LV mass 216 gram. Self-rated health was good, being rated as 2 (1 =very good, 2=good, 3= fair, 4=poor, 5=very poor). A 24h ECG recording showed an elevated mean heart rate of 91 beats per minute and a reduced heart rate variability (SDNN 96 ms) and 1 episode of non-sustained ventricular tachycardia was recorded (of 3 beats and a maximal heart rate of 115 beats/minute). The patient died after 158 days. The patient was very weak and with an ability to exercise that was very limited, taking 13.2 seconds to walk 4 meters and being unable to perform a 6 minute walk test.

Interpretation: This patient showed evidence of abnormal cardio-pulmonary measurements and heart muscle wasting due to cancer and this caused severe limitation in exercise capacity. This condition went untreated with effective heart failure drugs leading to life-threatening non-sustained ventricular tachycardia and an early death.

### CASE 2:

A subject with Lymphoma, Ann Arbor Stage 1E, received the following medication: Amlodipine 5mg (for hypertension), Amphotericin B 100mg/ml 4-6/day (for protection against fungal infection), Dexamethasone 1mg (as a cancer adjuvant therapy), Ranitidine hydrochloride 167mg (for acid reflux), folic acid cefuroxime 4500mg (to prevent bacterial infection) and Dalteparin sodium 5000 I.U. (to prevent thrombosis).

An echocardiogram showed a normal LVEF 68% but reduced LV mass (194 gram). The patient had a good self-rated health of 2 (1 =very good, 2=good, 3= fair, 4=poor, 5=very poor). A 24h ECG recording showed an elevated mean heart rate of 75 beats per minute and a borderline heart rate variability (SDNN 134 ms) and 1 episode of non-sustained ventricular tachycardia was recorded (of 3 beats and a maximal heart rate of 127 beats/minute). The patient was very weak and was unable to exercise being unable to perform a 6 minute walk test and showing a weak maximal handgrip strength of 12 kg. The patient died after 711 days.

Interpretation: This patient showed evidence of abnormal cardio-pulmonary measurements and heart muscle wasting due to cancer and this was associated with severe weakness. This condition went un-treated with effective heart failure drugs and led to life-threatening non-sustained ventricular tachycardia and death within two years.

### CASE 3:

A male Lymphoma patient, Ann Arbor stage 4 B, received the following medication: Pantozol 40mg (for acid reflux), MST 20mg (for pain relief), Aciclovir (to prevent viral infection).

His self-rated health was very poor 5 (1=very good, 2=good, 3= fair, 4=poor, 5=very poor). His 24 ECG showed an elevated heart rate (mean 104 bpm) and reduced heart rate variability (SDNN 65ms) indicative of cancer-associated heart muscle wasting, and he was extremely weak with a very low hand grip strength (17kg). He was too weak to even walk for 6 minutes at even a very slow pace. He died after 11 days.

Interpretation: This patient showed evidence of abnormal cardio-pulmonary measurements and heart muscle wasting due to cancer and these went un-treated leading to severe weakness, an inability to walk and an early death.

### CASE 4:

A female patient with Lung Cancer, UICC stage 4, received medication of pantozol 40mg (for acid reflux) Dexamethasone 2mg (as cancer supportive therapy), Novalgin 160mg and Morphine 50mg (for pain relief). An echocardiogram showed a normal LVEF 66% but reduced LV mass (109 gram). Her self-reported health was very poor, rated at 5 (1=very good, 2=good, 3= fair, 4=poor, 5=very poor). She was too weak to perform either a hand-grip test or to walk for 6 minutes. She died after 14 days.

Interpretation: This patient showed evidence of abnormal heart muscle wasting due to cancer and this went un-treated leading to severe weakness and death within two weeks.

### CASE 5:

A patient with Cervix Cancer, UICC stage 4, was receiving the following medication: Mirtazapin 7.mg (antidepressant), Pantozol 40 (for acid reflux), Zofran 24mg (anti-emetic), Palladon 56mg (pain killer) and Pregabalin 125mg (an anti-convulsant). An echocardiogram showed a normal LVEF of 51%. A 24 ECG recording showed an elevated heart rate (mean 91 bpm) and reduced heart rate variability (SDNN 98ms) suggestive of cancer-associated heart muscle wasting. The patient had considerable weakness with a very low strength on hand-grip testing (12kg) and very slow walking, taking 5.3 seconds to walk 4 meters. The patient died after 49 days.

Interpretation: This patient showed evidence of abnormal heart muscle due to cancer and this went un-treated with effective heart failure drugs, leading to severe weakness and death within two weeks.

All the above patients suffered from undetected and untreated cancer-related heart muscle wasting and its associated poor outcomes.

### Conclusion

All the above patients suffered from undetected and untreated cancer-related heart muscle wasting and its associated poor outcomes. The disclosure herein of a new heart condition (cancer-associated heart muscle wasting) explains these clinical courses. Disclosed herein are new ways to treat and thereby improve these patients as demonstrated in the following series of cases, describing those who by chance were receiving drugs that protect patients from heart failure-like syndromes.

### Example 13: Evidence that patients with advanced cancer who were receiving drugs that improve heart failure-like syndromes are protected against heart muscle wasting, poor exercise performance, ventricular tachycardia and early death, even though they have similar cancers to those who were not receiving these drugs. These include the known heart failure treatments Beta-Blockers, ACE inhibitors, angiotensin receptor blockers and the two SGLT2 inhibitors, Dapagliflozin or Empagliflozin.

Consistent with the findings in example 1, in which the presence and predictive impact of non-sustained ventricular tachycardia (NSVT) and premature ventricular contractions (PVC) was shown to be associated with advanced cancer, it was found that cancer patients who are not taking drugs which are beneficial for heart failure are not protected against heart muscle wasting, poor exercise performance, ventricular tachycardia and early death.

In some cases, SGLT2 inhibitors, Dapagliflozin or Empagliflozin can be used for treatment of type 2 diabetes, heart failure, or a combination thereof. Disclosed herein are methods comprising administering these drugs to advanced cancer patients to improve a heart muscle in these patients. We here describe 5 advanced cancer patients, who were treated with either an SGLT2 inhibitor, an ACE inhibitor, a beta-blocker or an Angiotensin receptor blocker, all of which are shown to be effective heart failure therapies.

### CASE 6:

A male patient with Colorectal Cancer, UICC stage 4, received the following medication: Metamizole 1500 mg (for pain relief), Candesartan 16mg (an angiotensin receptor blocker as claimed in claims 28 and 39, which in this case was prescribed as a treatment for hypertension, and not for his cancer affected heart) and Hydrochlorothiazide 12.5mg (for hypertension), Sitagliptin 128 mg, and Dapagliflozin 10 mg (an SGLT2 inhibitor, which in this case was prescribed as a treatment for his diabetes, and not for his cancer affected heart).

Echocardiogram showed a normal LVEF (68%) and LV mass (247 gram). The patient had a poor self-rated health of 4 (1=very good, 2=good, 3= fair, 4=poor, 5=very poor). Exercise capacity and strength was good with a hand grip strength of 46 kg, and being able to walk 504 meters during a 6-minute walk test. A 24h ECG recording showed normal mean heart rate of 70 beats per minute and a normal heart rate variability (SDNN 160 ms) and no non-sustained ventricular tachycardia. The patient was still alive after 507 days.

Interpretation: This patient, despite also having advanced cancer, was protected from heart muscle wasting, life-threatening non-sustained ventricular tachycardia and early death, and had normal cardiopulmonary measurements, and near normal exercise capacity, which can be explained by the subject being administered the effective heart failure medications (for another reason) Dapagliflozin, and also the angiotensin receptor blocker, Candesartan.

### CASE 7:

A subject with lymphoma, Ann Arbor Stage 2A, was administered the following medications; Aciclovir 400mg (for viral protection), Ipratropium/Fenoterol and Salmeterol (for asthma), Calciprotriol/Betamethasone 50/0,5 and Acitretin (for Psoriasis), Gabapentin 300mg (for neuralgia), Digitoxin (for atrial fibrillation), Dabigatran 150mg (as an antithrombotic), Ramipril 5mg (for hypertension), Sitagliptin/Metformin 50/1000 mg, Empagliflozin 10mg (an SGLT2 inhibitor, which in this case was prescribed for the subject's diabetes, and not for the cancer affected heart), Insulin glargine (for diabetes) Ondasentron 8 mg and Emend 80mg (for cancer therapy-associated nausea), and Pegfilgrastim 5mg (to stimulate white blood cell production).

An echocardiogram showed a normal LVEF (64%) and increased LV mass (365 gram). The subject had a good self-rated health of 2 (1 =very good, 2=good, 3= fair, 4=poor, 5=very poor). A 24h ECG recording showed a high mean heart rate of 84 beats per minute but a normal heart rate variability (SDNN 224 ms) and no non-sustained ventricular tachycardia. The patient had very good exercise capacity, with a hand grip strength of 46 and taking only 3.5 seconds to walk 4 meters. The subject was still alive after 324 days.

Interpretation: This patient, despite also having advanced cancer, was protected from heart muscle wasting, life-threatening non-sustained ventricular tachycardia and early death, and had normal cardiopulmonary measurements (apart from incidental atrial fibrillation) and good strength and exercise capacity, which can be explained by the administration of the effective heart failure medication, Empagliflozin.

These two cases show that effective treatment with the heart failure drugs, the SGLT2 inhibitors, Dapagliflozin or Empagliflozin improve the heart in advanced heart failure patients, protect them from heart muscle wasting, elevated heart rate, reduced heart rate variability, non-sustained ventricular tachycardia, and early death. These effects are seen despite similar characteristics of the cancer diagnoses.

### CASE 8:

A male subject with Pancreas Cancer, UICC stage 4, was administered the following medication; Allopurinol 100 mg (for gout), Amlodipine 5 mg (for hypertension), Pantoprazole 40 mg (for acid reflux), Nebivolol 7.5 mg (a beta-blocker, which in this case was prescribed for hypertension, and not for the subject's cancer affected heart), Torasemide 10 mg, (for hypertension) Lantus 26 IE (for diabetes), and Enoxaparin 0.3 ml (to prevent thrombosis) and Tamsulosin (for benign prostate hyperplasia). The subject's self-rated health was 3, fair (1 =very good, 2=good, 3= fair, 4=poor, 5=very poor). A 24h ECG recording showed normal mean heart rate of 62 beats per minute and a normal heart rate variability (SDNN 124 ms) and no non-sustained ventricular tachycardia. An echocardiogram showed a normal LVEF (65%) and normal LV mass (236 gram). The subject had reasonable hand grip muscle strength (27kg) and reasonable exercise tolerance with the ability to walk 4 meters in 4.6 seconds. The patient was still alive after 337 days.

Interpretation: This patient, despite also having advanced cancer, was protected from heart muscle wasting, life-threatening non-sustained ventricular tachycardia and early death, and had normal cardiopulmonary measurements and reasonably preserved exercise capacity which can be explained by the subject being administered an effective heart failure medication, the beta-blocker Nebivolol.

### CASE 9:

A male subject with Lung Cancer, UICC stage 4, received the following medication; Candesartan 32 mg (an angiotensin receptor blocker, which in this case was prescribed for hypertension, and not for the subject's cancer affected heart) and Metoprolol 95 mg (a beta-blocker, which in this case was prescribed for hypertension, and not for the subject's cancer affected heart) Lecarnidipine 20 mg, (for hypertension) and Metformin 2000 mg, (for diabetes) . An echocardiogram showed a normal LVEF (67%) and increased LV mass (326 gram). The subject had a good self-rated health of 2 (1=very good, 2=good, 3= fair, 4=poor, 5=very poor). The subject had good hand grip muscle strength (43kg) and good exercise tolerance with the ability to walk 440 meters in 6 minutes. A 24h ECG recording showed no non-sustained ventricular tachycardia.

Interpretation: This patient, despite also having advanced cancer, was protected from heart muscle wasting, life-threatening non-sustained ventricular tachycardia and early death, and had excellent muscle strength and exercise capacity which can be explained by the subject being administered an effective heart failure medication, the beta-blocker metoprolol and the angiotensin receptor blocker, Candesartan.

### CASE 10:

A female Lymphoma patient, Ann Arbor stage 4 E, was administered the following medication; Pantoprazol 40mg (for acid reflux), Ramipril 2.5mg (an ACE inhibitor, which in this case was prescribed for hypertension, and not as a treatment for the subject's cancer affected heart), Eliquis 5mg (to protect against thrombosis), and Amlodipine 10mg. The subject's self-rated health was 3, fair (1=very good, 2=good, 3= fair, 4=poor, 5=very poor). A 24h ECG recording showed normal mean heart rate of 64 beats per minute and a near-normal heart rate variability (SDNN 95 ms) and no non-sustained ventricular tachycardia. An echocardiogram showed a normal LVEF (63%) and normal LV mass (189 gram). The subject had good hand grip muscle strength (29kg) and reasonable exercise tolerance with the ability to walk 4 meters in 3.5 seconds and 440 meters in 6 minutes. The patient was still alive after 458 days.

Interpretation: This patient, despite also having advanced cancer, was protected from heart muscle wasting, life-threatening non-sustained ventricular tachycardia and early death, and had normal cardiopulmonary measurements and good exercise capacity which can be explained by taking an effective heart failure medication, the ACE inhibitor Ramipril.

### CASE 11:

A male Lymphoma patient, Ann Arbor stage 4 E, received medication of Metformin 2000 mg (for diabetes), Ramipril 10 mg (an ACE inhibitor as claimed in claim 28, which in this case was prescribed not as a treatment for his cancer affected heart, but for his hypertension), Amlodipine 10 mg (for hypertension), Aciclovir 800 mg (an antiviral) Cotrim forte 960 mg (an antiobiotic), Allopurinol 300 mg (for gout), Ondasentron 24 mg (for cancer therapy-associated nausea) and Fragmin 5000 IE (to prevent thrombosis). His symptom status was excellent (EQ5D5L Index: 0.999 (1 best possible, 0 worst possible). A 24h ECG recording showed normal mean heart rate of 77 beats per minute and a normal heart rate variability (SDNN 120 ms) and no non-sustained ventricular tachycardia. His echocardiogram showed a normal LVEF (67%) and normal LV mass (227 gram). He had a normal hand grip muscle strength (47kg). The patient is still alive after 666 days.

Interpretation: This patient, despite also having advanced cancer, was protected from heart muscle wasting, life-threatening non-sustained ventricular tachycardia and early death, and had normal cardiopulmonary measurements and good muscle strength which can be explained by taking the effective heart failure medication (for another reason), the ACE inhibitor Ramipril.

### CASE 12:

A female Multiple Myeloma, Durie and Salmon stage 3 patient taking medication of Spironolactone 25 mg (an angiotensin receptor blocker as claimed in claim 28, which in this case was prescribed not as a treatment for her cancer affected heart but for her hypertension), Pantoprazol 40mg, Indapamid 2,5mg, Candesartan 32mg, Amlodipin 10mg, Palladon 4mg (a pain killer), Pregabalin 50mg (an anticonvulsant), and Candesartan 8 mg (an angiotensin receptor blocker, which in this case was prescribed not as a treatment for her cancer affected heart but for her hypertension). Her symptom status was very good (EQ5D5L Index: 0.887 (1 best possible, 0 worst possible)). A 24h ECG recording showed normal mean heart rate of 69 beats per minute and a normal heart rate variability (SDNN 112 ms) and no non-sustained ventricular tachycardia. She had normal hand grip muscle strength (47kg) and good exercise tolerance with the ability to walk 4 meters in 2.5 seconds and 527 meters in 6 minutes. The patient was still alive after 237 days.

Interpretation: This patient, despite also having advanced cancer, was protected from heart muscle wasting, life-threatening non-sustained ventricular tachycardia and early death, and had normal cardiopulmonary measurements and good exercise capacity which we explain by the fact of taking the effective heart failure medication (for another reason), the ACE inhibitor Candesartan.

### CASE 13:

A female Lung cancer, UICC stage 4B patient taking medication of Spironolactone 25 mg (an aldosterone antagonist, which in this case was prescribed not as a treatment for her cancer affected heart but for her hypertension), Pantoprazol 40mg, (for acid reflux Indapamide 2.5mg (for hypertension), Candesartan 32mg (an angiotensin receptor blocker, which in this case was prescribed not as a treatment for her cancer affected heart but for her hypertension) and Amlodipine 10mg (for hypertension). An echocardiogram showed a normal LVEF (66%) and normal LV mass (148 gram). A 24h ECG recording showed a normal heart rate variability (SDNN 101 ms) and no non-sustained ventricular tachycardia. She had normal hand grip muscle strength (40kg). The patient was still alive after 112 days.

Interpretation: This patient, despite also having advanced cancer, was protected from heart muscle wasting, life-threatening non-sustained ventricular tachycardia and early death, and had normal cardiopulmonary measurements and LV muscle mass and good muscle strength which can be explained by her receiving the effective heart failure medication (for another reason), the ACE inhibitor Candesartan and the aldosterone antagonist, Spironolactone.

### CASE 14:

A female Breast Cancer, UICC stage 4 patient received the following medication: Febuxostat 80mg (for gout), Amlodipine 10mg (for hypertension), L-Thyroxine 75 ug (far hypothyroidism), Pantoprazol 40mg (for acid reflux), Torasemide 10mg (for hypertension), Spironolactone 25mg (an aldosterone antagonist as claimed in claims 28 and 37, which in this case was prescribed not as a treatment for her cancer affected heart but for her hypertension) and Fragmin 5000 IW (to prevent thrombosis). An echocardiogram showed a normal LVEF (67%) and normal LV mass (233 gram). A 24h ECG recording showed a normal average heart rate (85 bpm) and a normal heart rate variability (SDNN 142 ms) and no non-sustained ventricular tachycardia. She had near-normal hand grip muscle strength (40kg). The patient was still alive after 235 days.

Interpretation: This patient, despite also having advanced cancer, was protected from heart muscle wasting, life-threatening non-sustained ventricular tachycardia and early death, and had normal cardiopulmonary measurements and LV muscle mass and reasonable muscle strength which can be explained by the patient receiving the effective heart failure medication (for another reason), the aldosterone antagonist, Spironolactone.

### EXAMPLE 14: Treatment of a patient with iron therapy medication

A patient has a cardiac muscle wasting as a consequence of cancer, that is detected using the methods disclosed herein. The patient can be treated with iron. The Iron can be IV Ferric carboxymaltose and can be given to a patient with iron deficiency as estimated by a TSAT of <20% or without iron deficiency of 20% or higher. The patient can be treated with a 1000 mg dose, (or any dose between 50mg and 5000 mg) .

A cancer related heart muscle wasting can be identified in a patient using a method disclosed herein. The patient can be shown to have iron deficiency (TSAT <20%) that can be treated with IV ferric carboxymaltose at a dose of 1000 mg and 2-3 days. As a result of the treatment the patient may feel better as assessed by a KCCQ score and may have an improved 6 minute corridor walk test (6MCWT). One week to 2 weeks later the patient may show improvement in exercise tolerance of 10 meters or more in the 6MCWT.

A cancer related heart muscle wasting can be identified in a patient using a method disclosed herein. The patient can be shown to not have iron deficiency (TSAT = or greater than 20%) and can be treated with IV ferric carboxymaltose at a dose of 1000 mg and 2-3 days. They may feel better as assessed by a KCCQ score and may have an improved 6 minute corridor walk test (6MCWT). One week to 2 weeks later they may show improvement in exercise tolerance of 10 meters or more in the 6MCWT.

The invention includes the aspects in the following paragraphs
1. A method comprising detecting a cardiopulmonary measurement in a subject suspected of having cancer or suspected of having a risk of developing the cancer, and performing a cancer screening, wherein the cardiopulmonary measurement comprises:
   (i) a tachycardia or non-sustained ventricular tachycardia of at least about 100 bpm,
   (ii) from about 10 to about 5,000,000 premature ventricular contractions in a time period of from about 30 seconds to about 3 weeks,
   (iii) an average heart rate during a time period of from about 60 to about 100 bpm and a standard deviation of a 5-minute average NN interval (SDANN) during a time period of from about 1 minute to about 60 minutes that comprises less than about 100 msec,
   (iv) a percentage of successive normal sinus R-R intervals >50 msec (pNN50) that comprises less than 10% and an SDANN during a time period of from about 1 minute to about 60 minutes of at least 100 msec,
   (v) a left ventricular (LV) mass of from about 1% to about 70% below that of an LV mass of an average human or below the previously determined LV mass of the subject, as determined by a computer tomography (CT) scan, or
   (vi) any combination of (i)-(v).
2. A method comprising actuating a cardioverter defibrillator that is directly or indirectly contacted with a subject in need thereof based on a detection of a cardiopulmonary measurement, wherein the cardiopulmonary measurement comprises:
   (i) a tachycardia or non-sustained ventricular tachycardia of at least about 100 bpm,
   (ii) from about 10 to about 5,000,000 premature ventricular contractions in a time period of from about 30 seconds to about 3 weeks,
   (iii) an average heart rate during a time period of from about 60 to about 100 bpm and a standard deviation of a 5-minute average NN interval (SDANN) during a time period of from about 1 minute to about 60 minutes that comprises less than about 100 msec,
   (iv) a percentage of successive normal sinus R-R intervals >50 msec (pNN50) that comprises less than 10% and an SDANN during a time period of from about 1 minute to about 60 minutes of at least 100 msec,
   (v) a left ventricular (LV) mass of from about 1% to about 70% below that of an LV mass of an average human or below the previously determined LV mass of the subject, as determined by a computer tomography (CT) scan, or
   (vi) any combination of (i)-(v).
3. The method of paragraph 2, wherein the subject has or is suspected of having cardiac wasting, is at risk of developing cardiac wasting, or a combination thereof.
4. The method of paragraph 3, wherein the actuating treats, prevents, delays, or ameliorates the cardiac wasting in the subject.
5. The method of any one of paragraphs 1-4, wherein the cardiopulmonary measurement comprises a left ventricular (LV) mass of from about 2% to about 20% below that of an LV mass of an average human or below the previously determined LV mass of that patient, as determined by a computer tomography (CT) scan further comprising diagnosing the subject with cardiac wasting.
6. The method of paragraph 5, wherein the diagnosing comprises echocardiography, or a magnetic resonance imaging (MRI) assessment, or a positron emission tomography-computed tomography (PET-CT) assessment or a scintigraphy assessment.
7. The method of any one of paragraphs 3-6, wherein the subject has a terminal illness and is considered to be in need of palliative care, hospice care, end of life care, an improved quality of life, or any combination thereof.
8. The method of paragraph 7, wherein palliative care or hospice care is at home or in an institutional care structure.
9. The method of paragraph 7, wherein a life expectancy of the subject is less than about 12 months.
10. The method of paragraph 7, wherein a life expectancy of the subject is less than about 6 months.
11. The method of any one of paragraphs 3-10, wherein the subject has elevated plasma levels of a natriuretic peptide, as compared to a healthy subject.
12. The method of paragraph 11, wherein the natriuretic peptide comprises atrial natriuretic peptide (ANP), brain natriuretic peptide (BNP), c-type natriuretic peptide (CNP), dendroaspis natriuretic peptide (DNP), urodilatin, or any combination thereof.
13. The method of any one of paragraphs 1-12, wherein the subject has congestion.
14. The method of any one of paragraphs 3-11, wherein the subject has elevated plasma levels of a troponin, as compared to a healthy subject.
15. The method of paragraph 14, wherein the troponin comprises troponin I or troponin T.
16. The method of any one of paragraphs 3-15, wherein the subject has elevated plasma levels of aldosterone, as compared to a healthy subject.
17. The method of paragraph 2, wherein the cardioverter defibrillator comprises an implantable cardioverter defibrillator.
18. The method of paragraph 2, wherein the cardioverter defibrillator is leadless.
19. The method of paragraph 2, wherein the cardioverter defibrillator comprises a wearable cardioverter defibrillator.
20. The method of any one of paragraphs 1-19, wherein the subject is perambulatory.
21. The method of any one of paragraphs 1-19, wherein the subject is capable of self-hygiene.
22. The method of any one of paragraphs 1-19, wherein the subject has a loss of cardiomyocyte volume relative to a healthy subject.
23. The method of any one of paragraphs 1-19, wherein the subject has had a replacement of fibrotic tissue.
24. The method of paragraph 2, wherein the cardioverter defibrillator can be applied or held in proximity to a skin of the subject.
25. A method of preventing or treating cardiac wasting in a subject having cardiac wasting, or suspected of being at risk of cardiac wasting, comprising detecting a cardiopulmonary measurement, wherein the cardiopulmonary measurement comprises:
   (i) a tachycardia or non-sustained ventricular tachycardia of at least about 100 bpm,
   (ii) from about 10 to about 5,000,000 premature ventricular contractions in a time period of from about 30 seconds to about 3 weeks,
   (iii) an average heart rate during a time period of from about 60 to about 100 bpm and a standard deviation of a 5-minute average NN interval (SDANN) during a time period of from about 1 minute to about 60 minutes that comprises less than about 100 msec,
   (iv) a percentage of successive normal sinus R-R intervals >50 msec (pNN50) that comprises less than 10% and an SDANN during a time period of from about 1 minute to about 60 minutes of at least 100 msec,
   (v) a left ventricular (LV) mass of from about 1% to about 70% below that of an LV mass of an average human or below the previously determined LV mass of the subject, as determined by a computer tomography (CT) scan, or
   (vi) any combination of (i)-(v), and
   administering a medicament to the subject, thereby treating the cardiac wasting in the subject.
26. The method of paragraph 25, wherein the method improves an outcome selected from the group consisting of: a quality of life for the subject, a symptom of the cardiac wasting, a general wellbeing of the subject, a cardiac health of the subject, a predicted survival time, a duration of hospitalization of the subject, a cost of hospitalization for the subject, or any combination thereof.
27. The method of paragraph 26, wherein the improvement is indicated by a visual analog score.
28. The method of any one of paragraphs 25-27, wherein the medicament comprises: a beta receptor blocker, an aldosterone antagonist, an aldosterone synthesis inhibitor, cardioxyl, omecamtiv mecarbil, relaxin, serelaxin, staroxime, bucindolol, a phosphodiesterase 5 inhibitor, a vasopressin inhibitor, levosimendan, a sodium-glucose cotransporter-2 (SGLT2) inhibitor, an I*_{f}* channel blocker, an ACE inhibitor, an angiotensin receptor blocker, a mineralocorticoid receptor antagonist (MRA), a metabolism-modifying agent, a stereoisomer of any of these, a salt of any of these, or any combination thereof.
29. The method of paragraph 28, comprising the beta receptor blocker or salt thereof, wherein the beta receptor blocker or salt thereof comprise at least one stereocenter in an S-configuration.
30. The method of paragraph 29, wherein the beta-blocker comprises a long acting beta blocker.
31. The method of paragraph 29, wherein the beta-blocker comprises a short acting beta blocker.
32. The method of paragraph 30 or 31, wherein the long acting beta blocker or the short acting beta blocker comprises pindolol, oxprenolol, atenolol, acebutolol, bisoprolol, metoprolol, nadolol, nebivolol, propranolol, a stereoisomer of any of these, a polymorph of any of these, a diastereomer of any of these, or a salt of any of these.
33. The method of paragraph 30 wherein the long acting or short acting beta blockers comprise S-pindolol, S-oxprenolol, S-atenolol, S-acebutolol, S-bisoprolol, S-metoprolol, S-nadolol, 5-nebivolol, S-propranolol, a stereoisomer of any of these, a polymorph of any of these, a diastereomer of any of these, or a salt of any of these.
34. The method of paragraph 28, comprising the phosphodiesterase 5 inhibitor or salt thereof, wherein the phosphodiesterase 5 inhibitor comprises amrinone, milrinone, avanafil, lodenafil, mirodenafil, sildenafil, tadalafil, vardenafil, udenafil, zaprinast, benzaminenafil, carvedilol, or a salt of any of these.
35. The method of paragraph 28, comprising the vasopressin inhibitor of salt thereof, wherein the vasopressin inhibitor comprises conivaptan, relcovaptan, nelivaptan, lixivaptan, mozavaptan, satavaptan, tolvaptan, demeclocycline, lithium, or a salt thereof.
36. The method of paragraph 28, comprising the SGLT2 inhibitor or salt thereof, wherein the SGLT2 inhibitor comprises dapagliflozin, empagliflozin, canagliflozin, sotagliflozin, ertugliflozin, ipragliflozin, luseogliflozin, remogliflozin etabonate, sergliflozin etabonate, tofogliflozin a stereoisomer of any of these, or a salt of any of these, or any combination thereof.
37. The method of paragraph 28, comprising the aldosterone antagonist, or salt thereof, wherein the aldosterone antagonist or salt thereof comprises spironolactone, eplerenone, finerenone, potassium canrenoate, a stereoisomer of any of these, a salt of any of these, or any combination thereof.
38. The method of paragraph 28, comprising the aldosterone synthesis inhibitor or a salt thereof, wherein the aldosterone synthesis inhibitor or salt thereof comprises fadrozol, FAD 286, LCI699, or any combination thereof.
39. The method of paragraph 28, comprising the angiotensin receptor antagonist or a salt thereof, wherein the angiotensin receptor antagonist or salt thereof comprises a sartan, candesartan, irbesatan, valsartan, telmisartan, eprosartan, Olmesartan, azilsartan, fimasartan, sacubitril/valsartan, losartan, EXP 3174, amlodipine, a stereoisomer of any of these, a salt of any of these, or any combination thereof.
40. The method of paragraph 28, comprising the metabolism-modifying agent or a salt thereof, wherein the metabolism-modifying agent comprises elamipretide, trimetazidine, perhexiline, etomoxir, dichloroacetate, coenzyme Q10, ubiquinol, nicotinamide riboside, metformin, resveratrol, pterostilbene, rapamycin, a salt of any of these, or any combination thereof.
41. The method of any one of paragraphs 1-40, wherein the cardiopulmonary measurement comprises from about 10 to about 1000 premature ventricular contractions in the time period, wherein the time period comprises about 30 sec to 1 hour.
42. The method of any one of paragraphs 1-41, wherein the cardiopulmonary measurement comprises the average heart rate during the time period of from about 60 to about 100 bpm and the SDANN during the time period that comprises less than about 100 msec, wherein the time period comprises about an hour.
43. The method of any one of paragraphs 1-42, wherein the cardiopulmonary measurement comprises a pNN50 that comprises less than 10% and the SDANN during the time period of at least 100 msec, wherein the time period comprises about an hour.
44. The method of paragraph 1, wherein the cancer comprises a mutation in a protein kinase.
45. The method of paragraph 44, wherein the protein kinase is a tyrosine kinase or a tyrosine kinase receptor.
46. The method of paragraph 45, wherein the protein kinase is a tyrosine kinase, and wherein the tyrosine kinase is Abl, growth factor receptors, EGFR, (ERBB1), HER2 (ERBB2), c-Kit, VEGFR, PDGFR, PI3K pathway, PI3K(ρ110α), PI3K(p110y), Akt, PDK1, Ras/Raf/MEK/ERK pathway, Raf-1/B-Raf, MEK1/2, Cell cycle regulation, CDK, Aurora kinases, PLKs, mTOR, JAK2, or p38.
47. The method of paragraph 46, wherein the protein kinase is a tyrosine kinase receptor, and wherein the tyrosine kinase receptor is imatinib, imatinib D, imatinib N, bosutinib, gefitinib, lapatinib, XI,647, BIBW-2992, sunitinib, sorafenib pazopanib, vatalanib, vandetanib, cediranib, pazopanib, SF-1126, XL765, SF-1126, XL765, SF-1126, XL765, VQD-002, perifosine, UCN-01, sorafenib RAF-265, PD-0325901, AZD-6244, ARRY-162, alvocidib, BI2526, AZD-1152, BI2526, temsirolimus, everolimus, sirolimus, deforolimus, lestaurtinib, CP690550, TG101348, or GW856553.
48. A method comprising performing a cancer screen based on a detection of a cardiopulmonary measurement in a subject, wherein the cardiopulmonary measurement comprises:
   (i) a tachycardia or non-sustained ventricular tachycardia of at least about 100 bpm,
   (ii) from about 10 to about 5,000,000 premature ventricular contractions in a time period of from about 30 seconds to about 3 weeks,
   (iii) an average heart rate during a time period of from about 60 to about 100 bpm and a standard deviation of a 5-minute average NN interval (SDANN) during a time period of from about 1 minute to about 60 minutes that comprises less than about 100 msec,
   (iv) a percentage of successive normal sinus R-R intervals >50 msec (pNN50) that comprises less than 10% and an SDANN during a time period of from about 1 minute to about 60 minutes of at least 100 msec,
   (v) a left ventricular (LV) mass of from about 1% to about 70% below that of an LV mass of an average human or below the previously determined LV mass of the subject, as determined by a computer tomography (CT) scan, or
   (vi) any combination of (i)-(v).
49. The method of paragraph 48, wherein a subject is diagnosed using a method selected from the group consisting of: echocardiography, positron emission tomography-computed tomography (PET-CT), multigated acquisition scan (MUGA) imaging, or any combination thereof.
50. An interoperable electronic device configured to measure a cardiopulmonary measurement, interpret a risk of a presence of a cancer using an algorithm stored on computer readable memory operatively coupled to the device, and transmit a detection of the risk of the presence of the cancer to a second device.
51. An interoperable electronic device configured to measure a cardiopulmonary measurement, interpret a risk of a presence of wasting of left ventricular muscle mass using an algorithm stored on computer readable memory operatively coupled to the device, and transmit a detection of the presence of the wasting of the left ventricular muscle mass to a second device.
52. The interoperable electronic device of paragraph any one of paragraphs 50-51, wherein the device transmits the detection of the cancer to the second device by a wired or wireless transmission.
53. The interoperable electronic device of any one of paragraphs 50-52, wherein the device transmits the detection of the wasting of the left ventricular muscle mass to the second device by a wired or wireless transmission.
54. The interoperable electronic device of any one of paragraphs 50-53, wherein the device is a leadless electrical device.
55. A kit comprising the interoperable electronic device of any one of paragraphs 50-54 at least partially in a packaging.
56. The kit of paragraph 55, further comprising instructions to assess a risk of cancer by recording a cardiopulmonary measurement using the interoperable electronic device.
57. The kit of paragraph 56, further comprising instructions to assess a risk of wasting of left ventricular muscle mass by recording a cardiopulmonary measurement using the interoperable electronic device.
58. The kit of any one of paragraphs 55-57, wherein the cardiopulmonary measurement comprises:
   (i) a tachycardia or non-sustained ventricular tachycardia of at least about 100 bpm,
   (ii) from about 10 to about 5,000,000 premature ventricular contractions in a time period of from about 30 seconds to about 3 weeks,
   (iii) an average heart rate during a time period of from about 60 to about 100 bpm and a standard deviation of a 5-minute average NN interval (SDANN) during a time period of from about 1 minute to about 60 minutes that comprises less than about 100 msec,
   (iv) a percentage of successive normal sinus R-R intervals >50 msec (pNN50) that comprises less than 10% and an SDANN during a time period of from about 1 minute to about 60 minutes of at least 100 msec,
   (v) a left ventricular (LV) mass of from about 1% to about 70% below that of an LV mass of an average human or below the previously determined LV mass of the subject, as determined by a computer tomography (CT) scan, or
   (vi) any combination of (i)-(v).
59. The kit of paragraph 58, wherein the cardiopulmonary measurement is measured using a method selected from the group consisting of: echocardiography, positron emission tomography-computed tomography (PET-CT), multigated acquisition scan (MUGA) imaging, or any combination thereof.
60. A method of monitoring a cancer in a subject, comprising: (a) recording a cardiopulmonary measurement in the subject using the interoperable electronic device of any one of paragraphs 49-54, (b) comparing the cardiopulmonary measurement to a reference level, and (c) repeating (a) and (b).
61. A method of monitoring a wasting of a left ventricular muscle mass in a subject, comprising: (a) recording a cardiopulmonary measurement in the subject using the interoperable electronic device of any one of paragraphs 50-54, (b) comparing the cardiopulmonary measurement to a reference level, and (c) repeating (a) and (b).
62. A method of determining efficacy of an anti-cancer therapeutic in a subject administered the anti-cancer therapeutic, comprising: (a) recording a cardiopulmonary measurement in the subject using the interoperable electronic device of any one of paragraphs 50-54, (b) comparing the cardiopulmonary measurement to a reference level, (c) repeating (a) and (b) for as long as the cardiopulmonary measurement remains higher than the reference level, and (d) performing one of the following:
   (i) terminating the administering of the anti-cancer therapeutic;
   (ii) continuing the administering of the anti-cancer therapeutic; or
   (iii) recommending a change in an anti-cancer therapeutic if the cardiopulmonary measurement remains higher than the reference level for a time period of at least about a month after a first recording of (a).
63. A method of determining a cancer prognosis in a subject comprising (a) recording a number of premature ventricular contractions (PVCs) in a 24 hour time period in the subject using an electronic device configured to monitor the PVCs, (b) repeating (a), and (c) delivering a prognosis to the subject, wherein the subject has a more favorable prognosis if the subject displays a number of PVCs that comprises less than 10 or greater than 100, relative to a subject that displays a number of PVCs of from about 10 to about 100.
64. The method of paragraph 63, further comprising conducting a cancer diagnostic test, wherein the cancer diagnostic test determines a level of a biomarker.
65. The method of paragraph 64, wherein the biomarker comprises a genetic biomarker.
66. The method of paragraph 64, wherein the biomarker comprises an epigenetic biomarker.
67. The method of any one of paragraphs 64-66, wherein the determining comprises sequencing of a sample from the subject.
68. The method of paragraph 67, wherein the sequencing comprises next generation sequencing.
69. The method of paragraph 67 or 68, wherein the sample comprises cell free nucleic acid from the subject.
70. The method of any one of paragraphs 62-69, wherein the determining comprises imaging.
71. The method of any one of paragraphs 64-70, wherein the level of the biomarker is greater than a reference level.
72. The method of any one of paragraphs 64-70, wherein the level of the biomarker is less than a reference level.
73. A method of determining efficacy of a cardiac-active therapeutic in a subject administered the cardiac-active therapeutic, comprising: (a) recording a cardiopulmonary measurement in the subject using the interoperable electronic device of any one of paragraphs 50-54, (b) comparing the cardiopulmonary measurement to a reference level, (c) repeating (a) and (b) for as long as the cardiopulmonary measurement remains higher than the reference level, and (d) performing one of the following:
   (i) terminating the administering of the cardiac-active therapeutic;
   (ii) continuing the administering of the cardiac-active therapeutic; or
   (iii) recommending a change in a cardiac-active therapeutic if the cardiopulmonary measurement remains higher than the reference level for a time period of at least about a month after a first recording of (a).
74. The method of paragraph 73, wherein the cardiac-active therapeutic is selected from the group consisting of: a modulator of a renin angiotensin aldosterone system (RAAS); a neutral endopeptidase (NEP) inhibitor, a natriuretic peptide (NP) receptor agonist, a recombinant NP, a synthetic NP, ularitide, a relaxin receptor activator, a serelaxin receptor activator, a beta-adrenergic receptor modulating agent, a salt of any of these, or any combination thereof.
75. The method of paragraph 74, comprising the modulator of RAAS or salt thereof, wherein the modulator of RAAS comprises a renin inhibitor, an angiotensin-converting enzyme (ACE) inhibitor, an angiotensin receptor antagonist, an angiotensin receptor agonist, an aldosterone synthesis inhibitor, or a salt of any of these.
76. The method of paragraph 74, comprising the beta-adrenergic receptor modulating agent, wherein the beta-adrenergic receptor modulating agent comprises a competitive inhibitor, a partial agonist, an agonist, an activator, or an inverse agonist.
77. A method of determining a cardiac wasting prognosis in a subject comprising (a) recording a number of premature ventricular contractions (PVCs) in a 24 hour time period in the subject using an electronic device configured to monitor the PVCs, (b) repeating (a), and (c) delivering a prognosis to the subject, wherein the subject has a more favorable prognosis if the subject displays a number of PVCs comprising less than about 10 or greater than about 100, relative to a subject that displays a number of PVCs comprising from about 10 to about 100.
78. The method of paragraph 77, further comprising conducting a cardiac wasting diagnostic test, wherein the cardiac wasting diagnostic test determines a level of a biomarker.
79. The method of paragraph 78, wherein the biomarker comprises a genetic biomarker.
80. The method of paragraph 78 or 79, wherein the determining comprises sequencing a sample from the subject.
81. The method of paragraph 80, wherein the sequencing is performed on a cell free nucleic acid from the subject.
82. The method of any one of paragraphs 78-81, wherein the subject has a level of a biomarker that is greater than a reference level.
83. The method of any one of paragraphs 78-81, wherein the subject has a level of a biomarker that is less than a reference level.
84. A method for treating a subject with a medicament, wherein the subject is suffering from cancer or suspected of having a cancer, the method comprising:
   (a) determining whether a subject is suffering from cancer associated cardiac wasting, the method comprising detecting a cardiopulmonary measurement selected from the group consisting of:
      (i) a tachycardia or non-sustained ventricular tachycardia of at least about 100 bpm,
      (ii) from about 10 to about 5,000,000 premature ventricular contractions in a time period of from about 30 seconds to about 3 weeks,
      (iii) an average heart rate during a time period of from about 60 to about 100 bpm and a standard deviation of a 5-minute average NN interval (SDANN) during a time period of from about 1 minute to about 60 minutes that comprises less than about 100 msec,
      (iv) a percentage of successive normal sinus R-R intervals >50 msec (pNN50) that comprises less than 10% and an SDANN during a time period of from about 1 minute to about 60 minutes of at least 100 msec,
      (v) a left ventricular (LV) mass of from about 1% to about 70% below that of an LV mass of an average human or below the previously determined LV mass of the subject, as determined by a computer tomography (CT) scan, or
      (vi) any combination of (i)-(v).
   (b) administering to the patient a medicament, wherein the administering of the medicament improves a measure of cardiac health compared to an otherwise comparable subject which did not receive the medicament.
85. The method of any one of paragraphs 1-24, 26-49, or 60-83119, further comprising administering to the subject a medicament.
86. The method of paragraph 84 or 85, wherein the medicament comprises a cardiac drug selected from the group consisting of: a modulator of a renin angiotensin aldosterone system (RAAS); a neutral endopeptidase (NEP) inhibitor, a natriuretic peptide (NP) receptor agonist, a recombinant NP, a synthetic NP, ularitide, a relaxin receptor activator, a serelaxin receptor activator, a beta-adrenergic receptor modulating agent, an anticoagulant, an antiplatelet agent, an angiotensin-converting enzyme (ACE) inhibitor, an angiotensin II receptor blocker or inhibitor, an angiotensin receptor-neprilysin inhibitor (ARNI), an I*f* channel blocker, a Hydralazine and isosorbide dinitrate, a beta receptor blocker, an alpha receptor blocker, a calcium channel blocker, an aldosterone antagonist, an aldosterone receptor agonist, a calcium channel blocker, a cholesterol-lowering medication, a digitalis preparation, a diuretic, a vasodilator, an mineralocorticoid receptor antagonist, an aldosterone synthesis inhibitor, cardioxyl, omecamtive mecarbil, relaxin, serelaxin, staroxime, bucindolol, a phosphodiesterase 5 inhibitor, a vasopressin inhibitor, levosimendan, a sodium-glucose cotransporter-2 (SGLT2) inhibitor, an I*_{f}* channel blocker, an iron therapy medication, a soluble guanylate cyclase inhibitor, a stereoisomer of any of these, a salt of any of these, or any combination thereof.
87. The method of paragraph 86, wherein the cardiac drug is a heart failure drug.
88. The method of any one of paragraphs 84-87, wherein the patient has cardiac wasting.
89. The method of paragraph 86, comprising the anticoagulant or the stereoisomer or salt thereof, wherein the anticoagulant comprises an apixaban, a dabigatran, an edoxaban, a heparin, a rivaroxaban, a warfarin, a stereoisomer of any of these, a salt of any of these, or any combination thereof.
90. The method of paragraph 86, comprising the antiplatelet agent or the stereoisomer or salt thereof, wherein the antiplatelet agent comprises an aspirin, a clopidogrel, a dipyridamole, a prasugrel, a ticagrelor, a stereoisomer of any of these, a salt of any of these, or any combination thereof.
91. The method of paragraph 86, comprising the angiotensin-converting enzyme (ACE) inhibitor or the stereoisomer or salt thereof, wherein the angiotensin-converting enzyme (ACE) inhibitor comprises a benazepril, a captopril, an enalapril, a fosinopril, a lisinopril, a moexipril, a perindopril, a quinapril, a ramipril, a trandolapril, a stereoisomer of any of these, a salt of any of these, or any combination thereof.
92. The method of paragraph 86, comprising the angiotensin II receptor blocker or inhibitor or the stereoisomer or salt thereof, wherein the angiotensin II receptor blocker or inhibitor comprises an azilsartan, a candesartan, an eprosartan, an irbesartan, a losartan, an olmesartan, a telmisartan, a valsartan, a stereoisomer of any of these, a salt of any of these, or any combination thereof.
93. The method of paragraph 86, comprising the angiotensin receptor-neprilysin inhibitor (ARNI) or the stereoisomer or salt thereof, wherein the angiotensin receptor-neprilysin inhibitor (ARNI) comprises a sacubitril, a valsartan, a stereoisomer of any of these, a salt of any of these, or any combination thereof.
94. The method of paragraph 86, comprising the I*f* channel blocker or the stereoisomer or salt thereof, wherein the I*f* channel blocker comprises an ivabradine a stereoisomer thereof, a salt of any of these, or any combination thereof.
95. The method of paragraph 86, comprising the beta-blocker or the stereoisomer or salt thereof, wherein the beta-blocker comprises S-pindolol, S-oxprenolol, S-atenolol, acebutolol, atenolol, betaxolol bisoprolol, metoprolol, nadolol, nebivolol, propranolol, sotalolol, a stereoisomer of any of these, a salt of any of these, or any combination thereof.
96. The method of paragraph 86, comprising the calcium channel blocker or the stereoisomer or salt thereof, wherein the calcium channel blocker comprises an amlodipine, a diltiazem, felodipine, nifedipine, a nimodipine, a nisoldipine, a verapamil, a stereoisomer of any of these, a salt of any of these, or any combination thereof.
97. The method of paragraph 86, comprising the phosphodiesterase 5 inhibitor or the stereoisomer or salt thereof, wherein the phosphodiesterase 5 inhibitor comprises amrinone, milrinone, avanafil, lodenafil, mirodenafil, sildenafil, tadalafil, vardenafil, udenafil, zaprinast, benzaminenafil, carvedilol, a stereoisomer of any of these, or a salt of any of these, or any combination thereof.
98. The method of paragraph 86, comprising the vasopressin inhibitor or the stereoisomer or salt thereof, wherein the vasopressin inhibitor comprises tolvaptan, conivaptan, a stereoisomer of any of these, a salt of any of these, or any combination thereof.
99. The method of paragraph 86, comprising the SGLT2 inhibitor or the stereoisomer or salt thereof, wherein the SGLT2 inhibitor comprises dapagliflozin, empagliflozin, canagliflozin, sotagliflozin, ertugliflozin, ipragliflozin, luseogliflozin, remogliflozin etabonate, sergliflozin etabonate, tofogliflozin a stereoisomer of any of these, a salt of any of these, or any combination thereof.
100. The method of paragraph 86, comprising the aldosterone antagonist or the stereoisomer or salt thereof, wherein the aldosterone antagonist comprises spironolactone, eplerenone, a stereoisomer of any of these, a salt of any of these, or any combination thereof.
101. The method of paragraph 86, comprising the cholesterol-lowering medication or the stereoisomer or salt thereof, wherein the cholesterol-lowering medication comprises a statin, a nicotinic acid, a cholesterol absorption inhibitor, a stereoisomer of any of these, a salt of any of these, or any combination thereof.
102. The method of paragraph 101, comprising the statin or the stereoisomer or salt thereof, wherein the statin comprises an atorvastatin, a fluvastatin, a lovastatin, a pitavastatin, a pravastatin, a rosuvastatin, a simvastatin, a stereoisomer of any of these, a salt of any of these, or any combination thereof.
103. The method of paragraph 101, comprising the nicotinic acid or the stereoisomer or salt thereof, wherein the nicotinic acid can comprise niacin, a stereoisomer thereof, a salt of either of these, or any combination thereof.
104. The method of paragraph 101, comprising the cholesterol absorption inhibitor or stereoisomer or salt thereof, wherein the cholesterol absorption inhibitor can comprise ezetimibe, a stereoisomer thereof, a salt of either of these, or any combination thereof.
105. The method of paragraph 101, comprising the combination statin and cholesterol absorption inhibitor or the stereoisomer or salt thereof, wherein the combination statin and cholesterol absorption inhibitor can comprise ezetimibe and simvastatin, a stereoisomer of either of these, a salt of either of these, or any combination thereof.
106. The method of paragraph 86, comprising the digitalis preparation or the stereoisomer or salt thereof, can comprise digoxin, a stereoisomer thereof, a salt of either of these, or any combination thereof.
107. The method of paragraph 86, comprising the diuretic or the stereoisomer or salt thereof, wherein the diuretic comprises an acetazolamide, an amiloride, a bumetanide, a chlorothiazide, a chlorthalidone, a furosemide, a hydro-chlorothiazide, an indapamide, a metalozone, a spironolactone, a torsemide, a stereoisomer of any of these, a salt of any of these, or any combination thereof.
108. The method of paragraph 86, comprising the vasodilator or the stereoisomer or salt thereof, wherein the vasodilator comprises an isosorbide dinitrate, an isosorbide mononitrate, a hydralazine, a nitroglycerin, a minoxidil, a stereoisomer of any of these, a salt of any of these, or any combination thereof.
109. The method of paragraph 86, comprising the mineralocorticoid receptor antagonist or the stereoisomer or salt thereof, wherein the mineralocorticoid receptor antagonist comprises eplerenone, spironolactone, finerenone, potassium canrenoate, spironolactone, a stereoisomer of any of these, a salt of any of these, or any combination thereof.
110. The method of paragraph 86, comprising the aldosterone synthesis inhibitor, wherein the aldosterone synthesis inhibitor comprises fadrozol, FAD 286, LCI699, a stereoisomer of any of these, a salt of any of these, or any combination thereof.
111. The method of paragraph 86, comprising the angiotensin receptor antagonist or a stereoisomer or salt thereof, wherein the angiotensin receptor antagonist comprises a sartan, candesartan, irbesatan, valsartan, telmisartan, eprosartan, olmesartan, azilsartan, fimasartan, sacubitril/valsartan, losartan, EXP 3174, amlodipine, a stereoisomer of any of these, a salt of any of these, or any combination thereof.
112. The method of paragraph 86, comprising the I*f* channel blocker or a stereoisomer or a salt thereof, wherein the I*f* channel blocker comprises ivabradine, zatebradine, cilobradine, ZD7288, alinidine, a salt of any of these, or any combination thereof.
113. The method of paragraph 86, comprising the iron therapy medicament or the stereoisomer or salt thereof, wherein the iron therapy medicament comprises an oral iron, an injectable iron, an intravenous preparation, an IV ferric carboxymaltose, an iron sucrose, an iron isomaltose, an iron, a stereoisomer of any of these, a salt of any of these, or any combination thereof.
114. The method of paragraph 86, comprising the soluble guanylate cyclase stimulator or a salt thereof, wherein the soluble guanylate cyclase stimulator comprises a BAY 41-8543, a BAY 41-2272, a riociguat, a vericiguat, or any combination thereof.
115. The method of paragraph 86, comprising the aldosterone receptor antagonist or the stereoisomer or salt thereof, wherein the aldosterone receptor antagonist comprises spironolactone, eplerenone, finerenone, esaxerenone, apararenone, a stereoisomer of any of these, a salt of any of these, or any combination thereof.
116. The method of any one of paragraphs 84-115, wherein the detecting the cardiopulmonary measurement comprises using an interoperable electronic device.
117. The method of paragraph 116, wherein the interoperable electronic device comprises an implantable cardioverter defibrillator (ICD), an implantable loop recorder, a wearable device, an echocardiography device, or a combination thereof.
118. The method of any one of paragraphs 1-49, or 60-117, further comprising treating the subject with a device configured to alter an autonomic balance.
119. The method of paragraph 118, wherein the autonomic balance comprises a baroflex stimulator, a chemoflex inhibition, a sympathetic nerve ablation, a renal artery nerve ablation, phrenic nerve stimulation, a vagal nerve stimulation, arterial wall electrical stimulation, a cardiac plexus nerve stimulator, or any combination thereof.
120. The method of any one of paragraphs 1-49, or 60-119, further comprising treating the subject with a device configured to alter an abnormal intracardiac pressure.
121. The method of paragraph 120, wherein the device configured to alter an abnormal intracardiac pressure comprises an inter-atrial decompression device, an intracardiac stent, an intra-cardiac conduit, a tissue ablation device, or any combination thereof.

## Claims

1. A kit comprising an interoperable electronic device at least partially in a packaging, wherein the interoperable electronic device comprises a computer readable memory operatively coupled to the interoperable electronic device, and the interoperable electronic device is configured to measure a cardiopulmonary measurement, interpret a risk of a presence of wasting of left ventricular muscle mass based on the cardiopulmonary measurement using an algorithm stored on the computer readable memory operatively coupled to the interoperable electronic device, and transmit a detection of the presence of the wasting of the left ventricular muscle mass to a second device, wherein the cardiopulmonary measurement comprises:
(i) a non-sustained ventricular tachycardia of at least about 100 bpm,
(ii) from about 10 to about 5,000,000 premature ventricular contractions in a time period of from about 30 seconds to about 3 weeks,
(iii) an average heart rate during a time period of from about 60 to about 100 bpm and a standard deviation of a 5-minute average NN interval (SDANN) during a time period of from about 1 minute to about 60 minutes that comprises less than about 100 msec,
(iv) a percentage of successive normal sinus R-R intervals >50 msec (pNN50) that comprises less than 10% and an SDANN during a time period of from about 1 minute to about 60 minutes of at least 100 msec,
(v) a left ventricular (LV) mass of from about 1% to about 70% below that of an LV mass of an average human or below the previously determined LV mass of the subject, as determined by a computer tomography (CT) scan, or
(vi) any combination of (i)-(v).

2. The kit of claim 1, further comprising instructions to assess a risk of wasting of left ventricular muscle mass by recording a cardiopulmonary measurement using the interoperable electronic device.

3. The kit of claim 1 or 2, wherein the interoperable electronic device transmits the detection of the wasting of the left ventricular muscle mass to the second device by wireless transmission.

4. The kit of any one of claims 1-3, wherein the interoperable electronic device is a leadless electrical device.

5. The kit of any one of claims 1-4, wherein the interoperable electronic device is configured to compare a cardiopulmonary measurement to a reference level using the algorithm stored on computer readable memory operatively coupled to the interoperable electronic device.

6. The kit of any one of claims 1-5, wherein the interoperable electronic device comprises a wearable device.

7. The kit of any one of claims 1-6, wherein the interoperable electronic device comprises a cardiac defibrillator.

8. The kit of any one of claims 1-6, wherein the interoperable electronic device comprises a cardiac pacemaker.

9. The kit of any one of claims 1-8, wherein the interoperable electronic device is further configured to interpret a risk of degenerative cardiomyopathy.

10. The kit of any one of claims 1-9, wherein the interoperable electronic device is further configured to interpret a risk of sudden cardiac death.
